# EUROPEAN PATENT APPLICATION

(11) **EP 4 759 259 A1**
(43) Date of publication of application: **17.06.2026**
(21) Application number: 25222298.9
(22) Date of filing: 10.12.2025
(51) Int. Cl.: A61B 18/18

(54) **METHOD AND DEVICE FOR MICROWAVE TREATMENT**

(30) Priority: 11.12.2024 US 202463730544 P
(71) Applicant: BTL Healthcare Technologies a.s., Nové Mesto 120 00 Prague (CZ)
(72) Inventor: Sikora, Jan, 267 01 Kraluv Dvur (CZ)
(74) Representative: DTS Patent- und Rechtsanwälte PartmbB

(57) **Abstract**

The invention refers to a device for delivering microwave energy to a tissue, comprising at least one treatment element configured to emit microwave energy, wherein the microwave energy is used for the treatment of the tissue of a patient.

## Description

### Cross-Reference to Related Applications

This application claims priority to U.S. Provisional Patent Application No. 63/730,544, filed December 11, 2024, which is incorporated by reference herein in its entirety.

### Technical Field

The present invention relates to a device for the generation and delivery of microwave energy. The present invention relates to a device for microwave treatment. In particular, the field of the invention relates to systems for the delivery of microwave energy to the tissue of a subject to achieve various therapeutic and/or aesthetic results.

### Background

Face lift, skin tightening, wrinkle reduction, reduction of fat tissue, and muscle building are aesthetic treatments for which there is a growing demand. Various energy sources are commonly used for aesthetic treatment, such as light sources, ultrasound sources, and radio-frequency sources, each source having a different beneficial effect on the subject's tissue.

The term 'microwave' is generally used to denote a frequency range from 300 MHz to 300 GHz or more. It may include frequencies with wavelengths of 1 mm to 1 m. Microwave radiation can be used in aesthetics and physiotherapy, as well as in medicine.

Microwaves may be used in aesthetics to provide various aesthetic procedures. Microwaves may be used to tighten the skin and stimulate collagen and/or elastin production, leading to improved skin texture and elasticity. This application is commonly used for facial rejuvenation and reducing the appearance of wrinkles. Microwaves may also be used in body contouring to reduce fat in specific body parts. The heat generated by microwave radiation can destroy fat cells and/or heat fibrous bands beneath the skin's surface to smooth out the skin and reduce the appearance of cellulite.

Microwaves may be used in diathermy to generate deep heat within body tissues. Diathermy is a physical therapy commonly used to generate deep heat within body tissues. The diathermy may deliver moderate heat directly to pathologic lesions in the deeper tissues of the body.

Microwaves may be used to promote blood flow, help relieve pain, promote tissue healing, relax muscles, enhance the rehabilitation process, improve tissue repair, reduce inflammation, increase tissue elasticity, increase tissue flexibility, and/or reduce muscle spasms. It can be used to treat conditions such as arthritis, muscle pain, various injuries, inflammatory diseases, and/or conditions that affect the musculoskeletal system. It is often used in the recovery phase of sports injuries and postoperative recovery.

The heat generated by microwaves may also be used to treat and/or destroy structures such as sweat glands or hair follicles, to treat dermatological problems such as, for example, dermatitis, warts, eczema, psoriasis, acne, nail disorders such as onychocryptosis, and/or fungal infections.

The heat generated by microwaves may be also used in dentistry, to treat various dental and oral conditions, and/or to be used before the dental procedure, during the dental procedure or after the dental procedure to increase fluid circulation, help relieve pain, reduce swelling, and/or to accelerate the healing process of the affected tissues.

Alternatively, microwaves may be used for sterilization.

Microwave radiation may be used to treat conditions resulting from human papillomavirus infection (HPV), such as warts or precancerous lesions.

Microwaves may be used in medicine to heat body tissues to high temperatures to treat various diseases, such as cancer (e.g. by microwave ablation). The heat can cause cell death within a tumor. Hyperthermia also increases blood flow to the warmed area and improves oxygen delivery to the area. This mechanism is also beneficial when using hyperthermia in combination with other cancer treatments, such as radiotherapy or chemotherapy. Hyperthermia may be applied locally, heating a very small target area, such as a small tumor, without affecting and damaging other healthy tissue. Hyperthermia may also be applied regionally to a larger area, such as an entire organ or limb. Hyperthermia may also be applied to the entire body, which can be used to treat metastatic cancer.

Metastatic cancer may also be treated by low-heat energy by activation of the immune system.

Microwave radiation may also be used in various electrosurgical devices for tissue ablation and/or tissue coagulation.

Microwave radiation may be used to improve an immune response of a subject's body.

Microwaves may be used in combination with solutions or drugs applied on and/or into a patient. In one aspect, the microwaves may have a synergistic effect with the solutions or drugs. In another aspect the microwaves may promote absorption of used solutions or drugs, e.g. absorption of the solution or drugs into the skin of the patient, via thermal heating.

Microwave radiation may be used both externally and internally.

Internal microwave radiation may be used to treat cavities, such as vagina, cervix, urethra, rectum, anal canal, bladder, veins, nose, or mouth. The applicator may be an internal applicator shaped in a shape suitable to be inserted into cavities.

The internal applicator may comprise one or more detachable parts removably coupled to the applicator handle.

There is a continued need for improvement of systems for performing skin and body rejuvenation, face lifting, skin tightening, restoring collagen and elastin production, body contouring, fat tissue reduction, and fat tissue removal.

There is also a need to improve the devices and methods to affect the superficial musculoaponeurotic system (SMAS) which is a fibrous network comprising a platysma muscle, parotid fascia, and fibromuscular layer extending mostly from the neck up to the forehead, e.g. covering the cheek, forehead, neck and so on. One function of the SMAS is to transmit and distribute the activity of all facial muscles.

Surgical manipulation of the SMAS allows for, for example, complete facial rejuvenation. Rhytidectomy and other facial invasive procedures are typically performed under general anesthesia. Another major consideration in any facial surgery is the location of the facial nerve. The facial nerve exits the temporal bone of the skull via the stylomastoid foramen and travels through the parenchyma of the parotid gland, where it divides into its main branches at the pes anserinus. The nerve runs deep into the SMAS and its contiguous layers and innervates the mimetic muscles from their deep surfaces except the levator anguli oris, buccinator, and mentalis muscles. During rhytidectomy, the frontal and marginal mandibular branches are the most commonly injured motor nerves. Further, the great auricular nerve (GAN) is even more frequently injured. Thus, there is a need for improvement of the systems and methods of performing rhytidectomy and improving visual appearance of the face, neck, chest, décolletage area, and other areas. The décolletage area is a portion of skin revealed by a low neckline including mainly the chest and/or neck area. One of these promising methods may be noninvasive treatment by the microwave radiation, for example provided by a microwave waveguide and/or microwave antenna.

Substrate Integrated Waveguide (SIW) antenna is a type of antenna that integrates waveguide technology into a planar or substrate format. This technology offers several advantages over traditional waveguide systems and is used in radiofrequency, microwave, and millimeter-wave applications. It brings various benefits to the field of microwave devices and offers several advantages over traditional waveguides and microstrip lines, such as cost-effective fabrication, flexible design, material costs, easier integration into planar circuits, and lower propagation losses. SIW antennas are integrated into a planar substrate, making them much smaller and lighter than traditional waveguides or other standalone antennas. SIW antennas are also able to withstand higher frequencies than waveguides and microstrip antennas because SIW antennas exhibit lower propagation losses than microstrip antennas and can handle higher frequencies more efficiently due to reduced dielectric and radiation losses. Moreover, SIW antennas are easily integrated into planar systems, enabling direct connections to other RF components (e.g., filters, amplifiers) on the same substrate compared to traditional waveguides that require complex transitions to planar circuits, increasing design complexity and insertion loss. Last but not least, SIW supports various antenna designs and can be tailored to specific radiation patterns, bandwidths, and frequencies.

Microstrip components are very good for low frequency applications but tend to have higher radiation losses at microwave and millimeter-wave frequencies. Microstrip components also involve rigorous fabrication concessions and are sensitive to fabrication tolerances, especially in high-frequency applications. SIW on the other hand offers lower losses at the microwave and millimeter-wave frequencies and are less sensitive to fabrication tolerances.

Waveguide components are ideal for higher frequency applications, but can be expensive, difficult, and time-consuming to fabricate. They can also have a high radiation loss due to discontinuity and it can be challenging to integrate them with planar circuits. Traditional waveguides can also be bulky and heavy. SIW on the other hand can be fabricated using standard circuit board (CB) manufacturing techniques, which can be very cost-effective. They are planar, which makes integration with other planar components seamless and easy, allowing for more compact and efficient designs.

SIW antennas provide a middle ground between the low loss and high-power handling of traditional waveguides and the compact, planar, and cost-effective nature of microstrip lines. The SIW antenna may have much better loss characteristics than microstrips, allowing for the design of millimeter wave high-Q filters, diplexers, resonators and other circuits using a low-cost fabrication technique. They are compact and lightweight, have high-quality performance and low losses, are versatile, easy to fabricate and can be used in various applications, including therapeutic applications and hyperthermia treatment.

The SIW is usually composed of a dielectric substrate covered on both faces by a conductive layer. The substrate contains two parallel rows of plots or holes, connecting the top and bottom conductive layer. The organization of these plots or holes and the geometric parameters determine the parameters of the radiation. Just like in a conventional waveguide, electromagnetic waves propagate through the SIW. The plots or holes confine the electromagnetic fields, allowing for waveguide-like behavior but in a planar structure.

The fabrication of SIW antennas uses standard printed circuit board (PCB) manufacturing techniques, making it cost-effective and compatible with mass production. This ease of fabrication is a significant advantage over traditional waveguides, which require more complex machining processes.

One advantage of SIW is that the amount of metal that carries the signal is much greater than in, for example, a microstrip. Therefore, the conductor loss of the SIW is lower.

On the other hand, waveguides are mechanically robust and resistant to harsh environmental conditions. They can also handle significantly higher power levels compared to SIW or microstrip antennas and also offer superior directionality and focused control of EM radiation. Waveguides may exhibit extremely low losses at microwave and millimeter-wave frequencies due to their metallic walls and efficient confinement of electromagnetic waves, which ensures maximum energy transfer and minimal signal degradation. Additionally, waveguides may handle significantly higher power levels at 2.4 GHz compared to antennas like microstrip or SIW, which may be especially important in high-energy applications, such as therapeutic devices.

The present method provides a device and a method for tissue treatment by microwave energy without the need for surgery.

### Summary of the Invention

The application of microwave energy to the tissue may have various benefits on the body and skin function and/or appearance. The present invention incorporates an energy delivery applicator connected to a microwave treatment system in order to mainly heat the tissue adjacent to the skin surface and/or to heat the deeper layers of the tissue below the skin surface. Also, it may provide a predefined radiation pattern, forming radiation maxima and minima at predefined locations. The radiation pattern may affect at least one of the epidermis, dermis, hypodermis, SMAS, connective tissue (e.g. fibrous connective tissue such as ligaments), muscles, or adipose tissue. The radiation pattern may also affect collagen and/or elastin production. This present device and method may be able to provide face lifting, neck lifting, décolletage lifting, general lifting, skin tightening, skin rejuvenating, stimulation of collagen and/or elastin production and its use may result in a wide range of aesthetic benefits, without the need for invasive approach. In another aspect, the present device may also be used in the physiotherapy field, introducing deep tissue heating which results in increasing blood flow, reducing pain, enhancing tissue healing and reducing inflammation. The device may be used in sports medicine to treat various musculoskeletal and orthopedic conditions, to prevent disease and/or promote health. In yet another aspect, the present device may be used in the field of medicine, for example in hyperthermia treatment of cancer, or to treat various dermatological conditions such as sweat glands, warts, eczema and/or acne. In yet another aspect, the present device and methods may use microwaves in combination with solutions or drugs applied on and/or into a patient. In yet another aspect, the device generating microwaves may be also used in dentistry, to treat various dental and oral conditions, and/or to be used before, after or during the dental procedure to increase the fluid circulation, help relieve pain, reduce swelling, and/or to accelerate the healing process of the affected tissues. In yet another aspect, the microwaves may be used for sterilization. Therefore, the present invention attempts to overcome some limitations of the previous art.

In accordance with the first aspect of the present invention, an applicator for delivering microwave energy to the subject's body is provided.

In accordance with another aspect of the invention, an applicator comprising at least one treatment array comprising of at least two treatment elements is provided, alternatively at least four treatment elements, alternatively at least six treatment elements.

In accordance with still another aspect of the invention, a system for delivering microwave energy to the treated tissue is provided.

In accordance with still another aspect of the invention, a method for treating the patient's body is provided.

In accordance with still another aspect of the invention, a cooling unit is provided to cool the target tissue and/or the applicator.

In accordance with still another aspect of the invention, a system for delivering microwave energy to the treated tissue in combination with cooling of the target tissue and/or the applicator is provided.

In accordance with still another aspect of the invention, an applicator comprising a SIW antenna is provided.

In accordance with still another aspect of the invention, an applicator comprising a ceramic waveguide is provided.

The aspects of the invention may be combined.

The treated body regions may comprise all or parts of head (e.g. face, or chin), neck, décolletage/cleavage, abdomen, buttocks, thigh, back, breasts, legs and ankles. Furthermore, the treated body region may comprise the submentum, arm, shoulder, elbow, hand, palm, wrist, finger, ack, torso, hull, knee, feet, toe, or genital tissue.

The device may be configured to treat at least one body part. The body part may be for example all or parts of head, (e.g. face, cheeks, forehead, area under the eyes, area around nose, and/or eyes), neck, décolletage/cleavage, abdomen, buttocks, thigh, back, breasts, legs, ankles, hands, feet, torso, submentum, arm, shoulder, elbow, palm, wrist, finger, ack, hull, knee, toe, cavities such as oral cavities or vagina, and/or genital tissue.

### Brief description of the figures

Fig. 1 is a schematic diagram of a treatment system
Fig. 2 is a schematic diagram of treatment elements in the one or more applicators
Fig. 3 is a schematic diagram of the treatment elements
Fig. 4A is a schematic diagram of the treatment elements in one or more applicators
Fig. 4B is a schematic diagram of the treatment elements in one or more applicators
Fig. 4C is a schematic diagram of the treatment elements in one or more applicators
Fig.5 is a schematic diagram of SIW antenna
Fig. 6 is a schematic diagram of SIW antenna
Fig. 7A is a schematic diagram of SIW antenna
Fig. 7B is a schematic diagram of SIW antenna
Fig. 7C is a schematic diagram of SIW antenna
Fig. 7D is a schematic diagram of SIW antenna
Fig. 8A is a schematic diagram of the spacing element
Fig. 8B is a schematic diagram of the spacing element
Fig. 9A is a schematic diagram of the cooling unit
Fig. 9B is a schematic diagram of the cooling unit
Fig. 10A is a schematic diagram of the applicator
Fig. 10B is a schematic diagram of the applicator
Fig. 10C is a schematic diagram of the applicator
Fig. 10D is a schematic diagram of the applicator
Fig. 11A is schematic diagram of the applicator
Fig. 11B is schematic diagram of the applicator
Fig. 11C is schematic diagram of the applicator
Fig. 12 is a schematic diagram of the applicator
Fig. 13A is a schematic diagram of various configurations of at least two treatment elements
Fig. 13B is a schematic diagram of various configurations of at least two treatment elements
Fig. 13C is a schematic diagram of various configurations of at least two treatment elements
Fig. 13D is a schematic diagram of various configurations of at least two treatment elements
Fig. 14A is an exemplary diagram of the waveguide
Fig. 14B is an exemplary diagram of the waveguide
Fig. 15 is an exemplary system of a treatment system
Fig. 16 is a schematic diagram of an applicator
Fig. 17 is a schematic diagram of a connecting tube
Fig. 18A and Fig. 18B is a schematic diagram of a treatment element generating hot spots (Fig. 18A) and treatment element surrounded by shaping element uniforming the thermal field (Fig. 18B)

### Detailed description

Microwave treatment provides microwaves into the soft tissue in order to provide thermal effects in the subject's soft tissue. Soft tissue includes at least one of the skin, epidermis, dermis, hypodermis, muscle, adipose tissue, fascia, SMAS, fibrous tissue, connective tissue (e.g. fibrous connective tissue such as ligaments), and nervous tissue. Microwave treatment may have various stimulating effects, such as face lifting, skin tightening, skin rejuvenating, stimulation of collagen and/or elastin production, reduction of the number and/or volume of adipose cells, and others.

The elevation of the temperature of a living tissue using microwaves may have various therapeutic and/or aesthetic results. When the tissue is irradiated with radiation, such as microwave energy, the energy is absorbed by each layer of the tissue as the radiation passes through the tissue. The amount of energy absorbed by the tissue and the effect of the irradiation may be influenced by several parameters, such as, for example, the frequency of the electromagnetic (EM) radiation (e.g. microwaves), the amount of time each tissue layer is irradiated, the power of the EM radiation, the dielectric properties of tissue, the geometric properties and the dimensions of the tissue, the amount of cooling delivered to the tissue, and others.

In one aspect of the invention, the power of the EM radiation (e.g microwaves) at the output of the applicator may be in a range from 1 Watt to 10 000 Watts, or in a range from 250 Watts to 7500 Watts, or in a range from 450 Watts to 5500 Watts.

In one aspect of the invention, the power of the EM radiation (e.g microwaves) at the output of the applicator may be in a range from 1 Watt to 1000 Watts, or in a range from 250 Watts to 750 Watts, or in a range from 450 Watts to 550 Watts.

In another aspect, the power of the EM radiation (e.g. microwaves) at the output of the applicator may be in a range of 1 W to 550 W, in a range of 5 W to 450 W, or in a range of 12 W to 400 W, or in a range of 15 W to 300 W, or in a range of 17 W to 275 W, or in a range of 19 W to 225 W, or in a range of 26 W to 187 W.

The power of the EM radiation may be generated through the generator, and/or boosted by the amplifier. The output power in the form of an energy and/or amplified energy may be transferred through a treatment element to the output of the applicator. At the applicator's output, this energy may be distributed onto the target tissue.

The amplified energy may then be transferred through a treatment element, such as a waveguide or another intermediary component, and delivered to the output of the applicator.

When EM radiation (e.g. microwaves) is used to heat the tissue in the deeper layers below the skin, it is important to limit the possible heat-induced damage to the skin surface and tissue adjacent to the skin surface. This can be done using external mechanisms to remove heat from the tissue close to the skin's surface. For example, various cooling mechanisms, such as water bolus, have been used to cool the skin surface and prevent damage or heat-induced injuries.

According to one aspect of the invention, one or more applicators may be used to deliver energy to the subject's soft tissue. One or more applicators may contain one or more treatment elements, wherein the treatment element may comprise a microwave antenna, an electrode (e.g. a radiofrequency electrode), a light source, and/or ultrasound transducers. Also, one or more applicators and/or their surface may act as the treatment element.

The applicator, configured to deliver energy to the skin, may have a diameter of a contact surface in a range from 1 cm to 6 cm, a range from 2 cm to 5 cm, or a range from 3 cm to 4 cm. In another aspect, the applicator may have a contact surface in a range from 0.785 cm² to 28.3 cm², or from 1 cm² to 36 cm², or from 3.14 cm² to 20 cm², or from 7 cm² to 13 cm², or from 1 cm² to 16 cm², from 2 cm² to 7 cm², or in a range from 3 cm² to 6 cm², or 4 cm² to 5 cm². The contact surface is a surface of the applicator that comes into a contact with the patient during the treatment.

The applicator, configured to deliver energy to the skin, may have a diameter of a contact surface in a range from 1 mm to 4 cm, or a range from 1.5 mm to 3 cm, or a range from 3 mm to 2 cm. In another aspect, the applicator may have a contact surface in a range from 0.00785 cm² to 12.57 cm², or from 0.0177 cm² to 7.07 cm², or from 0.0707 cm² to 3.14 cm². The contact surface is a surface of the applicator that comes into a contact with the patient during the treatment.

In some aspects, the applicator may have a width of a contact surface in a range from 1 mm to 4 cm, or from 1.5 mm to 3 cm, or from 3 mm to 2 cm, or from 1 cm to 4 cm, or from 1.5 cm to 3 cm, or from 1.8 mm to 2.5 cm, or from 1.9 cm to 2.2 cm.

In some aspects, the applicator may have a height of a contact surface in a range from 1 mm to 4 cm, or from 1.5 mm to 3 cm, or from 3 mm to 2 cm, or from 1 cm to 4 cm, or from 1.5 cm to 3 cm, or from 1.8 mm to 2.5 cm, or from 1.9 cm to 2.2 cm.

In some aspects, the applicator may have a surface area of a contact surface in a range from 0.01 cm² to 16 cm², or from 0.015 cm² to 12 cm², or from 0.03 cm² to 8 cm², or from 0.1 cm² to 16 cm², or from 0.15 cm² to 12 cm², or from 0.018 cm² to 10 cm², or from 0.18 cm² to 10 cm², or from 0.19 cm² to 8.8 cm², or from 0.0225 cm² to 9 cm², or from 0.045 cm² to 6 cm², or from 0.225 cm² to 9 cm², or from 0.027 cm² to 7.5 cm², or from 0.27 cm² to 7.5 cm², or from 0.285 cm² to 6.6 cm², or from 0.09 cm² to 4 cm², or from 0.3 cm² to 8 cm², or from 0.45 cm² to 6 cm², or from 0.054 cm² to 5 cm², or from 0.342 cm² to 5.5 cm², or from 0.57 cm² to 4.4 cm², or from 1 cm² to 16 cm², or from 1.5 cm² to 12 cm², or from 1.9 cm² to 8.8 cm², or from 2.25 cm² to 9 cm², or from 2.85 cm² to 6.6 cm², or from 3.61 cm² to 4.84 cm², or from 0.02 cm² to 10 cm², or from 0.03 cm² to 9 cm², or from 0.09 cm² to 6.25 cm², or from 3.6 cm² to 4.9 cm².

In one aspect, the applicator may generate treatment spots with an area in a range from 2 cm² to 7 cm², or in a range from 3 cm² to 6 cm², or 4 cm² to 5 cm² to ensure precise energy delivery to the targeted tissue layers.

In one aspect, the applicator may generate treatment spots with an area in a range from 0.01 mm² to 7 cm², or in a range from 0.05 mm² to 6 cm², or 0.1 mm² to 5 cm² to ensure precise energy delivery to the targeted tissue layers.

One or more applicators may include a conductive surface, which may be covered by insulation.

Treated regions may include the face, neck, cleavage, chin, superficial muscular aponeurotic system (SMAS), abdomen, buttocks, thigh, back, breasts, legs, ankles, and more. Treated soft tissue may comprise soft tissue within the close vicinity of the applicator and/or one or more applicators. The invention may have various stimulating effects and may be used to perform facelifts (rhytidectomy), skin tightening, skin rejuvenating, stimulation of collagen and/or lasting production, reduction of the number and/or volume of adipose cells, and others.

In one aspect of the invention, the microwave treatment may be for the localized treatment of a wide range of superficial and mucosal conditions across various anatomical sites. Exemplary indications may include treatment of oral lesions such as aphthous ulcers (aphthae), ENT-related applications (e.g., nasal vestibule, oropharynx, external auditory canal), anorectal disorders (e.g., hemorrhoids, fissures), as well as vaginal mucosa, where microwave-induced controlled thermal stimulation may enhance local immune response and promote antibody production.

The treated soft tissue may be positioned, for example, at a distance from 0 to 20 cm or 0 to 15 cm or 0 to 10 cm, or 0 to 5 cm, or 0 to 2 cm from the applicator or the treatment element. During the treatment, one or more applicators may be located in one or more locations relative to the body regions.

The treated soft tissue may be positioned, for example, at a distance from 0.1 to 20 cm or 0.3 to 10 cm, or 0.5 to 5 cm, or 1 to 2 cm from the applicator or the treatment element. During the treatment, one or more applicators may be located in one or more locations relative to the body regions.

In another aspect, the applicator may be in direct contact with the treated soft tissue (e.g. skin).

The applicator 105 may comprise a protective bio-barrier 803 configured to cover at least the proximal portion of the applicator comprising the contact surface of the applicator, which is in direct contact with the patient. The bio-barrier 803 may be designed to enclose an area corresponding to the treatment spot in a range from 2 cm² to 7 cm², or in a range from 3 cm² to 6 cm², 2 cm² to 5 cm².

In one aspect of the invention, the bio-barrier 803 may extend at least partially toward a distal portion of the applicator.

The bio-barrier may be constructed from biocompatible materials, such as medical-grade silicone, latex, or any other suitable polymers to ensure patient safety during treatment. The bio-barrier 803 may be disposable or reusable, depending on the used biocompatible material.

In addition, the bio-barrier may also be formed from non-polymeric biocompatible materials, including but not limited to glass, ceramics, alumina-based materials, borosilicate, or medical-grade stainless steel, provided that the material allows for hygienic contact, sterilization (if reusable), and may not interfere with the energy transmission characteristics of the applicator.

In one aspect, the bio-barrier may be flexible, partially flexible, or rigid. In another aspect, the bio-barrier 803 may have a flexible part and a rigid part that may be attached to the proximal part of the applicator. The rigid part may create a frame for the flexible part of the bio-barrier 803.

According to some aspects of the invention, microwave treatment may be applied in combination with cooling to reduce the temperature induced in tissue adjacent to the skin surface and/or in the vicinity of the applicator. Heat may be removed from the tissue close to the surface (e.g. skin surface), for example, using a water bolus, fluid circulation, or a thermoelectric cooler, or cooling layer (e.g. cooling gel). Cooling the tissue may allow more microwave power and more microwave energy to be delivered to the soft tissue without causing an unwanted increase in the temperature in the tissue close to the skin surface and damage to the surrounding tissue and/or the applicator. This may allow the application of more microwave energy to the more distal parts of the soft tissue and/or body region into the deeper layers of the soft tissue, thus creating a thermal gradient in the treated region. Cooling the skin's surface may also ensure better soft tissue heating and impedance matching of the waveguide to the soft tissue load.

In one aspect of the invention, the term "permittivity" may be preferably understood and applied as a relative permittivity.

The device and method may provide one or more treatment energies in order to provide treatment to the subject. The treatment energy may comprise microwave energy, radiofrequency energy (RF), vacuum, ultrasonic energy, mechanical wave energy, thermal energy, light energy, plasma, magnetic field, and/or electric current. Different treatment energy may be used individually and/or may be combined. Different treatment elements may be combined in one applicator. Multiple treatment elements may provide various types of EM energies.

According to one aspect of the invention, the applicator may be stationary adjacent to the subject's surface. Moving the applicator or applicators may be preferred in another aspect of the invention. One or more applicators may be moved in a chosen geometric pattern or path. The movement may be simulated by alternately switching on and off the relevant treatment elements without moving the one or more applicators.

The applicator may be, for example, in the form of a handheld applicator or a pad.

The device may have several possible aspects based on invasive and non-invasive methods.

According to one aspect of the invention, the applicator may be in the form of a needle. The needle may penetrate the skin surface and deliver microwave energy to the soft tissue.

The applicator may include at least one antenna providing microwaves. The applicator may be coupled to the subject's body, or the applicator may be located in the subject's body.

The applicator may be created from rigid or at least partly flexible material, which can be adapted to the curve of the subject's body or face surface.

The control unit may be a part of one or more applicators and may be located in the applicator or out of the applicator (e.g. in a main unit).

One or more treatment elements may communicate with each other and/or with the control unit.

An electromagnetic field used for heating the tissue may be a microwave field, typically in a range from 300 MHz to 300 GHz, 400 MHz to 150 GHz, or 500 MHz to 100 GHz, 700 MHz to 70 GHz, 1 GHz to 30 GHz, 1 GHz to 10 GHz. The wavelength λ of the electromagnetic field may range from 1 mm to 1 m, or 2 mm to 75 cm, or 3 mm to 60 cm, or 4.3 mm to 43 cm, or 1 cm to 30 cm, or 3 cm to 30 cm.

An electromagnetic field used for heating the tissue may be a microwave field, typically in a range from 0.5 GHz to 7 GHz, from 1.3 GHz to 4.6 GHz, or from 2 GHz to 5.8 GHz. The wavelength λ of the electromagnetic field may be in a range from 4 cm to 0.6 m, or 6.5 cm to 23 cm, or 5.1 cm to 15 cm.

The frequencies may be within various ISM bands, such as, for example, 26.957 MHz, 40.66 MHz, 433.05 MHz, 902 MHz, 2.4 GHz, 5.725 GHz, 24 GHz, 61 GHz, 122 GHz or 244 GHz.

Specific absorption rate (also called SAR) is a critical parameter that refers to the rate at which the skin and underlying tissues absorb microwave energy, expressed in watts per kilogram (W/kg). The significance of SAR lies in its ability to quantify energy absorption, which directly influences the effectiveness and safety of the microwave treatment.

Under specific conditions, a standing wave may be created as a result of wave interference. The interference may occur when microwave energy is radiated into the adjacent tissue and some of the microwave energy is reflected back. The standing wave may occur because of the resonance phenomenon. The standing wave may result in the decrease of the SAR in the epidermis. The standing wave may result in a shift of the peak SAR to the deeper tissue layers such as the dermis, adipose tissue, fascia layer, SMAS, or muscles. This may result in various beneficial results such as stimulation of collagen production, stimulation of elastin production, wrinkle reduction, tightening and firming of the skin, improving skin texture and tone, acne reduction, body sculpting, skin tightening, cellulite reduction, strengthening of the connective tissue, body contouring, improvement of the body circulation, enhanced lymphatic drainage, contracting the fascia layer, SMAS lifting, facial contouring, muscle toning, muscle relaxation, increased blood flow, reducing pain, enhancing tissue healing, reducing inflammation and others. One or more treatment elements may be in direct contact with the surface of the skin or may be spaced from the subject's body by an air gap, spacing element or spacing material, or cooling element or cooling material.

One or more treatment elements may radiate electromagnetic energy with different frequencies at the same time, or one or more treatment elements may be alternatively switched on/off during the treatment.

In another aspect, one or more treatment elements may radiate electromagnetic energy with the same frequencies at the same time, or one or more treatment elements may be alternatively switched on/off during the treatment.

In one aspect, the applicator may provide a treatment energy to cause heating of the soft tissue within the body region in a range of 37.5 °C to 100 °C , or 37.5 °C to 95 °C or 38 °C to 90 °C, or 38 °C to 80 °C, or from 38 °C to 70 °C, or from 38 °C to 65 °C, or from 38 °C to 60 °C, or 40 °C to 70 °C, or 40 °C to 65 °C, or 42 °C to 50 °C.

In another aspect, the applicator may provide a treatment energy to cause heating of the body region combined with the cooling of the surface of the soft tissue (e.g. the surface of the skin) of the body region, thus creating a thermal reverse gradient in the body region. In that case, the surface of the soft tissue (e.g. the surface of the skin, such as the epidermis) may be cooled to room temperature, or in a range of 5 °C to 70 °C, or 10 °C to 60 °C, or 15 °C to 55 °C, or 20 °C to 50 °C, or 20 °C to 45 °C, or 25 °C to 40 °C or 30°C to 35 °C.

In another case, the surface of the soft tissue (e.g. the surface of the skin, as the epidermis) may be cooled to room temperature, or in a range of 0 °C to 40 °C, or 2 °C to 30 °C, or 5 °C to 25 °C, or 8 °C to 21 °C, or 12 °C to 17 °C, or 13 °C to 15 °C.

The first layer below the surface of the soft tissue, for example, a dermal layer (including at least one of collagen or elastin) may be heated to a temperature in a range of 37.5 °C to 90 °C, or 38 °C to 90 °C, or 42 °C to 90 °C, or 50 °C to 80 °C, or 55 °C to 75 °C, or 57 °C to 72 °C, or 60 °C to 70 °C, or 38 °C to 80 °C, or 38 °C to 70 °C, or 38 °C to 65 °C, or 38 °C to 60 °C, or 45 °C to 70 °C, or 45 °C to 65 °C.

The second layer, which may be even deeper than the first layer from the surface of the soft tissue, for example, SMAS, may be heated to a temperature in the range of 37.5 °C to 90 °C, or 38 °C to 90 °C, or 42 °C to 90 °C, or 50 °C to 80 °C, or 55 °C to 75 °C, or 57 °C to 72 °C, or 60 °C to 70 °C, or 38 °C to 80 °C, or 38 °C to 70 °C, or 38 °C to 65 °C, or 38 °C to 60 °C, or 45 °C to 70 °C, or 45 °C to 65 °C.

In another aspect of the invention, the applicator may provide a treatment energy to cause a heating of the adipose tissue that may be heated to a temperature in a range of 37.5 °C to 90 °C, or 38 °C to 90 °C, or 42 °C to 90 °C, or 50 °C to 80 °C, or 55 °C to 75 °C, or 57 °C to 72 °C, or 60 °C to 70 °C, or 38 °C to 80 °C, or 38 °C to 70 °C, or 38 °C to 65 °C, or 38 °C to 60 °C, or 45 °C to 70 °C, or 45 °C to 65 °C.

The applicator may be configured to provide heating of human skin. The heating may be homogenous and/or not homogenous. The applicator may be configured to provide heating of the epidermis, dermis and/or adipose tissue. The applicator may also be configured to provide heating of fascia, for example superficial, visceral or deep fascia.

The applicator may be configured to provide heating of SMAS.

The applicator may be configured to provide heating of the muscle layer.

In one aspect of the invention, the applicator may be configured to provide heating of the epidermis and/or dermis layers of the skin. This may result in stimulation of collagen production, stimulation of elastin production, wrinkle reduction, tightening and firming of the skin, improving skin texture and tone, acne reduction, skin rejuvenation, and others.

In another aspect of the invention, the applicator may be configured to provide heating of the adipose tissue. This may result in a reduction of fat in targeted areas, body sculpting, skin tightening, cellulite reduction, strengthening of the connective tissue, body sculpting, and body contouring, improvement of body circulation, enhanced lymphatic drainage, and others.

In another aspect of the invention, the applicator may be configured to provide heating of the fascia layer. This may result in lifting and tightening of the skin, contracting the fascia layer, stimulation of collagen and elastin production, facial contouring, minimization of wrinkles and fine lines, and others.

In another aspect of the invention, the applicator may be configured to provide heating of the muscles. This may result in muscle toning, muscle relaxation, increased blood flow, aid after injury or after a workout, reduction of cellulite, reducing pain, enhancing tissue healing, reducing inflammation, body contouring, and others.

In yet another aspect of the invention, the applicator may be configured to provide heating of the epidermis, dermis, and fascia layer. This may result in a combination of the beneficial results in each layer.

In yet another aspect of the invention, the applicator may be configured to provide heating of the epidermis, dermis, fascia layer, and muscle. This may result in a combination of the beneficial results in each layer.

In another aspect the applicator may be configured to provide heating of the SMAS. This may result in lifting and tightening of the SMAS, face lifting, stimulation of collagen and elastin production and others.

In yet another aspect of the invention, the applicator may be configured to provide heating of the epidermis, dermis, and adipose tissue. This may result in a combination of the beneficial results in each layer.

In yet another aspect of the invention, the applicator may be configured to provide heating of the epidermis, dermis, adipose tissue and fascia layer. This may result in a combination of the beneficial results in each layer.

In yet another aspect of the invention, the applicator may be configured to provide heating of the epidermis, dermis, adipose tissue, fascia layer and muscles. This may result in a combination of the beneficial results in each layer.

In yet another aspect of the invention, the applicator may be configured to provide heating of the tissue between adipose tissue and muscles.

In yet another aspect of the invention, the applicator may be configured to provide heating of the tissue between adipose tissue and muscles and parts of the muscles may also be heated.

In yet another aspect of the invention, the applicator may be configured to provide heating of the epidermis, dermis and tissue between adipose tissue and muscles.

In yet another aspect of the invention, the applicator may be configured to provide heating of the epidermis, dermis and tissue between adipose tissue and muscles and parts of the muscles may also be heated.

In yet another aspect of the invention, the applicator may be configured to provide heating of the epidermis, dermis and tissue between adipose tissue and muscles. The cooling may be used to prevent any destructive influence (e.g. apoptosis) of the heating on epidermis, dermis and/or adipose tissue.

In yet another aspect of the invention, the applicator may be configured to provide heating of the epidermis, dermis and tissue between adipose tissue and muscles. The cooling may be used to prevent any destructive influence (e.g. apoptosis) of the heating of the tissue.

In yet another aspect of the invention, the applicator may be configured to provide heating of the epidermis, dermis, fascia layer and muscles. This may result in a combination of the beneficial results in each layer. The applicator may be configured to provide heating of the SMAS. This may result in the stimulation of collagen and elastin production, tightening the skin and SMAS, face lifting, skin rejuvenation, and others, without reduction of adipose tissue and fat deposits.

In yet another aspect, the invention may be configured to provide a peak of treatment energy and heating to the epidermis and dermis. The treatment energy peaks may cause thermal effect in the epidermis and dermis, which may result in fascia contraction, face lifting, skin resurfacing, and create precise microchannels in the tissue and tissue coagulation. The coagulated tissue stimulates a natural healing process, resulting in face lifting, skin tightening, skin rejuvenating, stimulation of collagen and/or elastin production, and other effects.

In yet another aspect, the applicator may be configured to provide a peak of treatment energy and heating to the fascia layer. The treatment energy peaks may cause thermal effect in the fascia, which may result in fascia contraction, face lifting, fascia resurfacing, creating precise microchannels in the tissue and/or causing tissue coagulation in the microchannels and/or their vicinity. The coagulated tissue stimulates a natural healing process, resulting in face lifting, skin tightening, skin rejuvenating, stimulation of collagen and/or elastin production, and other effects.

The applicator may be configured to provide peaks of treatment energy and heating to SMAS.

In one aspect, the applicator may be configured to provide fractional treatment of SMAS.

In yet another aspect, the applicator may be configured to provide a peak of treatment energy and heating to the adipose layer. This may result in targeted fat reduction, skin tightening, body contouring, stimulation of the natural healing processes, and other effects.

The aspects may be combined.

Heating may result in stimulation of collagen production, stimulation of elastin production, wrinkle reduction, tightening and firming of the skin, improving skin texture and tone, and acne reduction.

In another aspect of the invention, the applicator may be configured to provide low-heat energy into the surface of the tissue to prevent and/or treat melanoma cancer, including superficial spreading melanoma, nodular melanoma, lentigo maligna melanoma, acral lentiginous melanoma, amelanotic melanoma.

In another aspect of the invention, the delivery of low-heat energy in a therapeutically effective dose and/or immunostimulatory dose or amount of microwave energy to the tissue may stimulate the immune system of a subject, thereby enhancing the immune response.

The immune response may be localized to the site of microwave energy application or to the site of the disease and/or condition to be treated. Alternatively, the immune response may involve a cell-based immune mechanism, including the induction, proliferation, and/or activation of one or more pathways or mechanisms of the innate immune system. The response may further involve stimulation of immune system cells, such as T cells, and/or the production of cytokines. The stimulated immune response may itself be useful in the treatment and/or prevention of melanoma.

In another aspect of the invention, the delivery of a therapeutically effective and/or immuno stimulatory dose or amount of microwave energy to diseased tissue may induce or elevate the production of one or more heat shock proteins in/or near the tissue, wherein the heat shock proteins may be selected from the group consisting of HSP90, HSP72, HSP70, HSP65. HSP60, HSP27, HSP16, and/or any other heat shock protein.

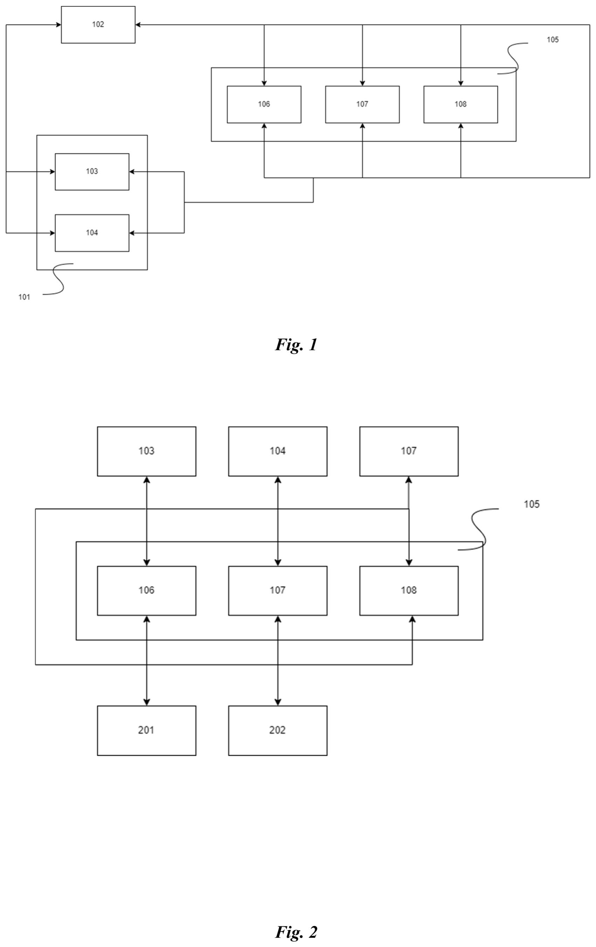

Fig. 1 shows one exemplary schematic diagram of the system. The system may comprise a main unit 101, a power supply 102, a control unit 103, a user interface 104, one or more applicators 105, one or more sensors 106, a cooling unit 107, and one or more treatment arrays 108.

In another aspect of the invention, the system may comprise a main unit 101, a power supply 102, an amplifier 801, a control unit 103, a user interface 104, an applicator 105, a sensor 106, a cooling unit 107, and a treatment element 301.

In one aspect of the invention, the main unit 101 may comprise a power supply 102, an amplifier 801, a control unit 103, and a user interface 104, and an applicator 105 may comprise a sensor 106, a cooling unit 107, and a treatment element 301. In another aspect, the cooling unit 107 may be part of the main unit 101 and the applicator 105.

In one aspect of the invention, the power supply 102 may comprise a generator. In another aspect of the invention, the power supply 102 may comprise a generator and a power plug configured to plug the device into the mains electricity, e.g. a residential or commercial electrical grid. Yet in another aspect of the invention, the power supply 102 may comprise a generator and a battery configured to power the generator. In another aspect of the invention, the power supply 102 may comprise a generator, a power plug and a battery.

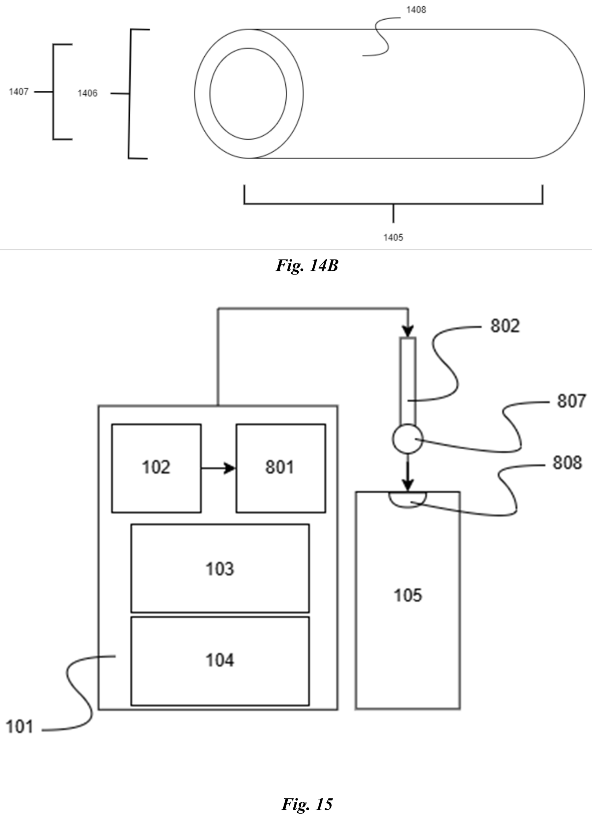

In a further aspect, the system may comprise a main unit 101, a power supply 102 (comprising a generator), an amplifier 801, a control unit 103, a user interface 104, a connecting tube 802, a plug 807, a socket 808, and an applicator 105 depicted in Figure 15.

In one aspect, the main unit 101 may comprise the power supply 102, the amplifier 801, the control unit 103, and the user interface 104, and the applicator 105 may be coupled (e.g., connected) to the main unit 101 by the connecting tube 802.

In one aspect, the main unit 101 may comprise the power supply 102, the amplifier 801, and the control unit 103. The user interface 104 may be connected with the main unit wirelessly, and the applicator 105 may be coupled (e.g. connected) to the main unit 101 by the connecting tube 802.

The power supply 102 may communicate with the control unit 103 and the user interface 104. Regulation of delivered energy may be controlled by the control unit 103. The control unit 103 may also evaluate feedback from one or more sensors 106 and adjust the stimulation parameters accordingly. The control unit 103 may control the treatment parameters, such as treatment time, frequency of provided energy, the amount of delivered energy, the intensity of energy, the shape of the pulses, duration of the pulses, controlling switching on/off different groups of treatment elements, the amount of cooling delivered to the tissue, position of the applicator, suction power, subject's temperature, temperature of the applicator, pulse mode and/or others. The treatment parameters may be chosen from a predetermined group of protocols or may be set by the user. The treatment parameters may be chosen from a range of safe thresholds. The treatment parameters may be adjusted automatically by the user or by the control unit 103 during the treatment. The treatment parameters may be adjusted by the control unit 103 based on the feedback measurements from one or more sensors 106. If the chosen treatment parameters are safe, the treatment may start.

The device may comprise one or more sensors 106. One or more sensors 106 may include a temperature sensor, a sensor for measuring the distance of the applicator from the subject's surface, a flow sensor measuring the flow of the cooling fluid, a pressure sensor, a voltage sensor, a current sensor, a voltage sensor, an impedance sensor, a gyroscope, a biochemical sensor, a biosensor, a thermal camera, a sensor detecting velocity, a sensor detecting movements, a sensor measuring one or more of permittivity, conductivity, inductance, or capacitance, a sensor measuring a value of the electromagnetic field, or a sensor for measuring the contact between the applicator and the skin surface. One or more sensors 106 may be used in an invasive or non-invasive manner.

Tissue temperature may be monitored using one or more temperature sensors. The temperature sensor can be a thermocouple, a resistance thermometer, a thermometer, a thermistor, an optical sensor, or an infrared thermometer. One or more temperature sensors may be used to measure the temperature of the treated tissue, the skin, the skin surface, the applicator, one or more treatment arrays 108, and/or the treatment elements.

The temperature may be evaluated indirectly. Indirect measurement of temperature may be a measure of a different physical parameter other than temperature. Such parameters can, for example, be the impedance of the tissue and/or others.

The position of one or more applicators may be monitored using a position sensor. The position of individual applicators may be monitored, or the position of one applicator in relation to another applicator may be monitored. A position sensor may be, for example, an accelerometer or gyroscope.

In one aspect of the invention, at least one sensor may be located in the main unit of the device.

One or more sensors 106 may serve as feedback and may be in communication with the control unit 103. One or more sensors, 106, may be used to improve the treatment's efficiency and prevent any discomfort and health risks. The treatment parameters may be manually or automatically optimized based on the feedback.

One or more sensors 106 may be configured to ensure safe treatment.

One or more sensors 106 may be a temperature sensor that measures the temperature of the adjacent tissue and/or the adjacent objects (e.g. spacing element, attaching element, clothes, cloth, or others)

The temperature sensor may be configured to provide information about the adjacent object to the control unit 103. The control unit 103 may evaluate the measured temperature and determine whether the adjacent object has the temperature of a human body and whether it is safe to start the treatment. If the measured temperature is a temperature in a range of temperatures of a human body, the treatment may start. If the measured temperature is not in the range of temperatures of a human body, the user and/or operator may be warned and the treatment may not start. If during treatment, the applicator 105 is moved away from the adjacent tissue, it may be indicated by a shift in the measured temperature and evaluated by the control unit 103. If the applicator 105 is moved away from the adjacent tissue during treatment, the user and/or operator may be warned and the treatment may be paused. After the applicator 105 is placed in the correct location in vicinity to the adjacent tissue, the treatment may start again.

One or more sensors 106 may be an impedance sensor that measures the impedance of the adjacent tissue and/or the adjacent objects.

The impedance sensor may be configured to provide information about the adjacent object to the control unit 103. The impedance sensor may be configured to provide information about the coupling and/or contact of the applicator 105 to the adjacent tissue. The impedance sensor may be configured to provide information about the coupling and/or contact of the applicator 105 to the skin. The control unit 103 may evaluate the measured impedance and may evaluate whether the adjacent object has an impedance of a human body and whether it is safe to start the treatment. If the measured impedance is impedance in a range of impedances of a human body, the treatment may start. If the measured impedance is not in a range of impedances of a human body, the user and/or operator may be warned and the treatment may not start. If during treatment, the applicator 105 is moved away from the adjacent tissue, it may be indicated by a shift in the measured impedance and evaluated by the control unit 103. If the applicator 105 is moved away from the adjacent tissue during treatment, the user and/or operator may be warned and the treatment may be paused. After the applicator 105 is placed in the correct location in vicinity to the adjacent tissue, the treatment may start again.

The device may also comprise a cooling unit 107. The cooling unit 107 may obtain a better coupling of the irradiated electromagnetic field with the soft tissue. The cooling unit 107 may also serve for cooling areas affected by superficial heating, prevent the formation of hot spots and overheating of the non-targeted tissue and the tissue adjacent to the skin surface, cool the antenna, and considerably reduce the size of the antenna. The cooling unit 107 may also allow the heating of deeper tissues. The cooling unit 107 may comprise an object filled with a suitable substance, such as fluid, liquid, water, distilled water, deionized water, water-doped NaCl, ethanol, air, CO2, and others. The parameters of the substance, such as temperature, viscosity, impedance, flow, etc., may be monitored with one or more sensors 106. The monitored parameters may provide feedback information to control unit 103.

A cooling unit 107 may include a water bolus, a fluid circulation unit, a spacing element filled with a suitable substance, one or more thermoelectric coolers, one or more Peltier elements, heat conductors, a heat sink, a nozzle spraying a cooling fluid to cool the applicator 105 and/or to cool the target tissue, and others. The cooling unit 107 may be configured to remove heat from the tissue and create a reverse thermal gradient. When a specific temperature gradient is reached, the amount of energy delivered to the soft tissue may be adjusted. Cooling may prevent the tissue from reaching temperatures that may be destructive to the tissue. The device may also comprise a heating unit to heat the tissue. The soft tissue may be cooled and/or heated during the treatment or may be selectively switched on/off during the treatment. The heating/cooling may be administered pre-treatment or post-treatment.

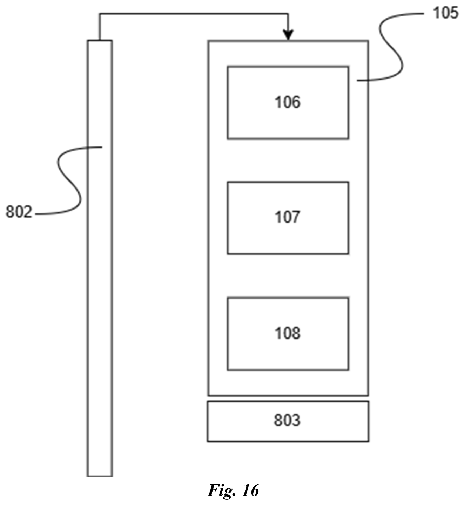

In one aspect of the applicator, the applicator 105 connected to connecting tube 802, may comprise one or more sensors 106, a cooling unit 107, a treatment array 108, and the proximal part of the applicator 105 may comprise bio-barrier 803 depicted in figure 16.

Fig. 2 shows a schematic diagram of one or more applicators 105. One or more applicators 105 may comprise a treatment array 108, one or more sensors 106, and a cooling unit 107. One or more applicators 105 may communicate with the control unit 103 and the user interface 104. The device may include a means of attaching the applicator 105 to the skin surface: the attaching element 201. The attaching element 201 may be a sticky layer, a suction unit using a vacuum, a belt, a band, a felt, a mask, a garment, or other. The device may include one or more spacing elements 202.

The attaching element 201 may be a sticker that may be attached to the contact surface of the applicator 105 and may be able to attach the applicator 105 to the skin's surface. The sticker may also be attached to the subject's body or other device parts. The sticker may have a top side and a bottom side. The bottom side of the sticker may comprise a sticky layer configured to attach the applicator 105 to the skin surface. The top side of the sticker may be bonded to the applicator 105. The sticker may have the same shape and size as the applicator 105 or may be bigger or smaller than the applicator 105. The sticker may have a surface in a range from 1 cm² to 10,000 cm², or from 1 cm² to 1,000 cm², or from 1 cm² to 500 cm², or from 1 cm² to 100 cm², or from 10 cm² to 100 cm².

The attaching element 201 may be a belt designed to fit any type and size of the treated subject's body area. The belt may be used to attach the applicator 105 to the subject's body. The belt may be in contact with the subject's body surface and may be made from a rigid or at least partially flexible material. The belt may be configured to hold one or more applicators 105 in contact with the subject's body. The belt may comprise a fastening mechanism, such as, for example, snap, clamp, rails, zipper, holes, Velcro, and others. One or more applicators 105 may have various sizes and shapes.

The attaching element 201 may be in communication with a vacuum unit, and the applicator 105 may be attached to the subject's body region (e.g. face) by a vacuum created by negative pressure.

The device may include one or more spacing elements 202. The spacing element 202 may be provided between the applicator 105 and/or one or more treatment arrays 108 and the subject's body. The spacing element 202 may improve electromagnetic energy transfer to the soft tissue. Various materials may be included in the spacing element 202 having a suitable dielectric constant and impedance and may be used to improve energy transfer. The spacing element 202 may also prevent any harmful influence on the energy delivery and may prevent overheating the tissue adjacent to the skin surface. The spacing element 202 may provide cooling to the skin surface, adjacent tissue, applicator 105, treatment array 108 and/or treatment elements. The spacing element 202 may be in communication with the cooling unit 107. In one aspect, the spacing element 202 and the attaching element 201 may be one element.

The spacing element 202 may be detachable. The spacing element 202 may be replaceable. The spacing element 202 may be a single-use element or can be used multiple times. The spacing element 202 may be changed prior to the treatment, during the treatment, and/or post-treatment.

The spacing element 202 may be made from a material that provides a good impedance matching of the applicator 105 to the skin surface. The spacing element 202 may be made of various materials, such as, for example, water-based gels, conductive gels, metals, silicone-based compounds, polymers, elastomers, ceramics, hydrogels or saline solutions, other dielectric or insulating materials (e.g. aluminum nitride), and others. The spacing element 202 may be made of a combination of the listed materials.

In some aspects, the spacing element 202 may comprise the same material as a material of the treatment element, for example the same ceramics that is used in the ceramic waveguide.

In some aspects, the spacing element 202 may be flexible, partially flexible, or rigid. In other aspects, the spacing element 202 may have a flexible part and a rigid part that may be attached to the proximal part of the applicator. The rigid part may create a frame for the flexible part of the spacing element 202.

The spacing element 202 may be disposable and may ensure biological compatibility, safety, and sterility and may be configured to prevent cross-contamination.

The spacing element 202 may be cooled. The spacing element 202 may be in communication with the cooling unit 107. The spacing element 202 may be cooled by a cooling element. The cooling element may include a cooling means, such as one or more Peltier elements, one or more heat sinks, a cooling fluid, a heat conductor, and others. The spacing element 202 may be cooled by a circulating fluid, e.g. the cooling fluid. The spacing element 202 may be cooled by a nozzle spraying the cooling fluid. The cooling fluid may be a liquid or a gas. The cooling fluid may be air, oxygen, water, ethanol, oil, or any combination of these fluids.

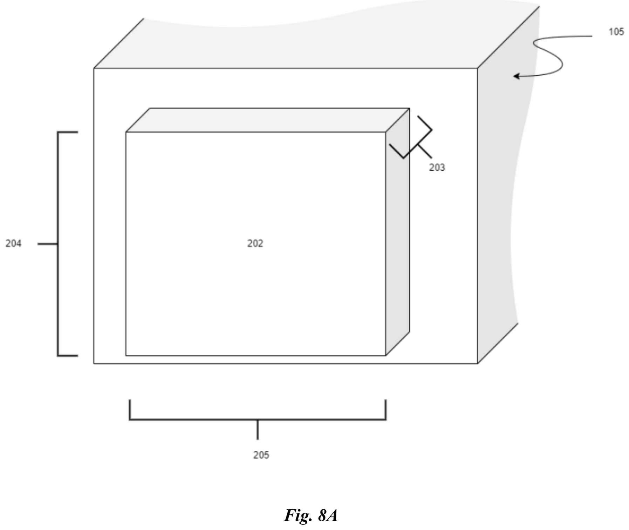

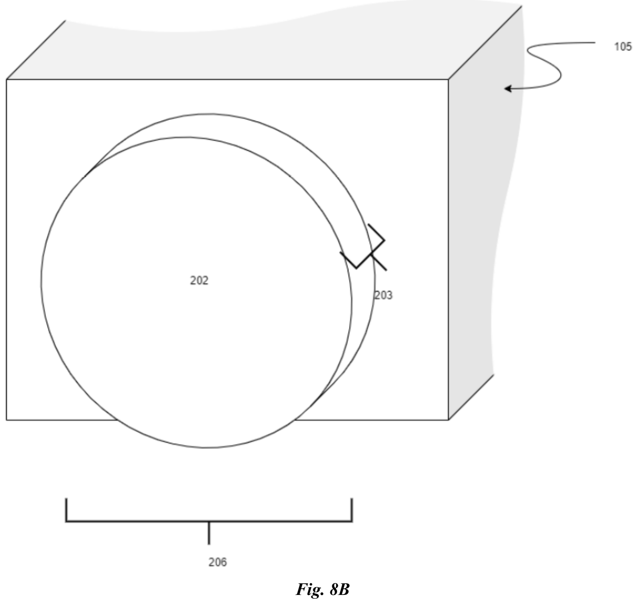

Figures 8A-B show schematic diagrams of the spacing element 202. The spacing element 202 may have, for example, a rectangular shape, circular shape, oval shape or square shape. The spacing element 202 may have a thickness 203, height 204 and width 205. The spacing element may have a diameter 206.

In some aspects, the spacing element have any cross-sectional shape suitable for microwave propagation, including but not limited to a rectangular, square, circular, oblong or oval shape, an elliptical shape, a ridged or double-ridged rectangular shape, a ridged circular shape, a coaxial or annular (ring-like) shape, a racetrack or rounded-rectangular shape, a trapezoidal or sectoral shape, or a composite cross-section formed by straight and curved segments, for example chamfered-polygonal or partially corrugated profiles, provided that the resulting geometry supports the desired microwave mode structure and impedance characteristics and allows mechanical integration with the surrounding components.

In some aspects, the spacing element may have a general shape, e.g. a free-form shape defined by its outer boundary, the boundary not conforming to a regular polygonal or circular geometry, suitable for maintaining a gap between the applicator and the tissue surface.

In some aspects, the cross section of the shaping element may have a smooth, continuously curved perimeter, thereby avoiding angular transitions. The cross section of the shaping element may be formed by smoothly connected curved surfaces, such as circular, elliptical, or rounded-rectangular profiles.

The spacing element 202 may have a thickness 203 in a range from 0.001 mm to 100 mm, or from 0.01 mm to 20 mm, or from 0.01 mm to 10 mm, or from 0.05 mm to 1 mm, or from 0.1 mm to 1 mm, or from 0.2 mm to 1 mm, or from 0.2 mm to 0.5 mm, or from 0.5 mm to 1 mm, or from 1 mm to 10 mm, or from 1 mm to 5 mm, or from 1.5 mm to 4 mm, or from 2 mm to 4 mm.

In some aspects, the spacing element 202 may have a thickness 203 in a range from 0.1 mm to 10 mm, or from 0.2 mm to 7 mm, or from 0.5 mm to 3.5 mm, or from 0.8 mm to 1.8 mm.

The spacing element 202 may have a height 204 in range from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1· 10⁻²mm , or from 1·10⁻⁶mm to 1·10⁻³mm , or from 1·10⁻⁶mm to 1·10⁻⁵mm , or from 1 mm to 1000 mm, or from 100 mm to 1000 mm, or from 500 mm to 1000 mm, or from 1 · 10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻³mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 1 mm to 50 mm, or from 2 mm to 15 mm, or from 5 mm to 50 mm, or from 5 mm to 30 mm, or from 0.5 mm to 5 mm, or from 1 mm to 5 mm.

In some aspects, the spacing element 202 may have a height 204 in range from 5 mm to 35 mm, or from 10 mm to 30 mm, or from 15 mm to 25 mm, or from 17 mm to 23 mm, or from 18 mm to 22 mm, or from 19 mm to 21 mm, or from 19.5 mm to 20.5 mm.

The spacing element 202 may have a width 205 in range from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1· 10⁻²mm , or from 1·10⁻⁶mm to 1·10⁻³mm , or from 1·10⁻⁶mm to 1·10⁻⁵mm , or from 1 mm to 1000 mm, or from 100 mm to 1000 mm, or from 500 mm to 1000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻³mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 1 mm to 50 mm, or from 2 mm to 15 mm, or from 5 mm to 50 mm, or from 5 mm to 30 mm, or from 0.5 mm to 5 mm, or from 1 mm to 5 mm.

In some aspects, the spacing element 202 may have a width 205 in range from from 5 mm to 35 mm, or from 10 mm to 30 mm, or from 15 mm to 25 mm, or from 17 mm to 23 mm, or from 18 mm to 22 mm, or from 19 mm to 21 mm, or from 19.5 mm to 20.5 mm.

The spacing element 202 may have a diameter 206 in range from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻³mm , or from 1 mm to 1000 mm, or from 100 mm to 1000 mm, or from 500 mm to 1000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 1 mm to 50 mm, or from 2 mm to 15 mm, or from 5 mm to 50 mm, or from 5 mm to 30 mm, or from 0.5 mm to 5 mm, or from 1 mm to 5 mm.

In some aspects, the spacing element 202 may have a diameter 206 in range from 5 mm to 35 mm, or from 10 mm to 30 mm, or from 15 mm to 25 mm, or from 17 mm to 23 mm, or from 18 mm to 22 mm, or from 19 mm to 21 mm, or from 19.5 mm to 20.5 mm.

The spacing element 202 may be characterized by its permittivity. The permittivity may depend on the frequency, magnitude, direction of the applied field, humidity, temperature and other parameters.

The spacing element 202 may be characterized by its relative permittivity. Relative permittivity is the permittivity of a material expressed as a ratio with the electric permittivity of a vacuum. The spacing element may have a relative permittivity from 1 to 150, or from 1 to 50, or from 1 to 25, or from 1 to 15, or from 1 to 12, or from 1 to 10, or from 1 to 7, or from 2 to 6, or from 2 to 5, each range accommodating the unique electrical characteristics required for specific applications. The spacing element 202 may be characterized by its dielectric strength. The dielectric strength of the spacing element 202 may be in a range from 0.1 MV/m to 100 MV/m, or from 5 MV/m to 50 MV/m, or from 10 MV/m to 60 MV/m, or from 15 MV/m to 30 MV/m, or from 20 MV/m to 24 MV/m, or from 24 MV/m to 28 MV/m, or from 10 MV/m to 30 MV/m, or from 15 MV/m to 30 MV/m, or from 24 MV/m to 30 MV/m, or from 20 MV/m to 35 MV/m. This range encompasses the dielectric strengths of various materials that could be used to make the spacing element, ensuring compatibility with different application requirements.

The spacing element 202 may be in direct contact with the treatment array 108, and/or the spacing element may be spaced from the treatment array 108 by, for example, air and/or vacuum.

In one aspect of the invention, the spacing element may be a shaping element.

In another aspect of the invention, the device may comprise a shaping element positioned on or integrated with the part of the treatment element or the treatment array that is in contact with the patient within the applicator. In another aspect of the invention, the shaping element may be used to improve field homogeneity, reduce the influence of metallic walls, reduce reflected waves, or perform any other process.

The shaping element may include a dielectric plate (e.g. ceramics), low-loss dielectric materials, absorbing materials or any other appropriate material. In one aspect of the invention, the shaping element may be made from the same material as the treatment element and/or from a different material.

The shaping element may also be utilized in a disposable part of the applicator, enhancing its functionality and ensuring hygiene for single-use applications. In one aspect of the invention, the shaping element may be characterized by various dimensions depending on the specific application and ergonomic requirements. The shaping element may have a length in a range of 5 mm to 70 mm, or in a range of 15 mm to 50 mm, or in a range of 25 mm to 35 mm.

In one aspect of the shaping element, a length of shaping element may be in a range of 0.5 cm to 5 cm, or in a range of 1 cm to 3.5 cm, or in a range of 1.5 cm to 3 cm, or in a range of 1.7 cm to 2.8

In one aspect of the shaping element, a width of shaping element may be in a range of 5 mm to 70 mm, or in a range of 15 mm to 50 mm, or in a range of 25 mm to 35 mm.

In one aspect of the shaping element, a width of shaping element may be in a range of 0.5 cm to 5 cm, or in a range of 1 cm to 3.5 cm, or in a range of 1.5 cm to 3 cm, or in a range of 1.7 cm to 2.8 cm.

In one aspect of the shaping element, a thickness of the shaping element may be in a range of 0.5 mm to 5 mm, or in a range of 1 mm to 4 mm, or in a range of 2 mm to 3 mm.

In one aspect of the shaping element, a thickness of the shaping element may be in a range of or in a range of 0.01 mm to 5 mm, or in a range of 0.05 mm to 4 mm, 0.1 mm to 3 mm, or in a range of 1 mm to 2 mm, or in a range of 1.1 mm to 1.9 mm, or in a range of 1.3 mm to 1.7 mm.

In one aspect of the shaping element, a thickness of the shaping element may be in a range of 0.01 mm to 1 mm, or in a range of 0.05 mm to 0.8 mm, or in a range of 0.1 mm to 0.5 mm, or in a range of 0.15 mm to 0.35 mm.

In one aspect of the invention, the applicator may comprise a treatment element configured to generate and/or emit microwave energy for microwave treatment. The treatment element may be at least partially coupled to, connected to, surrounded by, or bounded by a shaping element, which may be configured for homogeneity of the resulting thermal field and/or SAR pattern in the patient's tissue and thereby also homogeneity of the resulting heat distribution. Without the shaping element, the emitted microwave energy may produce non-uniform heating patterns, including localized hot spots on the surface of the treatment element and/or on the patient's tissue. The shaping element may be configured to absorb and redistribute excess microwave energy, particularly at peripheral or high-intensity regions of the field, thereby equalizing the emitted field and smoothing thermal gradients so as to provide a more uniform and controlled heat distribution across the treatment area. The shaping element may be arranged to extend laterally beyond at least one side, and optionally beyond opposite (e,g, both longer sides) of the treatment element aperture or active region of the treatment element.

In some aspects, the shaping element may be provided at the patient-facing side of the microwave waveguide, i.e. at the aperture through which the microwave field is emitted.

In some aspects, the shaping element may extend across and fully cover the aperture surface of the treatment element (e.g. waveguide).

In other aspects, the shaping element may extend at least partially across the surface of the treatment element (e.g. waveguide) aperture, e.g. along the perimeter of the treatment element (e.g. waveguide).

In some aspects, the shaping element may have a lateral extension that is greater than the cross-sectional dimension of the treatment element (e.g. waveguide) in one or more lateral directions or in all directions.

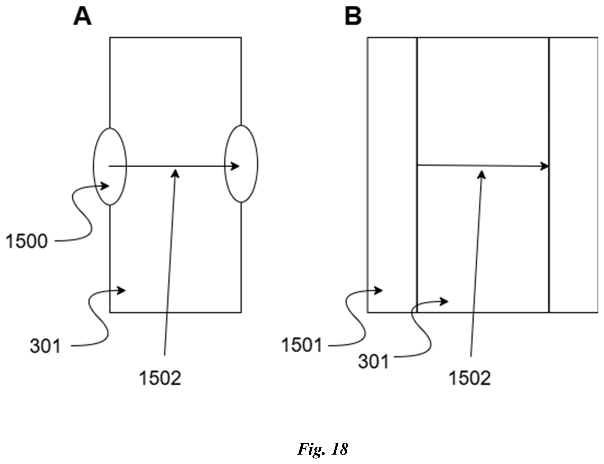

In some aspects, the shaping element may extend across and fully covers the aperture surface of the treatment element (e.g. waveguide), and has a lateral extent that is greater than the cross-sectional dimension of the treatment element (e.g. waveguide) in one or more directions on the sides transversal to the electric field vector 1502 of the transmitted microwave domain mode, as illustrated schematically in Fig. 18B. By extending beyond the treatment element (e.g. waveguide) cross-section in the directions on the sides transversal to the electric field vector 1502, the shaping element modifies the field distribution at the surface of and/or in the patient's tissue and conditions the microwave field prior to interaction with the patient's tissue, while maintaining full coverage of the aperture to ensure controlled and uniform field delivery.

In some aspects, the shaping element may comprise 1 to 15, or 1 to 12, or 1 to 10, or 2 to 10, or 2 to 8, or 2 to 6, or 2 to 4, or at least 2, or exactly 2 lateral extents. The at least two lateral extents may be positioned on the opposite side of the waveguide aperture.

In one aspect of the rectangular treatment element (e.g. waveguide), the lateral extent of the shaping element may be on opposite long sides of the treatment element. In another aspect of the rectangular treatment element (e.g. waveguide), the lateral extent of the shaping element may be on opposite short sides of the treatment element. Yet in another aspect of the rectangular treatment element (e.g. waveguide), the lateral extent of the shaping element may be on all sides of the treatment element.

Figs. 18A and 18B schematically show a view from the aperture side of the treatment element. Fig. 18A shows the treatment element 301 configured to emit microwave energy with generated hot spots 1500 along the perimeter sides of the treatment element transverse to the electric field vector 1502 of the transmitted microwave domain mode in the absence of the extended shaping element 1501. Fig. 18B shows the treatment element 301 configured to emit microwave energy surrounded by the shaping element 1501 resulting in a more uniform field distribution.

In some aspects, the shaping element may have a lateral extent that is greater than the cross-sectional dimension of the treatment element (e.g. waveguide) in a range of from 1.5 mm to 3.5 mm, or from 1.8 mm to 3.0 mm, or from 2.0 mm to 3.0 mm, or from 2.0 mm to 2.8 mm, or from 2.0 mm to 2.5 mm, or from 0.1 mm to 10 mm, or from 0.5 mm to 8 mm, or from 1 mm to 6 mm, or from 1 mm to 5 mm, or from 1.5 mm to 4 mm, or from 1 mm to 7 mm, or from 2 mm to 6 mm, or from 3 mm to 5 mm per side.

In some aspects, the ratio between the lateral width of the shaping element and the lateral width of the treatment element is in a range of from 1.1 to 1.5, or from 1.2 to 1.5, or from 1.25 to 1.4, or from 1.3 to 1.4, or from 1.2 to 1.4.

In some aspects, the ratio between the lateral extent of the shaping element (per side) and the width of the treatment element is in a range of from 0.10 to 0.25, or from 0.12 to 0.22, or from 0.15 to 0.20, or from 0.16 to 0.18, or from 0.165 to 0.175, or from 0.05 to 0.20, or from 0.10 to 0.15, or from 0.11 to 0.14, or from 0.12 to 0.13, or from 0.123 to 0.127.

In some aspects, the ratio between the lateral extent of the shaping element (pre side) and its thickness is in a range of from 120% to 250%, or from 130% to 220%, or from 140% to 200%, or from 150% to 180%, or from 160% to 170%.

In some aspects, the ratio between the total lateral extent of the shaping element on two opposite sides and the thickness of the shaping element is in a range of from 200% to 400%, or from 225% to 350%, or from 250% to 330%, or from 260% to 340%, or from 265% to 335%.

In some aspects, the ratio between the thickness of the shaping element and the length of the treatment element is in a range of from 1% to 5%, or from 1.5% to 4%, or from 2% to 3.5%, or from 2.5% to 3.0%, or from 2.6% to 2.8%.

In one aspect of the invention, the shaping element and/or the treatment element may be provided with one or more dielectric cover layers, configured to further influence and shape the electromagnetic field distribution. The dielectric cover layer may be uniform or non-uniform in thickness, and may be selected to provide graded dielectric properties along the surface of the treatment area. The thickness of the dielectric cover layer may be in a range from 0.1 mm to 5 mm, or from 0.5 mm to 2 mm, depending on the desired modulation of field intensity and the thermal properties of the interface.

Suitable materials for the dielectric cover layer include, but are not limited to, PTFE, silicone, ceramics, PEEK, polyimides, and fluoropolymers, optionally filled or layered with other dielectric or thermally conductive materials. In some embodiments, the cover layer may be configured to attenuate field peaks, improve impedance matching, or redistribute microwave energy from high-intensity zones toward peripheral areas, thereby enhancing the homogeneity of the thermal profile within the target tissue.

To improve robustness and minimize unintended field concentration, the shaping element and/or treatment element may be geometrically optimized. In particular, sharp edges, abrupt corners, or discontinuities along the periphery of the treatment element are preferably avoided, as such features may result in a uniformed microwave field.

In one aspect, the shaping element may be characterized by a relative permittivity (εr) in a range from 2 to 120, or from 5 to 80, or from 10 to 50, or from 13 to 35, or from 15 to 27, and a dielectric loss tangent (tan δ) in a range from 0.0001 to 0.05, depending on the operating frequency and desired field shaping effect.

The shaping element may have a thermal conductivity in a range from 0.1 W/m·K to 10 W/m-K, or from 0.5 to 5 W/m-K, and a heat capacity in a range from 0.5 to 2.0 J/g-K, thereby acting as a thermal buffer to dissipate concentrated thermal energy and prevent localized overheating.

The shaping element may have a flat, domed, curved, angled, or stepped geometry, depending on the anatomy of the treatment area and desired field distribution. In some cases, the shaping element may comprise multiple segments, chambers, or layered structures with different dielectric or thermal properties.

In one aspect of the invention, the shaping element may comprise a surface coating, such as a microwave-transparent polymer, non-stick layer, or antibacterial surface treatment, particularly when intended for mucosal or reusable applications.

In one aspect of the invention, the shaping element may be made from the same material as the treatment element, or from a material having similar electromagnetic, thermal, or mechanical properties.

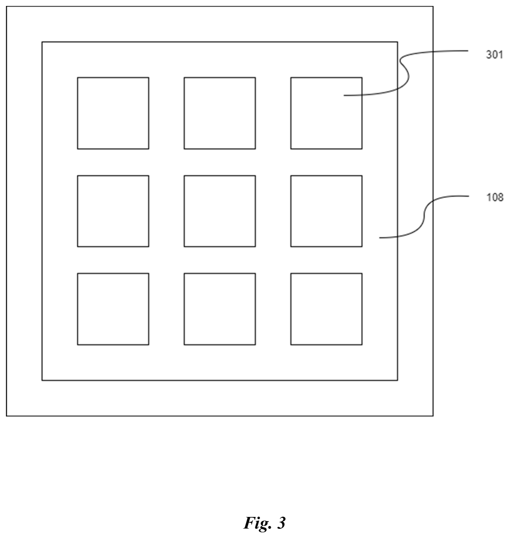

Fig. 3 shows a schematic diagram of one or more treatment arrays 108. One or more treatment arrays 108 may comprise one or more treatment elements 301. The applicator 105 may comprise at least one treatment element 301. One or more treatment arrays 108 may comprise one or more layers. The treatment element 301 may be for example a microwave antenna, SIW antenna, waveguide, electrode (e.g. radiofrequency electrode), light source, ultrasound source, or others.

In one aspect of the invention, the treatment array 108 may comprise two or more treatment elements 301.

In another aspect of the invention, the applicator may comprise one treatment element 301.

In another aspect of the invention, at least one treatment array 108 may be configured to simultaneously activate treatment elements 301 within the treatment array 108.

In another aspect of the invention, at least one treatment array 108 may be configured for the sequential activation of treatment elements 301 within the treatment array 108. For example, one treatment element 301 may be activated first, followed by the activation of a second treatment element 301, and subsequently by the activation of additional treatment elements 301 in sequence.

In another aspect of the invention, the treatment elements 301 may be combined into groups, and at least one treatment array 108 may be configured for the sequential activation of treatment elements groups within the treatment array 108. For example, one group of treatment elements 301 may be activated first, followed by the activation of a second group of treatment elements 301, and subsequently by the activation of additional groups of treatment elements 301 in sequence, while one group of treatment elements 301 may comprise 1 to 10 treatment elements 301, or 2 to 8 treatment elements 301, or 2 to 6 treatment elements 301, or 2 to 4 treatment elements 301.

The applicator 105 may include one or more treatment elements 301, which provide microwave energy to the soft tissue. The applicator 105 may include 1 to 300 treatment elements, 1 to 100 treatment elements, 1 to 50 treatment elements, 1 to 40 treatment elements, 1 to 25 treatment elements, 1 to 16 treatment elements, 1 to 9 treatment elements, or 1 to 4 treatment elements, or 1 to 2 treatment elements, or 2 to 100 treatment elements, or 2 to 40 treatment elements, or 2 to 16 treatment elements, or 2 to 8 treatment elements, or 3 to 6 treatment elements. The treatment elements may be arranged in the form of a matrix or in the form of an array, or in any other form, such as in a circle, ellipsoid, etc. The matrix may have dimensions of 16x16, 10x10, 6x6, 5x5, 4x4, 3x3, or 2x2 treatment elements. One or more treatment elements 301 may form a treatment array 108 comprising multiple identical or non-identical treatment elements 301.

The length of the treatment element 301 may be in a range from 0.1 mm to 1000 mm, or from 1 mm to 500 mm, or from 50 mm to 500 mm, or from 50 mm to 250 mm, or from 1 mm to 100 mm, or from 1 mm to 75 mm, or from 1 mm to 50 mm, or from 1 mm to 25 mm, or from 1 mm to 20 mm, or from 1 mm to 15 mm, or from 1 mm to 10 mm, or from 10 mm, to 50 mm, or from 10 mm to 30 mm, or from 20 mm to 80 mm, or from 20 mm to 50 mm, or from 5 mm to 10 mm, or from 0.1 mm to 10 mm.

The width of the treatment element 301 may be in a range from 0.1 mm to 1000 mm , or from 1 mm to 500 mm, or from 50 mm to 500 mm, or from 50 mm to 250 mm, or from 1 mm to 100 mm, or from 1 mm to 75 mm, or from 1 mm to 50 mm, or from 1 mm to 25 mm, or from 1 mm to 20 mm, or from 1 mm to 15 mm, or from 1 mm to 10 mm, or from 10 mm, to 50 mm, or from 10 mm to 30 mm, or from 20 mm to 80 mm, or from 20 mm to 50 mm, or from 5 mm to 10 mm. , or from 0.1 mm to 10 mm.

The thickness of the treatment element 301 may be in a range from 0.001 mm to 100 mm, or from 0.01 mm to 10 mm, or from 0.05 mm to 1 mm, or from 0.1 mm to 1 mm, or from 0.2 mm to 1 mm, or from 0.2 mm to 0.5 mm, or from 0.5 mm to 1 mm, or from 1 mm to 10 mm, or from 1 mm to 5 mm, or from 1.5 mm to 4 mm, or from 2 mm to 4 mm, or from 2 mm to 15 mm, or from 10 mm to 15 mm.

The treatment element 301 may comprise a surface area in a range from 0.01 mm² to 1 ·10⁶ mm², or from 0.01 mm² to 1 ·10,000 mm², or from 1 mm² to 1 ·10,000 mm², or from 1 mm² to 2500 mm², or from 4 mm² to 500 mm², or from 4 mm² to 225 mm², or from 25 mm² to 2500 mm², or from 25 mm² to 1000 mm², or from 25 mm² to 900 mm², 0.25 mm² to 25 mm², or from 1 mm² to 25 mm², or from 1000 mm² to 5000 mm², or from 1000 mm² to 4000 mm², or from 1500 mm² to 3000 mm², or from 2000 mm² to 2500 mm², or from 2200 mm² to 2400 mm².

The treatment element 301 may comprise a volume in a range from 1 · 10⁻⁵ mm³ to 1 · 10⁸ mm³, or from 1 · 10⁻⁴ mm³ to 1 · 10⁵ mm³, or from 0.05 mm³ to 1 · 10,000 mm³, or from 0.1 mm³ to 10,000 mm³, or from 1 mm³ to 7500 mm³, or from 1 mm³ to 5000 mm³, or from 1 mm³ to 2500 mm³, or from 1 mm to 1000 mm³, or from 1000 to 5000, or from 100 mm³ to 10,000 mm³, or from 1000 mm³ to 10,000 mm³, or from 5000 mm³ to 10,000 mm³, or from 2500 mm³ to 8000 mm³, or 5000 mm³ to 50,000 mm³, or from 10,000 mm³ to 40,000 mm³, or from 25,000 mm³ to 35,000 mm³, or from 3000 mm³ to 10,000 mm³, or from 4000 mm³ to 9000 mm³, or from 5000 mm³ to 9000 mm³, or from 7000 mm³ to 8000 mm³, or from 7500 mm³ to 8000 mm³.

A treatment element 301 may comprise various energy sources, such as, for example, an antenna, electrode, light source, ultrasound source, or other. The antenna may be rigid, at least partially flexible, or flexible. The antenna may be a dipole antenna, patch antenna, biconical antenna, turnstile antenna, multi-dipole antenna, bow-tie antenna, ring antenna, choke dipole antenna, modified dipole antenna, spiral antenna, helical antenna, monopole antenna, loop antenna, horn antenna, or pyramidal horn antenna, waveguide antenna and/or others.

Furthermore, a treatment element 301 may comprise SIW, or microstrip antenna.

The treatment array 108 may comprise one or more antennas which may have various shapes, properties, and dimensions. One or more antennas may output a signal with identical or various parameters, such as frequency and power.

One or more antennas may be made out of a conductive material. One or more antennas may be made out of a material with conductivity in a range from 1 S/m to 7·10⁷ S/m, or from 1·10⁵ S/m to 7·10⁷ S/m, or from 1.5·10⁷ S/m to 6.5·10⁷ S/m at 20 °C. The antenna may be made from, for example, aluminum, brass, silver, copper, gold, annealed copper, stainless steel, iron, nickel, graphene, or other materials.

One or more antennas may be arranged in the form of an array and may form a treatment array 108. One or more antennas may have a function of phase shift or steering to improve the distribution of heat in the soft tissue. A fixed phase shift may be assigned to one or more antennas or pairs of antennas may be shifted together or a group of antennas may be shifted together. A fixed angle may be assigned to one or more antennas or pairs of antennas, or a group of antennas may be rotated according to a predetermined pattern. The adjacent antennas may have various spacing distances. The spacing distance may determine the phase difference between the two adjacent antennas. The change of phase may determine the radiation maxima and peaks and the radiation pattern of the array.

The controlling of the phase and relative amplitude of the treatment array 108 to achieve various radiating patterns, constructive interference at particular angles, and destructive interference at other angles is called beamforming. Beamforming is used to output optimal signal transmission for a certain application.

The treatment array 108 may comprise narrow beam antennas and/or wider beam antennas. The treatment array 108 may comprise narrow bandwidth antennas and/or wider bandwidth antennas. The combination of various types of antennas and stimulation signals may result in various treatment methods and modes. Both surface skin cells and deeper layers of the skin may be affected by the radiation. The treatment may affect both epidermal and dermal layers of the skin. The narrow bandwidth antennas may be used to model various radiation patterns, resulting in radiation maxima and radiation minima. This may result in the formation of lesions in the soft tissue. Wide bandwidth antennas may stimulate and/or heat deeper layers of the skin, which may stimulate the production of collagen and/or elastin.

A treatment array 108 may comprise one or more waveguides 1408. A waveguide 1408 typically comprises a hollow metallic tube, which can have a quadrangle, rectangular, oblong, circular, conic, square with rounded edges, or other cross-sectional shape. The interior of this tube acts as a conduit for electromagnetic waves. The walls of the waveguide 1408, made of a conductive material like metal, confine the electromagnetic waves inside and guide them along its length.

In one aspect, the waveguide 1408 may have all its sides metalized except the side facing the patient, e.g., around the output of the applicator.

The operation of the waveguide 1408 is based on supporting certain modes of wave propagation that depend on its shape and size. These modes dictate the electromagnetic field distribution within the waveguide 1408. Crucial to its function is the waveguide's 1408 dimensions, such as width and height in a rectangular waveguide 1408, which determine its cutoff frequency. This frequency is the threshold below which the waveguide 1408 cannot effectively transmit signals.

Above this cutoff frequency, the waveguide 1408 becomes an efficient medium for guiding electromagnetic waves, primarily through the process of total internal reflection within its conductive walls. The material and thickness of these walls can influence the waveguide's 1408 performance, affecting power loss and signal attenuation. In treatment array 108, the waveguide 1408 is used to transport microwave signals across various frequencies.

The waveguide 1408 may be made out of a material with good electrical conductivity, such as copper, aluminum, brass, silver, gold, and others.

The electrical conductivity of the waveguide material may be in range from 4 ·10⁶ S/m to 7 ·10⁷ S/m, or from 5 ·10⁶ S/m to 7 ·10⁷ S/m, or from 7 ·10⁶ S/m to 7 ·10⁷ S/m, or from 9 ·10⁶ S/m to 7 ·10⁷ S/m, or from 1 ·10⁷ S/m to 7 ·10⁷ S/m, or from 1.5 ·10⁷ S/m to 6.5 ·10⁷ S/m, or from 1.60 ·10⁷ S/m to 2 ·10⁷ S/m, or from 3.3 ·10⁷ S/m to 4 ·10⁷ S/m, or from 3.7 ·10⁷ S/m to 4 ·10⁷ S/m, or from 4 ·10⁷ S/m to 4.5 ·10⁷ S/m, or from 5 ·10⁷ S/m to 6 ·10⁷ S/m, or from 5.9 ·10⁷ S/m to 6 ·10⁷ S/m, or from 6 ·10⁷ S/m to 6.4 ·10⁷ S/m.

The waveguide 1408 may be hollow, partially loaded, or completely loaded with a waveguide 1408 loading material. The waveguide 1408 loading material may be, for example, air, pressurized air, or any dielectric material (e.g., ceramics).

In some aspects, the loading material may be water.·

In one aspect of the waveguide, the waveguide loading material may be made of a dielectric material. The dielectric material may be a composition of various materials, such as, for example, ceramics, polycarbonate, polyethylene, polypropylene, glass fiber, or epoxy composite with copper foil laminated on one or both sides (e.g. FR-4), Rogers materials, polytetrafluoroethylene (PTFE) (e.g. Teflon), Ceramic-filled PTFE composites, and Polyimide, glass-filled PTFE composites (e.g. Duroid), or any other appropriate material suitable for loading material. The waveguide loading material may be characterized by its relative permittivity. The waveguide loading material may have a relative permittivity from 1 to 150, from 1 to 50, from 1 to 25, from 1 to 15, from 1 to 12, from 1 to 10, from 1 to 7, from 2 to 6, or from 2 to 5.

In some aspects, the waveguide loading material may have a relative permittivity from 12 to 30, from 15 to 27, from 16 to 26, from 17 to 25, from 18 to 24, from 19 to 23, from 20 to 22, from 20.5 to 21.5.

The waveguide loading material may be characterized by its dielectric strength. The dielectric strength of the waveguide loading material may be in a range from 0.1 MV/m to 100 MV/m, or from 5 MV/m to 50 MV/m, or from 10 MV/m to 60 MV/m, or from 15 MV/m to 30 MV/m, or from 20 MV/m to 24 MV/m, or from 24 MV/m to 28 MV/m, or from 10 MV/m to 30 MV/m, or from 15 MV/m to 30 MV/m, or from 24 MV/m to 30 MV/m, or from 20 MV/m to 35 MV/m.

The waveguide 1408 may be a rectangular waveguide, circular waveguide, elliptical waveguide, cylindrical, flexible waveguide, dielectric waveguide, planar waveguide, slab waveguide, strip waveguide, metal pipe waveguide, photonic crystal waveguide, single-ridged waveguide, double-ridged waveguide, parallel plate waveguide, single mode waveguide, dual mode waveguide, orthogonal mode waveguide, square waveguide, single mode circular waveguide, dual-mode circular waveguide, single mode rectangular waveguide, dual mode rectangular waveguide, any combination thereof, and others. The waveguide 1408 may be curved inwards, curved outwards, stepped, tapered, or have any other curved profile.

In one aspect, the device may contain at least one applicator 105.

In another aspect, the device may contain two or more applicators 105. Each applicator 105 may comprise different components and may have different treatment parameters.

For example, the device may comprise at least one applicator 105. The at least one applicator 105 may be configured to treat at least one body part. The at least one applicator 105 may comprise at least one treatment element 301. The choice of the applicator 105 and the treatment element 301 may depend on the type of the body part and on the specific parameters of the body part, such as for example the thickness of the individual layers, such as for example skin, fat and muscle within the body part.

For example, the first applicator 105 may be configured to treat the first body part (e.g. cheeks). The second applicator 105 may be configured to treat the second body part (e.g. forehead). The first applicator 105 may comprise one type of treatment element 301. The second applicator 105 may comprise another type of treatment element 301.

For example, the device may comprise a first applicator 105 and a second applicator 105. The first applicator 105 may comprise a first waveguide 1408. The second applicator 105 may comprise a second waveguide 1408. The first waveguide 1408 may be a different type of waveguide than the second waveguide 1408. Each applicator 105 may be configured to treat a different body part.

The first waveguide 1408 may be the same type of waveguide as the second waveguide 1408.

In one aspect of the device, the first applicator 105 may be configured to treat cheeks. The second applicator 105 may be configured to treat the forehead. The first applicator 105 may comprise a circular waveguide. The second applicator 105 may comprise a rectangular waveguide.

In another aspect of the device, the first applicator 105 may be configured to treat cheeks. The second applicator 105 may be configured to treat the forehead. The first applicator 105 may comprise a dual-mode circular waveguide. The second applicator 105 may comprise a single mode rectangular waveguide.

In another aspect of the invention, the device may comprise three applicators 105. The device may comprise a first applicator 105 comprising a first treatment element 301, a second applicator 105 comprising a second treatment element 301 and a third applicator 105 comprising a third treatment element 301, wherein the first, the second and the third treatment element 301 are each different. The first applicator 105 may be configured to treat the cheeks. The second applicator 105 may be configured to treat the forehead. The third applicator 105 may be configured to treat the neck.

In another aspect of the device, the device may comprise at least one detachable part. The detachable part might contain the treatment array 108 and/or the at least one treatment element 301. The detachable part may comprise at least one antenna, at least one waveguide 1408 or at least one SIW antenna 501. Instead of changing the applicators 105 in between the treatments and using the first applicator 105 to treat the first body part and the second applicator 105 to treat the second body part, only the detachable part needs to be switched between the treatments. Yet in another aspect, the device may comprise a first detachable part and a second detachable part. The first detachable part may comprise the first treatment element 301 and the second detachable part may comprise the second treatment element 301. The detachable parts may be reusable or single-use. Each of the detachable parts may comprise specific parameters and may be configured to treat a specific body part.

In one aspect, the applicator may comprise the at least one detachable part.

In one aspect, the detachable part may comprise bio-barrier 803. In another aspect, the detachable part may comprise a bio-barrier 803 and at least one treatment element 301.

In one aspect, the device may utilize a connection by a connecting tube to the applicator, wherein the applicator 105 may be detachable from the connecting tube 802, thereby not requiring an individual connecting tube. This aspect of the invention may simplify operation by allowing the connecting tube 802 to remain connected to the main unit 101 while only the applicator 105 may be exchanged as needed. Thus it may be possible to have the connecting tube 802 coupled to the main unit during the treatment and only exchange the applicators 105 as needed. For example a first applicator with a smaller spot may be used for treatment of facial area, while a second applicator with larger spot may be used for large body areas like abdomen or buttocks. Thus, if necessary, it may be possible to exchange only the first applicator by the second applicator instead of changing the whole applicator with the connecting tube.

Alternatively, in another aspect of the invention, the applicator 105 itself may comprise its connecting tube allowing for independent connection to the main unit 101 of the device. In one aspect, a plurality of applicators having respective connecting tubes may be connected to the main unit 101 at the same time. In another aspect a first applicator 105 having the connecting tube 802 may be removably connected to the main unit 101 at a given time, while different applicator may be removably connected to the main unit 101 at a different time by disconnecting the connecting tube of the first applicator from the main unit 101 and connecting the connecting tube of the second applicator.

The connecting tube may comprise a cable, e.g. coaxial cable, hybrid cable, fiber optic cable, high-voltage power cable, instrumentation cable, or any other suitable cable. In another aspect of the invention, the connecting tube may include a cable and an integrated cooling unit 107 (e.g. some parts of cooling unit 107 like a tubing system) for cooling purposes, enabling simultaneous energy transfer and cooling functionality. Yet in another aspect of the invention, the connecting tube may comprise a cable, an integrated cooling unit 107 (e.g. some parts of cooling unit 107 like a tubing system) and additional transmission lines (e.g. link between a control unit inside the main unit and a sensor inside or on the applicator).

In one aspect of the invention, the applicator 105 may be permanently affixed to the connecting tube 802.

In another aspect of the invention, the applicator 105 may be detachable from the connecting tube 802.

In one aspect, the connecting tube 802 may comprise at least one plug 807, and the applicator may comprise at least one socket 808 (or vice versa) to secure and efficient connection between the connecting tube 802 and the applicator 105, thus between the main unit 101 and the applicator 105. This mechanism may also communicate with additional systems, such as sensors 106 embedded within the applicator or the cooling unit 107, providing feedback to the control unit for optimized operation.

In one aspect, the connecting tube 802 may be affixed to the main unit.

When the connecting tube is connected to the applicator, the cable may link the treatment element with the generator or the amplifier. The cooling unit 107 (e.g. some parts of cooling unit 107 like a tubing system) inside the connecting tube may link the cooling unit 107 (e.g. some parts of cooling unit 107 like a tubing system) inside the applicator 105 with the cooling unit (e.g. some parts of cooling unit 107 like a tubing system, a pump or a fluid container) inside the main unit 101.

In another aspect, the connecting tube 802 may comprise at least one socket 808, and the applicator 105 may comprise at least one plug 807 for a secure and efficient connection between the main unit 101 and the applicator 105. This mechanism may also communicate with additional systems, such as sensors 106 embedded within the applicator or the cooling unit 107, providing feedback to the control unit for optimized operation.

In another aspect of the device, the device may comprise one applicator 105. The applicator 105 may comprise at least one treatment element 301. The operator may choose a predefined treatment protocol with predefined treatment parameters and/or choose the treatment parameters directly. The predefined treatment protocol and/or the range of safe treatment parameters that the operator may choose from may be optimized based on the body part (such as, for example, a forehead, cheeks, neck and others) that the treatment will be performed on. The treatment parameters may include at least the temperature of the applicator 105, the temperature of the treated soft tissue, the temperature of the microwave antenna, the impedance of the microwave antenna, treatment time, rate of cooling, the temperature of the cooling unit 107, the temperature of the cooling fluid, the output energy and/or output power and pressure. Treatment parameters may be changed before the treatment or during the treatment. The software may include one or more predefined protocols, from which the operator may choose the most suitable one. The operator may also create his own protocol, e.g. via a user interface 104.

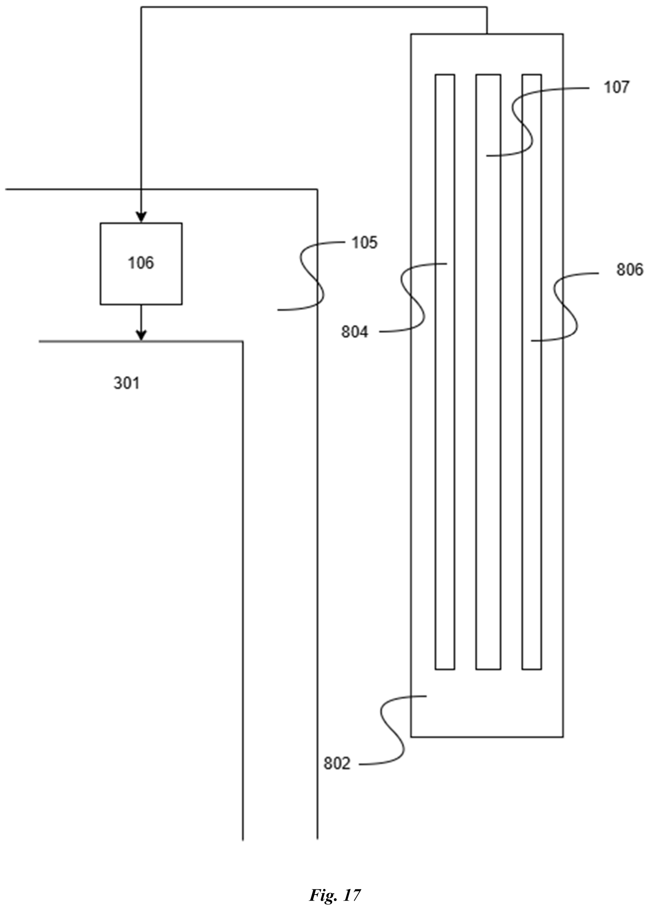

In one aspect of the invention, the connecting tube 802 may comprise cooling unit 107, vacuum circuit 804, and cable 806 as shown in Fig. 17.

In another aspect of the invention, the connecting tube 802 may comprise a cable 806, a cooling unit 107 (e.g. some parts of cooling unit 107 like a tubing system), a vacuum circuit 804 and additional transmition lines (e.g. connection between a control unit inside the main unit and a sensor inside or on the applicator).

In another aspect of the invention, at least one sensor 106 may be in a close location to the feeding point of the treatment element 301 of the applicator 105 as shown in Fig. 17.

In one aspect of the invention, the device may comprise a vacuum source, wherein the vacuum source may be configured to generate a vacuum. The vacuum source may communicate with the main unit 101 of the device. The connecting tube 802 may connect the main unit 101 with the applicator 105, wherein the connecting tube 802 may comprise cooling unit 107 and/or a vacuum circuit 804. The vacuum circuit 804 may be configured to transport the vacuum generated by the vacuum source to the applicator 105. The vacuum circuit 804 may include tubing or channels designed to maintain the vacuum pressure, ensuring efficient energy transfer and secure attachment of the applicator to the treatment area. Additionally, the vacuum circuit 804 may be integrated with control mechanisms to regulate vacuum levels and optimize performance for different applications.

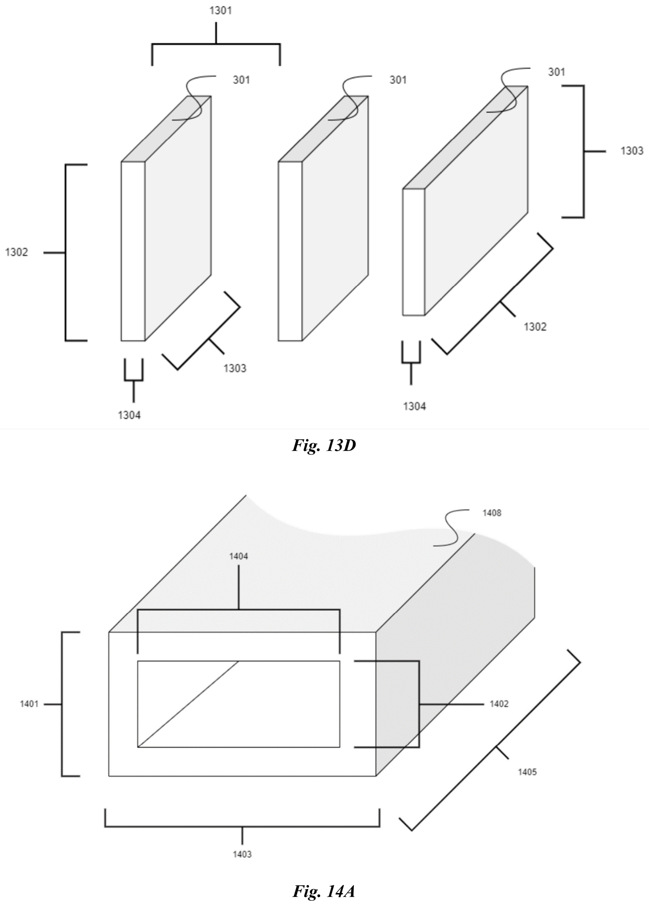

Figures 14A-B show exemplary diagrams of the waveguide 1408.

The waveguide 1408 may comprise an outer height 1401 and an inner height 1402.

The waveguide 1408 may comprise an outer width 1403 and an inner width 1404.

The waveguide may comprise a length 1405.

The waveguide 1408 may comprise an outer diameter 1406 and an inner diameter 1407.

The outer height 1401 of the waveguide 1408 may be in a range from 1·10⁻⁶mm to 2000 mm, or from 1·10⁻⁶mm to 1500 mm, or from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻³mm , or from 1·10⁻⁵mm to 2000 mm, or from 1·10⁻³mm to 2000 mm, or from 1·10⁻³mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1 · 10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm.

The inner height 1402 of the waveguide 1408 may be in a range from 1·10⁻⁶mm to 2000 mm, or from 1·10⁻⁶mm to 1500 mm, or from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻³mm , or from 1·10⁻⁵mm to 2000 mm, or from 1·10⁻³mm to 2000 mm, or from 1·10⁻³mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1 · 10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm.

The outer width 1403 of the waveguide 1408 may be in a range from 1·10⁻⁶mm to 2000 mm, or from 1· 10⁻⁶mm to 1500 mm, or from 1·10⁻⁶mm to 1000 mm, or from 1· 10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻⁵mm , or from 1·10⁻⁵mm to 2000 mm, or from 1·10⁻³mm to 2000 mm, or from 1·10⁻²mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1·10⁻³mm to 500 mm, or from 1·10⁻³mm to 200 mm, or from 1 · 10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm.

The inner width 1404 of the waveguide 1408 may be in a range from 1·10⁻⁶mm to 2000 mm, or from 1· 10⁻⁶mm to 1500 mm, or from 1·10⁻⁶mm to 1000 mm, or from 1· 10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻⁵mm , or from 1·10⁻³mm to 2000 mm, or from 1·10⁻³mm to 2000 mm, or from 1·10⁻²mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm.

The length 1405 of the waveguide 1408 may be in a range from 1·10⁻⁶mm to 2000 mm, or from 1·10⁻⁶mm to 1500 mm, or from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10 ⁶mm to 1·10⁻⁵mm , or from 1·10⁻⁵mm to 2000 mm, or from 1·10⁻³mm to 2000 mm, or from 1·10⁻²mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1·10⁻³mm to 500 mm, or from 1·10⁻³mm to 200 mm, or from 1·10⁻³mm to 100 mm, or from 1·10⁻³mm to 10 mm, or from 0.1 mm to 200 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm.

The outer diameter 1406 of the waveguide 1408 may be in a range from 1·10⁻⁶mm to 2000 mm, or from 1·10⁻⁶mm to 1500 mm, or from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻³mm , or from 1·10⁻⁵mm to 2000 mm, or from 1·10⁻³mm to 2000 mm, or from 1· 10⁻²mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm.

The inner diameter 1407 of the waveguide 1408 may be in a range from 1·10⁻⁶mm to 2000 mm, or from 1·10⁻⁶mm to 1500 mm, or from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻³mm , or from 1·10⁻⁶mm to 1·10⁻³mm , or from 1·10⁻⁶mm to 1·10⁻³mm , or from 1·10⁻⁵mm to 2000 mm, or from 1·10⁻³mm to 2000 mm, or from 1·10⁻³mm to 2000 mm, or from 1 mm to 2000 mm, or from 5 mm to 2000 mm, or from 10 mm, to 2000 mm, or from 50 mm to 2000 mm, or from 100 mm to 2000 mm, or from 500 mm to 2000 mm, or from 1000 mm to 2000 mm, or from 1500 mm to 2000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻²mm to 200 mm, or from 1·10⁻¹mm to 100 mm, or from 1 mm to 100 mm, or from 1 mm to 75 mm, or from 1 mm to 50 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm.

The outer height and the outer width may have a ratio, for example, in a range from 1:1 to 1:10, or from 1:1 to 1:9, or from 1:1 to 1:8, or from 1:1 to 1:7, or from 1:1 to 1:6, or from 1:1 to 1:5, or from 1:1 to 1:4, or from 1:1 to 1:3, or from 1:1 to 1:2, or from 2:3 to 2:9, or from 3:4 to 3:7, or from 4:5 to 4:9, or from 5:7 to 5:9, or from 7:8 to 7:10, or from 8:9 to 9:10, or from 1:1 to 3:5, or from 3:5 to 1:2.

The outer height and the outer width may have a ratio, for example 1: 1, or 1:2, or 1:3, or 1:4, or 1:5, or 1:6, or 1:7, or 1:8, or 1:9, or 1:10, or 2:3 ,or 2:5, or 2:7, or 2:9, or 3:4, or 3:5, or 3:7, or 3:10, or 4:5, or 4:7, or 4:9, or 5:6, or 5:7, or 5:8, or 5:9, or 6:7, or 7:8, or 7:9, or 7:10, or 8:9, or 9:10.

The outer width and the outer height may have a ratio, for example in a range from 1:1 to 1:10, or from 1:1 to 1:9, or from 1:1 to 1:8, or from 1:1 to 1:7, or from 1:1 to 1:6, or from 1:1 to 1:5, or from 1:1 to 1:4, or from 1:1 to 1:3, or from 1:1 to 1:2, or from 2:3 to 2:9, or from 3:4 to 3:7, or from 4:5 to 4:9, or from 5:7 to 5:9, or from 7:8 to 7:10, or from 8:9 to 9:10, or from 1:1 to 3:5, or from 3:5 to 1:2.

The outer width and the outer height may have a ratio, for example 1:1, or 1:2, or 1:3, or 1:4, or 1:5, or 1:6, or 1:7, or 1:8, or 1:9, or 1:10, or 2:3 ,or 2:5, or 2:7, or 2:9, or 3:4, or 3:5, or 3:7, or 3:10, or 4:5, or 4:7, or 4:9, or 5:6, or 5:7, or 5:8, or 5:9, or 6:7, or 7:8, or 7:9, or 7:10, or 8:9, or 9:10.

The inner height and the inner width may have a ratio for example in a range from 1:1 to 1:10, or from 1:1 to 1:9, or from 1:1 to 1:8, or from 1:1 to 1:7, or from 1:1 to 1:6, or from 1:1 to 1:5, or from 1:1 to 1:4, or from 1:1 to 1:3, or from 1:1 to 1:2, or from 2:3 to 2:9, or from 3:4 to 3:7, or from 4:5 to 4:9, or from 5:7 to 5:9, or from 7:8 to 7:10, or from 8:9 to 9:10, or from 1:1 to 3:5, or from 3:5 to 1:2.

The inner height and the inner width may have a ratio, for example 1: 1, or 1:2, or 1:3, or 1:4, or 1:5, or 1:6, or 1:7, or 1:8, or 1:9, or 1:10, or 2:3 ,or 2:5, or 2:7, or 2:9, or 3:4, or 3:5, or 3:7, or 3:10, or 4:5, or 4:7, or 4:9, or 5:6, or 5:7, or 5:8, or 5:9, or 6:7, or 7:8, or 7:9, or 7:10, or 8:9, or 9:10.

The inner width and the inner height may have a ratio for example in a range from 1:1 to 1:10, or from 1:1 to 1:9, or from 1:1 to 1:8, or from 1:1 to 1:7, or from 1:1 to 1:6, or from 1:1 to 1:5, or from 1:1 to 1:4, or from 1:1 to 1:3, or from 1:1 to 1:2, or from 2:3 to 2:9, or from 3:4 to 3:7, or from 4:5 to 4:9, or from 5:7 to 5:9, or from 7:8 to 7:10, or from 8:9 to 9:10, or from 1:1 to 3:5, or from 3:5 to 1:2.

The inner width and the inner height may have a ratio, for example 1: 1, or 1:2, or 1:3, or 1:4, or 1:5, or 1:6, or 1:7, or 1:8, or 1:9, or 1:10, or 2:3 ,or 2:5, or 2:7, or 2:9, or 3:4, or 3:5, or 3:7, or 3:10, or 4:5, or 4:7, or 4:9, or 5:6, or 5:7, or 5:8, or 5:9, or 6:7, or 7:8, or 7:9, or 7:10, or 8:9, or 9:10.

A spacing element 202 may be positioned between the waveguide 1408 and the adjacent tissue.

The spacing element 202 may have the same height 204 as the outer height 1401 of the waveguide 1408, or the height 204 of the spacing element 202 may be larger than the outer height 1401 of the waveguide 1408, or the height 204 of the spacing element 202 may be smaller than the outer height 1401 of the waveguide 1408. For example, the difference between the height 204 of the spacing element 202 and the outer height 1401 of the waveguide 1408 may be in a range from 0 to 0.001, or from 0.001 mm to 100 mm, or from 0.001 mm to 50 mm, or from 0.001 mm to 25 mm, or from 0.001 mm to 10 mm, or from 0.001 mm to 5 mm, or from 0.001 mm to 2 mm, or from 0.001 mm to 1 mm, or from 0.001 mm to 0.1 mm, or from 0.001 mm to 0.01 mm, or from 0.1 mm to 10 mm, or from 1 mm to 10 mm.

The spacing element 202 may have the same width 205 as the outer width 1403 of the waveguide 1408, or the width 205 of the spacing element 202 may be larger than the outer width 1403 of the waveguide 1408, or the width 205 of the spacing element 202 may be smaller than the outer width 1403 of the waveguide 1408. For example, the difference between the width 205 of the spacing element 202 and the outer width 1403 of the waveguide 1408 may be in a range from 0 to 0.001, or from 0.001 mm to 100 mm, or from 0.001 mm to 50 mm, or from 0.001 mm to 25 mm, or from 0.001 mm to 10 mm, or from 0.001 mm to 5 mm, or from 0.001 mm to 2 mm, or from 0.001 mm to 1 mm, or from 0.001 mm to 0.1 mm, or from 0.001 mm to 0.01 mm, or from 0.1 mm to 10 mm, or from 1 mm to 10 mm.

The spacing element 202 may have the same diameter 206 as the outer diameter 1406 of the waveguide 1408, or the diameter 206 of the spacing element 202 may be larger than the outer diameter 1406 of the waveguide 1408, or the diameter 206 of the spacing element 202 may be smaller than the outer diameter 1406 of the waveguide 1408. For example, the difference between the diameter 206 of the spacing element 202 and the outer diameter 1406 of the waveguide 1408 may be in a range from 0 to 0.001, or from 0.001 mm to 100 mm, or from 0.001 mm to 50 mm, or from 0.001 mm to 25 mm, or from 0.001 mm to 10 mm, or from 0.001 mm to 5 mm, or from 0.001 mm to 2 mm, or from 0.001 mm to 1 mm, or from 0.001 mm to 0.1 mm, or from 0.001 mm to 0.01 mm, or from 0.1 mm to 10 mm, or from 1 mm to 10 mm.

The spacing element 202 may be disposable and may ensure biological compatibility, safety, and sterility and may be configured to prevent cross-contamination.

The waveguide 1408 may have a power in a range from 0.001 Watt to 10,000 Watt, or from 0.001 Watt to 1000 Watt, or from 0.001 Watt to 750 Watt, or from 1 Watt to 500 Watt, or from 1 Watt to 400 Watt, or from 1 Watt to 300 Watt, or from 1 Watt to 250 Watt, or from 1 Watt to 200 Watt, or from 1 Watt to 150 Watt, or from 1 Watt to 100 Watt, or from 1 Watt to 85 Watt, or from 1 Watt to 75 Watt, or from 1 Watt to 50 Watt, or from 1 Watt to 40 Watt, or from 1 Watt to 30 Watt, or from 1 Watt to 20 Watt, or from 1 Watt to 11 Watt, or from 5 Watt to 150 Watt, or from 5 Watt to 125 Watts or from 5 Watt to 100 Watt, or from 5 Watt to 90 Watt.

The device may comprise at least one waveguide 1408.

In another aspect, the device may comprise a plurality of waveguides 1408.

The at least two waveguides 1408 in the treatment array 108 may be positioned in applicator 105 in various configurations, depending on the intended use of the device.

The waveguide 1408 may be fed by at least one feeding point. The waveguide 1408 may comprise a first end and a second end. The position of the feeding point, for example its distance from the first end and/or the second end, or the waveguide wall that the feeding point is coupled to, may influence the parameters of the radiation. The parameters of the radiation may be, for example, radiation wavelength, radiation loss, size of the radiation field, distribution of the radiation field, mode excitation, impedance matching, and/or efficiency. The feeding point may be positioned anywhere on the waveguide. The feeding point may be, for example, positioned on the wall of the waveguide that is parallel to the spacing element 202, and/or on the walls of the waveguide that are perpendicular to the spacing element 202. The position of the feeding point also plays a significant role in determining which mode is excited in the waveguide. The position of the feeding point may also have an influence on whether a single mode or a dual mode is excited in the waveguide 1408.

The waveguide 1408 may comprise at least two feeding points. The parameters of the radiation may be influenced by feeding the waveguide 1408 by at least two feeding points. Feeding by at least two feeding points may allow for control of mode excitation. Feeding by at least two feeding points may have an influence on whether a single mode or a dual mode is excited in the waveguide 1408. Also, the position of the at least two feeding points may influence the parameters of the radiation.

In one aspect of the invention, the waveguide 1408 may comprise a post. In another aspect, the number of posts may range from 2 to 10, or 2 to 7, or 2 to 5, or 2 to 4. In one configuration, the posts may extend completely through the waveguide, while in another configuration, the posts may partially extend into the waveguide without penetrating through its entirety.

In another aspect of the invention, the waveguide 1408 may comprise tuning elements that may be used for the impedance matching, resonance tuning, suppressing reflecting waves, model control, frequency filtering, adjustment of standing wave patterns, controlling power distribution, directing radiation patterns, phase adjustment, impedance transformation, enhancing system efficiency or any other related process.

In another aspect of the invention, the tuning element may be selected from the tuning pins, tuning probes, tuning screws, adjustable stubs, iris plates, dielectric posts, sliding shorts, capacitive couplers, inductive couplers, waveguide inserts, flaps, ferrite elements or any other tuning element.

A tuning element may be inserted in a post. In one aspect the tuning element may be fixedly attached in the post. In another aspect, the tuning element may be loosely inserted in the post and may be configured to slide in and out of the post for fine-tuning.

A treatment element 301 may be a waveguide 1408. The treatment array 108 may comprise at least one treatment element 301.

In another aspect, the treatment array may comprise more than one treatment element 301.

A treatment array 108 may comprise one or more microstrip antennas. The microstrip antenna may comprise a metallic patch on top of a grounded substrate. The patch may have a flat rectangular, circular, ellipsoid or other shape.

The patch and the ground plane may form a resonant structure with a particular resonant frequency, which is determined by the dimensions of the patch and the properties of the substrate. The radiation pattern formed by the microstrip antenna may be unidirectional or bidirectional, depending on the design, and is typically broadside to the patch.

The patch may be made out of a material with good electrical conductivity, such as copper, aluminum, brass, silver, gold, and others.

The electrical conductivity of the patch material may be in a range from 4 ·10⁶ S/m to 7 ·10⁷ S/m, or from 5 ·10⁶ S/m to 7 ·10⁷ S/m, or from 7 ·10⁶ S/m to 7 ·10⁷ S/m, or from 9 ·10⁶ S/m to 7 ·10⁷ S/m, or from 1 ·10⁷ S/m to 7 ·10⁷ S/m, or from 1.5 ·10⁷ S/m to 6.5 ·10⁷ S/m, or from 1.60 ·10⁷ S/m to 2 ·10⁷ S/m, or from 3.3 ·10⁷ S/m to 4 ·10⁷ S/m, or from 3.7 ·10⁷ S/m to 4 ·10⁷ S/m, or from 4 ·10⁷ S/m to 4.5 ·10⁷ S/m, or from 5 ·10⁷ S/m to 6 ·10⁷ S/m, or from 5.9 ·10⁷ S/m to 6 ·10⁷ S/m, or from 6 ·10⁷ S/m to 6.4 ·10⁷ S/m.

The substrate of the microstrip antenna may be made out of a dielectric material. The dielectric material may be a composition of various materials, such as, for example, ceramics, polycarbonate, polyethylene, polypropylene, glass fiber or epoxy composite with copper foil laminated on one or both sides (e.g. FR-4), Rogers materials, polytetrafluoroethylene (PTFE) (e.g. Teflon), Ceramic-filled PTFE composites, and Polyimide, glass-filled PTFE composites (e.g. Duroid), and other materials suitable for a microstrip.

The substrate of the microstrip antenna may be characterized by its relative permittivity. The substrate may have a relative permittivity from 1 to 150, or from 1 to 50, or from 1 to 25, or from 1 to 15, or from 1 to 12, or from 1 to 10 or from 1 to 7, or from 2 to 6, or from 2 to 5.

The substrate of the microstrip may be characterized by its dielectric strength. The dielectric strength of the microstrip substrate may be in a range from 0.1 MV/m to 100 MV/m, or from 5 MV/m to 50 MV/m, or from 10 MV/m to 60 MV/m, or from 15 MV/m to 30 MV/m, or from 20 MV/m to 24 MV/m, or from 24 MV/m to 28 MV/m, or from 10 MV/m to 30 MV/m, or from 15 MV/m to 30 MV/m, or from 24 MV/m to 30 MV/m, or from 20 MV/m to 35 MV/m.

The choice of the substrate of the microstrip may have a great influence on the parameters of the radiation, such as, for example, the radiation wavelength, radiation loss, size of the radiation field, size of the hot spots and others.

A treatment element 301 may be a microstrip antenna. The treatment array 108 may comprise at least one treatment element 301. The treatment array may comprise more than one treatment elements 301.

A treatment array 108 may comprise one or more SIW antennas. The SIW antenna may comprise a top metallic layer, a bottom metallic layer, a dielectric substrate and at least one post.

In one aspect, the SIW antenna may comprise the dielectric layer covered by the top metallic layer on a first side (e.g. top side) and the bottom metallic layer on a second side - an opposite side to the first side (e.g. bottom side). Thus the SIW antenna may comprise the dielectric layer sandwiched between the top metallic side and the bottom metallic side. In another aspect, the SIW antenna may comprise one or more additional layers between the top metallic side and the dielectric layer or between the bottom metallic side and the dielectric layer, wherein the one or more additional layers may comprise dielectric layers or conductive layers or their combinations.

The post may comprise a via, a pin, a hole, a cutout or other. The post (e.g. via) may form an interconnection between the top metallic plate and the bottom metallic plate, e.g. the post may be a hole in the dielectric layer between the top and bottom metallic plates. The interconnection may be metalized, thus creating electrical interconnection between the top metallic plate and the bottom metallic plate. The posts (e.g. vias) may be the source of the microwaves. The position of the at least one post may influence the parameters of the microwave radiation.

In one aspect of the post, the interconnection between the top metallic plate and the bottom metallic plate may be non-metalized.

In one aspect of the invention, at least one part of the treatment element may remain non-metalized. For example, the part facing the output of the the applicator may be non-metalized.

In one aspect of the invention, the metalized parts of the treatment element may be covered by a cover layer configured to prevent oxidation. The material used for the cover layer may be selected from the group of polytetrafluoroethylene (PTFE), polyimide, epoxy resins, alumina, zirconia, silicon nitride, anodized aluminium, chromium oxide, or any other suitable material to prevent oxidation of the metalized parts of treatment element.

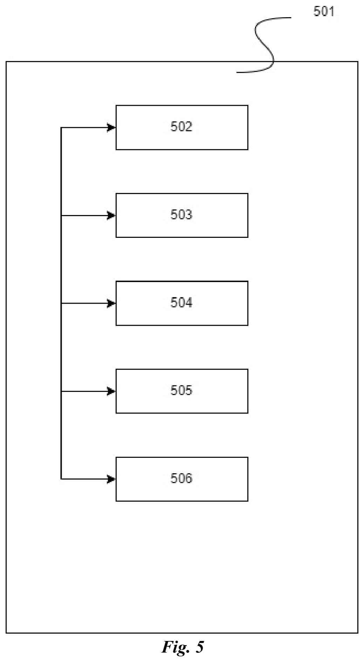

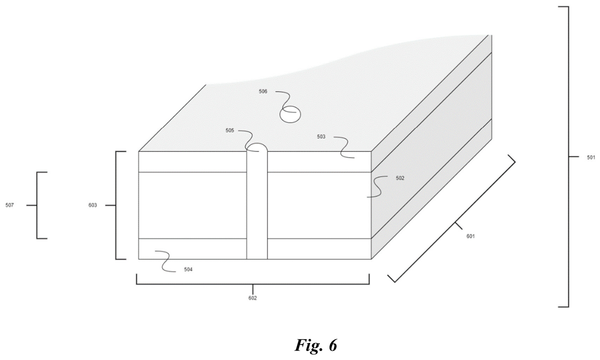

Figures 5 and 6 show an exemplary diagram of the SIW antenna 501. The SIW antenna 501 may comprise a substrate 502, a top metallic layer 503, a bottom metallic layer 504, and at least one post 505 and a feeding point 506.

The feeding point 506 may be integrated into the substrate 502, and the current applied to the feeding point 506 may flow to the SIW antenna 501. The power transfer to the feeding point 506 is improved by an impedance matching. The SIW antenna 501 may comprise a first end and a second end. The position of the feeding point 506, for example, its distance from the first end and/or the second end may influence the parameters of the radiation. The parameters of the radiation may be for example radiation wavelength, radiation loss, size of the radiation field, distribution of the radiation field, mode excitation, impedance matching, and/or efficiency.

In one aspect of the invention, the treatment element may comprise one or more feeding points. The number of feeding points for the treatment element may be in a range of 1 to 8, in a range of 3 to 6, or in a range of 4 to 5. The higher number of feeding points may result in improved field uniformity, enhanced power handling capacity, reduced reflections, and impedance matching, and reduced system losses. In one aspect the treatment element may comprise two feeding points. Feeding by the two feeding points may have an influence on whether a single mode or a dual mode is excited in the treatment element. Also, the position of the at least two feeding points may influence the parameters of the radiation.

The SIW antenna 501 may comprise a length 601, width 602 and thickness 603.

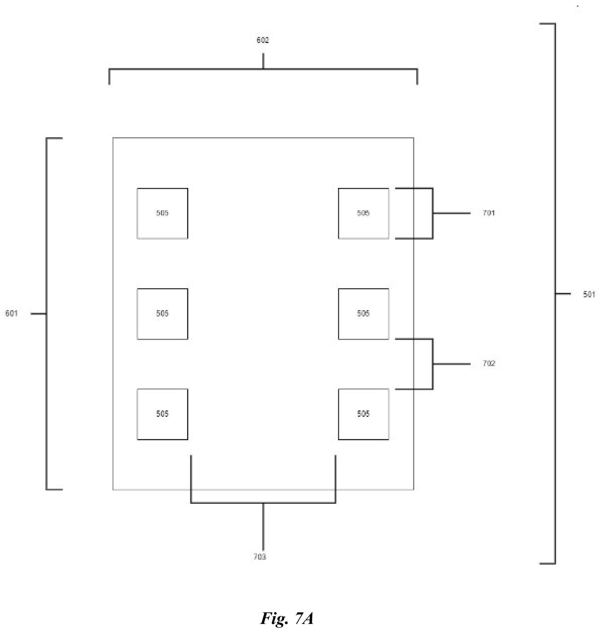

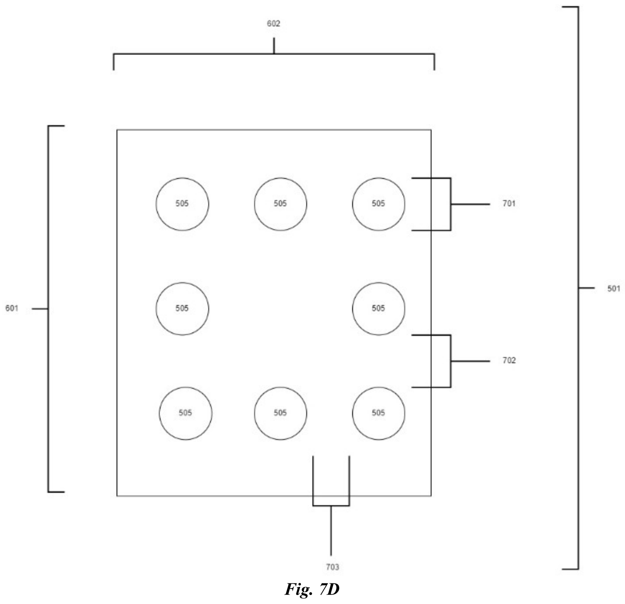

Figures 7A-D show additional exemplary diagrams of the SIW antenna 501.

The SIW antenna 501 may comprise at least one post 505. In one aspect, the at least one post 505 may have a circular or semi-circular shape. The at least one post 505 may have a diameter 701. The diameter 701 may have a size in a range from 0.001 mm to 100 mm, or 0.001 mm to 10 mm, or from 0.01 mm to 10 mm, or from 0.01 mm to 1 mm, or from 0.01 mm to 1 mm, or from 0.01 mm to 0.1 mm, or from 0.1 mm to 10 mm, or from 1 mm to 10 mm, or from 1 mm to 7 mm, or from 1 mm to 5 mm, or from 1 mm to 3 mm.

In another aspect, the at least one post 505 may have a square, a rectangular, a quadrangular shape, or any other shape (e.g. an arbitrary polygon). The at least one post 505 may have a width and/or a length. The width and/or length may have a size in a range from 0.001 mm to 100 mm, or 0.001 mm to 10 mm, or from 0.01 mm to 1 mm, or from 0.1 mm to 1 mm, or from 0.01 mm to 0.1 mm, or from 0.1 mm to 10 mm, or from 1 mm to 10 mm, or from 1 mm to 7 mm, or from 1 mm to 5 mm, or from 1 mm to 3 mm.

In another aspect of the invention, the at least one post 505 of SIW antenna may have an oblong shape, elliptical shape or any other shape.

Two neighboring posts 505 may have a y-axis spacing distance 702 between them (e.g. vertical spacing distance).

Two neighboring posts 505 may have an x-axis spacing distance 703 between them (e.g. horizontal spacing distance).

The y-axis spacing distance 702 may have a size in a range from 0.001 mm to 100 mm , or 0.001 mm to 10 mm, or from 0.01 mm to 1 mm, or from 0.1 mm to 1 mm, or from 0.01 mm to 0.1 mm, or from 0.01 mm to 10 mm, or from 0.1 mm to 10 mm, or from 1 mm to 10 mm, or from 1 mm to 20 mm, or from 10 mm to 20 mm, or from 14 mm to 16 mm, or from 9 mm to 11 mm, or from 1 mm to 15 mm, or from 1 mm to 10 mm, or from 1 mm to 5 mm, or from 1 mm to 4 mm, or from 1 mm to 3 mm, or from 1 mm to 2 mm, or from 3 mm to 4 mm, or from 2 mm to 3 mm.

The x-axis spacing distance 703 may have a size in a range from 0.001 mm to 100 mm , or 0.001 mm to 10 mm, or from 0.01 mm to 1 mm, or from 0.1 mm to 1 mm, or from 0.01 mm to 0.1 mm, or from 0.01 mm to 10 mm, or from 0.1 mm to 10 mm, or from 1 mm to 10 mm, or from 1 mm to 20 mm, or from 10 mm to 20 mm, or from 13 mm to 16 mm, or from 8 mm to 11 mm, or from 1 mm to 15 mm, or from 1 mm to 10 mm, or from 1 mm to 5 mm, or from 1 mm to 4 mm, or from 1 mm to 3 mm, or from 1 mm to 2 mm, or from 3 mm to 4 mm, or from 2 mm to 3 mm.

The posts 505 may have a geometric center. The two neighboring posts 505 may comprise a distance between geometric centers, such as for example vertical distance, horizontal distance or diagonal distance. The distance between the geometric centers of two neighboring posts 505 may be in range from 0.001 mm to 100 mm, or 0.001 mm to 10 mm, or from 0.01 mm to 1 mm, or from 0.1 mm to 1 mm, or from 0.01 mm to 0.1 mm, or from 0.01 mm to 10 mm, or from 0.1 mm to 10 mm, or from 1 mm to 10 mm, or from 1 mm to 20 mm, or from 10 mm to 20 mm, or from 13 mm to 16 mm, or from 8 mm to 11 mm, or from 1 mm to 15 mm, or from 1 mm to 10 mm, or from 1 mm to 5 mm, or from 1 mm to 4 mm, or from 1 mm to 3 mm, or from 1 mm to 2 mm, or from 3 mm to 4 mm, or from 2 mm to 3 mm.

The posts 505 may be positioned in various configurations on the SIW antenna 501. The posts 505 may be positioned in a matrix-like shape. The posts 505 may be positioned in a sparse matrix-like shape. The number of posts 505 may form rows and columns.

The sparse matrix may be a matrix that only contains posts 505 at certain locations on the SIW antenna 501. For example, the posts 505 may form 1% to 80% of the surface area of the SIW 501 antenna, or 1 % to 60 % of the surface area of the SIW antenna 501, or 2 % to 50 % of the surface area of the SIW antenna 501, or 2 % to 30 % of the surface area of the SIW antenna 501, or 2 % to 20 % of the surface area of the SIW antenna 501, or 1 % to 10 % of the surface area of the SIW antenna 501, or 5 % to 30 % of the surface area of the SIW antenna 501, or 8 % to 15 % of the surface area of the SIW antenna 501, or 10 % to 20 % of the surface area of the SIW antenna 501, or 10 % to 50 % of the surface area of the SIW antenna 501.

The SIW antenna 501 may comprise a length 601, width 602 and thickness 603, wherein the length 601 multiplied by the width 602 equals the surface area of the SIW antenna.

The length 601 of the SIW antenna may be in a range from 1 mm to 1000 mm, or from 1 mm to 100 mm , or from 1 mm to 100 mm, or from 1 mm to 50 mm, or from 2 mm to 15 mm, or from 5 mm to 50 mm, or from 5 mm to 30 mm, or from 0.5 mm to 5 mm, or from 1 mm to 5 mm, or from 1 mm to 200 mm, or from 100 mm to 200 mm, or from 10 mm to 150 mm, or from 10 mm to 100 mm, or from 10 mm to 80 mm, or from 40 mm to 80 mm, or from 60 mm to 70 mm, or from 50 mm to 100 mm, or from 10 mm to 50 mm, or from 20 mm to 50 mm, or from 30 mm to 40 mm, or from 40 mm to 50 mm, or from 40 mm to 80 mm, or from 50 mm to 80 mm, or from 60 mm to 70 mm.

The width 602 of the SIW antenna may be in a range from 1 mm to 1000 mm, or from 1 mm to 100 mm, or from 1 mm to 100 mm, or from 1 mm to 50 mm, or from 2 mm to 15 mm, or from 5 mm to 50 mm, or from 5 mm to 30 mm, or from 0.5 mm to 5 mm, or from 1 mm to 5 mm or from 1 mm to 200 mm, or from 100 mm to 200 mm, or from 10 mm to 150 mm, or from 10 mm to 100 mm, or from 10 mm to 80 mm, or from 40 mm to 80 mm, or from 60 mm to 70 mm, or from 50 mm to 100 mm, or from 10 mm to 50 mm, or from 20 mm to 50 mm, or from 30 mm to 40 mm, or from 40 mm to 50 mm, or from 10 mm to 40 mm, or from 20 mm to 40 mm, or from 30 to 40 mm.

The thickness 603 of SIW antenna may be in a range from 0.001 mm to 100 mm, or from 0.01 mm to 10 mm, or from 0.05 mm to 1 mm, or from 0.1 mm to 1 mm, or from 0.2 mm to 1 mm, or from 0.2 mm to 0.5 mm, or from 0.5 mm to 1 mm, or from 1 mm to 10 mm, or from 1 mm to 5 mm, or from 1.5 mm to 4 mm, or from 2 mm to 4 mm, or from 2 mm to 15 mm, or from 10 mm to 15 mm, or from 1 mm to 15 mm, or from 1 mm to 7 mm, or from 1 mm to 5 mm, or from 3 mm to 4 mm.

The SIW antenna 501 may comprise a surface area in a range from 0.01 mm² to 1 ·10⁶ mm², or from 0.01 mm² to 1 ·10,000 mm², or from 1 mm² to 1 ·10,000 mm², or from 1 mm² to 2500 mm², or from 4 mm² to 500 mm², or from 4 mm² to 225 mm², or from 25 mm² to 2500 mm², or from 25 mm² to 1000 mm², or from 25 mm² to 900 mm², 0.25 mm² to 25 mm², or from 1 mm² to 25 mm² , or from 1000 mm² to 5000 mm², or from 1000 mm² to 4000 mm², or from 1500 mm² to 3000 mm², or from 2000 mm² to 2500 mm², or from 2200 mm² to 2400 mm².

The SIW antenna 501 may comprise a volume in a range from 1 · 10⁻⁵ mm³ to 1 · 10⁸ mm³, or from 1 · 10⁻⁴ mm³ to 1 · 10⁵ mm³, or from 0.05 mm³ to 1 · 10 000 mm³, or from 0.1 mm³ to 10,000 mm³, or from 1 mm³ to 7500 mm³, or from 1 mm³ to 5000 mm³, or from 1 mm³ to 2500 mm³, or from 1000 to 5000, or from 100 mm³ to 10,000 mm³, or from 10 mm³ to 50,000 mm³ , or from 100 mm³ to 50,000 mm³ , or from 100 mm³ to 20,000 mm³, or from 1000 mm³ to 10,000 mm³, or from 5000 mm³ to 10,000 mm³, or from 2500 mm³ to 7500 mm³, or 5000 mm³ to 50,000 mm³, or from 10,000 mm³ to 40,000 mm³, or from 25,000 mm³ to 35,000 mm³, or from 3000 mm³ to, 10 000 mm³, or from 4000 mm³ to 9000 mm³, or from 5000 mm³ to 9000 mm³, or from 7000 mm³ to 8000 mm³, or from 7500 mm³ to 8000 mm³, or from 7000 mm³ to 7500 mm³.

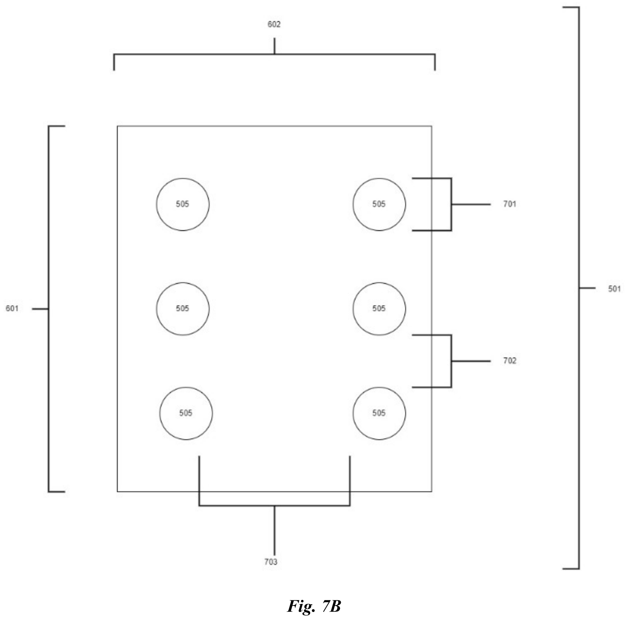

In one aspect, each row may have the same number of posts 505 as all the other rows, as may be seen in Fig 7B, for example.

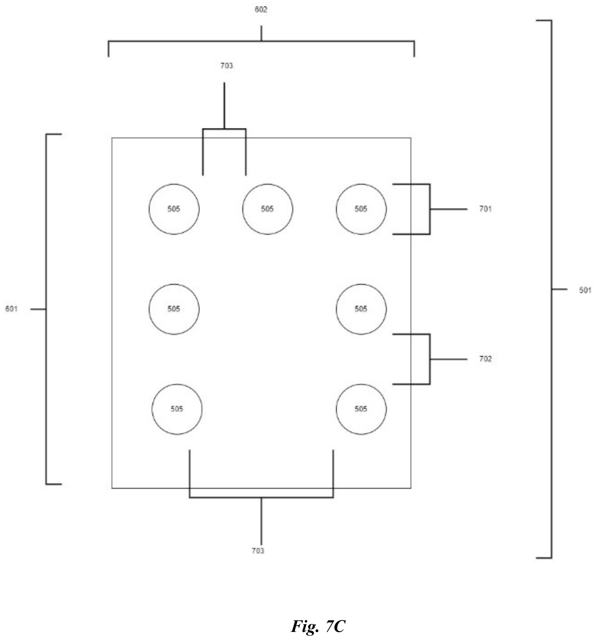

In another aspect, rows may have a different number of posts 505, or some rows may have the same number of posts 505 and some may have a different number of posts 505, as may be seen in Fig 7C, for example.

In one aspect, each column may have the same number of posts 505 as all the other columns, as may be seen in Fig 7B, for example.

In another aspect, columns may have different numbers of posts 505 or some columns may have the same number of posts 505 and some may have a different number of posts 505, as may be seen in Fig 7C, for example.

The number of rows may be the same or may be different from the number of columns.

The number of rows may be in a range from 1 to 1000, or from 1 to 500, or from 1 to 100, or from 1 to 75, or from 1 to 50, or from 1 to 25, or from 1 to 15, or from 1 to 10, or from 1 to 5, or from 1 to 4, or from 1 to 3, or from 1 to 2, or from 2 to 10, or from 2 to 5.

The number of columns may be in a range from 1 to 1000, or from 1 to 500, or from 1 to 100, or from 1 to 75, or from 1 to 50, or from 1 to 25, or from 1 to 15, or from 1 to 10, or from 1 to 5, or from 1 to 4, or from 1 to 3, or from 1 to 2, or from 2 to 10, or from 2 to 5.

The SIW antenna 501 may comprise a total number of 1 to 10⁶ posts, or from 1 to 10,000 posts, or from 1 to 1000 posts, or from 1 to 500 posts, or from 1 to 100 posts, or from 1 to 75 posts, or from 1 to 50 posts, or from 1 to 25 posts, or from 2 to 25 posts, or from 1 to 10 posts, or from 1 to 5 posts, or from 5 to 25 posts, or from 10 to 25 posts, or from 3 to 15 posts, or from 5 to 50 posts, or from 25 to 75 posts, or from 50 to 100 posts, or from 50 to 75 posts, or from 25 to 70 posts, or from 25 to 60 posts, or from 35 to 60 posts, or from 40 to 60 posts, or from 50 to 60 posts, or from 40 to 50 posts.

One row may comprise 1 to 1000 posts, or from 1 to 500 posts, or from 1 to 100 posts, or from 1 to 75 posts, or from 1 to 50 posts, or from 1 to 25 posts, or from 1 to 15 posts, or from 1 to 10 posts, or from 1 to 5 posts, or from 1 to 4 posts, or from 1 to 3 posts, or from 1 to 2 posts, or from 2 to 10 posts, or from 2 to 5 posts, or from 5 to 50 posts, or from 5 to 35 posts, or from 10 to 30 posts, or from 12 to 25 posts, or from 15 to 20 posts, or from 2 to 20 posts, or from 5 to 15 posts, or from 25 to 100 posts, or from 25 to 75 posts, or from 5 to 15 posts, or from 7 to 12 posts.

One column may comprise 1 to 1000 posts, or from 1 to 500 posts, or from 1 to 100 posts, or from 1 to 75 posts, or from 1 to 50 posts, or from 1 to 25 posts, or from 1 to 15 posts, or from 1 to 10 posts, or from 1 to 5 posts, or from 1 to 4 posts, or from 1 to 3 posts, or from 1 to 2 posts, or from 2 to 10 posts, or from 2 to 5 posts, or from 5 to 50 posts, or from 5 to 35 posts, or from 10 to 30 posts, or from 12 to 25 posts, or from 15 to 20 posts, or from 2 to 20 posts, or from 5 to 15 posts, or from 25 to 100 posts, or from 25 to 75 posts, or from 15 to 25 posts, or from 17 to 20 posts.

The SIW antenna 501 may comprise y-spacing distances 702 and x-spacing distances 703. In one aspect, all the y-spacing distances 702 between neighboring posts 505 may be the same.

In another aspect, the SIW antenna may comprise various y-axis spacing distances 702 between the neighboring posts 505, e.g. a first y-axis spacing distance between a first and a second posts 505 is different than a second y-axis spacing distance between the second and third posts 505.

In yet another aspect, some y-axis spacing distances may be the same between a plurality of neighboring posts and some y-axis spacing distances may be different between a different plurality of neighboring posts.

In one aspect, all the x-spacing distances 703 may be the same.

In another aspect, the SIW antenna may comprise various x-axis spacing distances 703 between the neighboring posts 505.

In yet another aspect, some x-axis spacing distances may be the same between a plurality of neighboring posts and some x-axis spacing distances may be different between a different plurality of neighboring posts.

The diameter 701, the y-axis spacing distance 702, and the x-axis spacing distance 703 may determine the radiation parameters of the SIW antenna. The diameter 701 may significantly affect the return loss of the SIW antenna. The ratio of diameter 701 to y-axis spacing distance 702 is considered to be one of the critical parameters of the radiation.

The substrate 502 may have various shapes, for example, a quadrangle, rectangular, square, oblong, circular, semi-circular, conic, other cross-sectional or any arbitrary polygonal shape.

The thickness 507 of the substrate 502 may be in a range from 0.001 mm to 100 mm, or from 0.01 mm to 10 mm, or from 0.05 mm to 1 mm, or from 0.1 mm to 1 mm, or from 0.2 mm to 1 mm, or from 0.2 mm to 0.5 mm, or from 0.5 mm to 1 mm, or from 1 mm to 10 mm, or from 1 mm to 5 mm, or from 1.5 mm to 4 mm, or from 2 mm to 4 mm, or from 0.1 mm to 4 mm, or from 0.1 mm to 3.5 mm, or from 0.5 mm to 3.5 mm, or from 1 mm to 3.5 mm, or from 0.01 mm to 3 mm, or from 0.01 mm to 2 mm, or from 0.1 mm to 2 mm.

The substrate 502 may comprise a length and a width. The length of the substrate 502 may be identical to the length 601 of the SIW antenna. The width of the substrate 502 may be identical to the width 602 of the SIW antenna.

The substrate 502 may be made out of a dielectric material. The dielectric material may be a composition of various materials, such as, for example, ceramics, polycarbonate, polyethylene, polypropylene, glass fiber or epoxy composite with copper foil laminated on one or both sides (e.g. FR-4), Rogers materials, PTFE based materials, Ceramic-filled PTFE composites, and Polyimide, glass-filled PTFE compo sites (e.g. Duroid), Arlon, Arlon 1000, and other materials suitable for SIW applications.

The substrate 502 may be characterized by its relative permittivity. The substrate 502 may have a relative permittivity from 1 to 150, or from 1 to 50, or from 1 to 25, or from 1 to 15, or from 1 to 12, or from 1 to 10 or from 1 to 7, or from 2 to 6, or from 2 to 5, or from 5 to 20, or from 5 to 15, or from 10 to 20, or from 10 to 15, or from 7 to 13, or from 8 to 11.

The substrate 502 may be characterized by its relative permittivity. The substrate 502 may have a relative permittivity from 1 to 20, or from 3 to 15, or from 5 to 11, or from 6 to 8.

The substrate 502 may be characterized by its dielectric strength. The dielectric strength of the substrate 502 may be in a range from 0.1 MV/m to 100 MV/m, or from 5 MV/m to 50 MV/m, or from 10 MV/m to 60 MV/m, or from 15 MV/m to 30 MV/m, or from 20 MV/m to 24 MV/m, or from 24 MV/m to 28 MV/m, or from 10 MV/m to 30 MV/m, or from 15 MV/m to 30 MV/m, or from 24 MV/m to 30 MV/m, or from 20 MV/m to 35 MV/m.

The choice of the substrate 502 may have a great influence on the parameters of the radiation, such as for example the radiation wavelength, radiation loss, size of the radiation field, size of the hot spots and others.

The SIW antenna may comprise at least one feeding point 506. The feeding point 506 may be integrated into the substrate 502.

The choice of the y-axis spacing distance 702 and the x-axis spacing distance 703 may have a great influence on the parameters of the radiation, such as for example the radiation wavelength, radiation loss and others.

Fig 7A shows one exemplary aspect of the invention, where the at least one post 505 has a rectangular shape. Figures 7B - D show other exemplary aspects of the invention, where the at least one post 505 has a circular shape. The at least one post 505 may be located in at least one row and in at least one column. The rows and columns may have a different number of posts 505. The SIW antenna may also comprise various other aspects.

The parameters of the SIW antenna, such as the relative permittivity of the substrate 502, dielectric strength of the substrate 502, length of the substrate 502, width of the substrate 502, thickness of the substrate 502, diameter 701 of the at least one post 505, post spacing distance 702, x-axis spacing distance 703, and others may be in various different combinations. The combination of the parameters influences the parameters of the radiation.

The top metallic layer 503 and/or the bottom metallic layer 504 and the interconnections between the top metallic layer 503 and/or the bottom metallic layer 504 may be made out of a metallic material.

The metallic material may be a material with good electrical conductivity, such as copper, aluminum, brass, silver, gold, graphene and others.

The electrical conductivity of the metallic material may be in a range from 4 ·10⁶ S/m to 7 ·10⁷ S/m, or from 5 ·10⁶ S/m to 7 ·10⁷ S/m, or from 7 ·10⁶ S/m to 7 ·10⁷ S/m, or from 9 ·10⁶ S/m to 7 ·10⁷ S/m, or from 1 ·10⁷ S/m to 7 ·10⁷ S/m, or from 1.5 ·10⁷ S/m to 6.5 ·10⁷ S/m, or from 1.60 ·10⁷ S/m to 2 ·10⁷ S/m, or from 3.3 ·10⁷ S/m to 4 ·10⁷ S/m, or from 3.7 ·10⁷ S/m to 4 ·10⁷ S/m, or from 4 ·10⁷ S/m to 4.5 ·10⁷ S/m, or from 5 ·10⁷ S/m to 6 ·10⁷ S/m, or from 5.9 ·10⁷ S/m to 6 ·10⁷ S/m, or from 6 ·10⁷ S/m to 6.4 ·10⁷ S/m.

The SIW antenna 501 may be cooled. The SIW antenna 501 may be in communication with the cooling unit 107. The cooling unit 107 may comprise a cooling element. The cooling element may provide cooling to the skin surface, adjacent tissue, applicator 105, treatment array 108 and/or treatment elements. The cooling element may be integrated in the SIW antenna 501.

A treatment element 301 may be a SIW antenna 501. The treatment array 108 may comprise at least one treatment element 301. The treatment array may comprise more than one treatment elements 301, e.g. a plurality of SIW antennas.

A plurality of SIW antennas 501 may be arranged in the form of an array and may form a treatment array 108. A plurality of SIW antennas 501 may have a function of phase shift or steering to improve the distribution of heat in the soft tissue. The adjacent SIW antennas 501 may have various spacing distances. The various spacing distances may determine the phase difference between the two adjacent antennas. A plurality of SIW antennas 501 may have a fixed angle, or a group of antennas may be rotated according to a predetermined pattern.

The SIW antenna may be an integration of SIW antenna and rectangular antenna, patch antenna, dipole antenna, bow-tie antenna, biconical antenna, turnstile antenna, multi-dipole antenna, ring antenna, choke dipole antenna, modified dipole antenna, spiral antenna, a helical antenna, monopole antenna, loop antenna, horn antenna, or pyramidal horn antenna, waveguide antenna and/or others.

In one aspect, the applicator 105 may provide a treatment energy by the treatment array 108 comprising multiple treatment elements 301 (e.g. narrow beam antennas and/or wider beam antennas) that are phase shifted to cause heating of the body region combined with the cooling of the surface of the body region (e.g. surface of the skin) and thus creating a thermal reverse gradient in the body region. The device may be configured such that the skin surface (such as the epidermis) is maintained at room temperature, or in a range of 5 °C to 70 °C , or 10 °C to 60 °C , or 15 °C to 55 °C , or 20 °C to 50 °C , or 20 °C to 45 °C, or 25 °C to 40 °C or 30 °C to 37 °C or 30 °C to 35 °C using a cooling element.

The treatment elements (e.g. antennas) may be configured such that a first layer (e.g. dermal layer including at least one of collagen or elastin) may be homogeneously heated to a first temperature in a range of 37.5 °C to 90 °C , or 38 °C to 90 °C , or 42 °C to 90 °C, or 50 °C to 80 °C, or 55 °C to 75 °C, or 57 °C to 72 °C, or 60 °C to 70 °C, or 38 °C to 80 °C, or 38 °C to 70 °C or 38 °C to 65 °C, or 38 °C to 60 °C, or 45 °C to 70 °C, or 45 °C to 65 °C.

A second layer (e.g. SMAS), which may be even deeper from the skin surface than the first layer, may be influenced by the radiation maxima and the radiation minima, which may result in the formation of lesions in the second layer (e.g. SMAS). In that case, the parts of the second layer (e.g. parts of the SMAS) treated by the radiation maxima may be heated to a second temperature in the range of 37.5 °C to 90 °C , or 38 °C to 90 °C , or 42 °C to 90 °C, or 50 °C to 80 °C, or 55 °C to 75 °C, or 57 °C to 72 °C, or 60 °C to 70 °C, or 38 °C to 80 °C, or 38 °C to 70 °Cm or 38 °C to 65 °C, or 38 °C to 60 °C, or 45 °C to 70 °C, or 45 °C to 65 °C. The parts of the second layer (e.g. parts of the SMAS) treated by the radiation minima may be heated to a first temperature. In one aspect, the second temperature is higher than the first temperature.

In another aspect, the body region is cooled such that the skin surface (such as the epidermis) and the first layer (e.g. a dermal layer including at least one of collagen or elastin) are not influenced/treated by the heating and only the second layer (e.g. SMAS), may be influenced/treated by the radiation maxima and the radiation minima, which may result in the formation of lesions in the second layer (e.g. SMAS). In that case, the parts of the second layer (e.g. SMAS) treated by the radiation maxima may be heated to a temperature in the range of 42 °C to 100°C, or 50 °C to 95 °C, or 65 °C to 92 °C, or 70 °C to 90 °C, or 42 °C to 85 °C, or 42 °C to 80 °C, or 42 °C to 75 °C, or 42 °C to 70 °C and the parts of the second layer (e.g. SMAS) treated by the radiation minima may not be influenced/treated by the heating.

Therefore, the device and method provide controlled lesions in the SMAS area (floor projection of the SMAS) surrounded by areas with different, e.g. limited, or without any thermal damage. The device and methods may provide this effect without perforation of the epidermis and/or dermis area.

In another aspect, the body region is cooled such that the skin surface (such as the epidermis) is affected/treated by the heating and only the first layer (e.g. dermal layer), may be influenced/treated by the radiation maxima and the radiation minima, which may result in the formation of lesions in the first layer (e.g. dermis). In that case, the parts of the first layer (e.g. dermis) treated by the radiation maxima may be heated to a temperature in the range of 37.5 °C to 90 °C , or 38 °C to 90 °C , or 42 °C to 90 °C, or 50 °C to 80 °C, or 55 °C to 75 °C, or 57 °C to 72 °C, or 60 °C to 70 °C, or 38 °C to 80 °C, or 38 °C to 70 °Cm or 38 °C to 65 °C, or 38 °C to 60 °C, or 45 °C to 70 °C, or 45 °C to 65 °C and the parts of the skin surface (e. g. epidermis) treated by the radiation minima may not be influenced/treated by the heating.

In another aspect, the body region is cooled such that the skin surface (such as the epidermis) is influenced/treated by the heating and only the first layer (e.g. dermal layer) may be influenced/treated by the radiation maxima and the radiation minima, which may result in the formation of lesions in the first layer (e.g. dermis). In that case, the parts of the first layer (e.g. dermis) treated by the radiation maxima may be heated to a temperature in the range of 36 °C to 90 °C , or 38 °C to 90 °C , or 42 °C to 90 °C, or 50 °C to 80 °C, or 55 °C to 75 °C, or 57 °C to 72 °C, or 60 °C to 70 °C, or 38 °C to 80 °C, or 38 °C to 70 °Cm or 38 °C to 65 °C, or 38 °C to 60 °C, or 45 °C to 70 °C, or 45 °C to 65 °C and the parts of the skin surface (e. g. epidermis) treated by the radiation minima may not be influenced/treated by the heating.

Therefore, the device and method provides controlled lesions in the dermal area (floor projection of the dermis) surrounded by areas with different, e.g. limited, or without any thermal damage. The device and methods may provide this effect without perforation of the epidermis area.

One or more applicators 105 may comprise one or more layers, where one of the layers may comprise one or more treatment arrays 108, where one or more treatment arrays 108 may comprise one or more treatment elements 301, such as, for example, microstrip patch antenna arrays or one or more waveguide arrays or other.

One or more treatment arrays 108 may comprise one or more individual antennas. Neighboring individual antennas may be distanced by a spacing distance in a range of 0.001 mm to 0.5 m, or from 0.01 mm to 0.25 m or from 0.1 mm to 0.1 m, or from 0.1 mm to 50 mm, or from 0.1 mm to 25 mm, or from 0.1 mm to 10 mm, or from 0.5 mm to 10 mm, or from 0.5 mm to 5 mm. The spacing distance may determine the phase difference between two adjacent antennas and may affect the resulting radiation pattern.

One or more individual antennas may have a volume in a range from 0.001 mm³ to 100·10³ mm³ or from 0.01 mm³ to 10·10³ mm³, or from 0.1 mm³ to 1·10³ mm³, or from 1 mm³ to 100 mm³, or from 10 mm³ to 50 mm³.

One or more individual antennas may have a length in a range of 0.001 mm to 1 m, or from 0.01 mm to 0.1 m, or from 0.1 mm to 0.1 m, or from 0.1 mm to 50 mm, or from 0.5 mm to 25 mm, or from 1 mm to 25 mm, or from 5 mm to 25 mm.

One or more individual antennas may have a diameter in a range of 0.001 mm to 1 m, or from 0.01 mm to 0.1 m, or from 0.1 mm to 0.1 m, or from 0.1 mm to 50 mm, or from 0.5 mm to 25 mm, or from 1 mm to 25 mm, or from 5 mm to 25 mm. The dimensions of the antenna may determine its operating frequency and may affect the resulting radiation pattern.

In one aspect, the applicator 105 may include a plurality of treatment arrays 108 which may be distanced by a spacing distance in a range of 0.001 mm to 0.5 m, or from 0.01 mm to 0.25 m, or from 0.1 mm to 0.1 m, or from 0.1 mm to 50 mm, or from 0.1 mm to 25 mm, or from 0.1 mm to 10 mm, or from 0.5 mm to 10 mm, or from 0.5 mm to 5 mm.

The treatment may be based on moving the applicator 105 according to a predefined treatment pattern. The treatment pattern may include circular, linear, or other moves along a curve. Alternatively, the treatment may comprise a stationary applicator 105 and switching on/off certain treatment elements 301 and/or individual antennas. The alternative switching on/off certain treatment elements 301 and/or individual antennas may form a predetermined treatment pattern, such as, for example, circular, linear, or other moves along a curve. In another aspect, the treatment may be based on moving the applicator 105 in combination with switching on/off certain treatment elements 301 and/or individual antennas according to a predefined treatment pattern.

The treatment may be based on stamping of the applicator 105 onto the skin according to a predefined treatment pattern. The treatment pattern may involve sequentially stamping in a grid-like arrangement, overlapping spots, or any other predefined positions to ensure comprehensive coverage of the treatment area of the skin.

The duration of each stamping action may be in a range from 0.5 seconds to 10 seconds, or in a range from 3 seconds to 8 seconds, or in a range from 4 seconds to 7 seconds.

The duration of each stamping action may be in a range from 0.5 seconds to 25 seconds, or in a range from 3 seconds to 20 seconds, or in a range of 6 seconds to 15 seconds, or in a range of 9 seconds to 12 seconds.

The duration of each stamping action may be in a range from 10 milliseconds to 2 seconds, 10 milliseconds to 10 seconds, or 10 milliseconds to 25 seconds; or in a range from 30 milliseconds to 5 seconds, 30 milliseconds to 8 seconds, or 30 milliseconds to 20 seconds; or in a range from 50 milliseconds to 7 seconds, 50 milliseconds to 15 seconds; or in a range from 90 milliseconds to 12 seconds.

The stamping action means bringing the applicator into contact with the patient, delivering a predefined amount of energy on a specific location or region of the patient - ensuring precision and minimizing damage to surrounding tissue - and then moving the applicator to another location on the patient and repeating these steps.

The device may also be combined with the delivery of various substances that, for example, provide hydration for the subject. The substances may modulate the subject's normal metabolism, lipolysis rate, or the effect of electromagnetic radiation, among others.

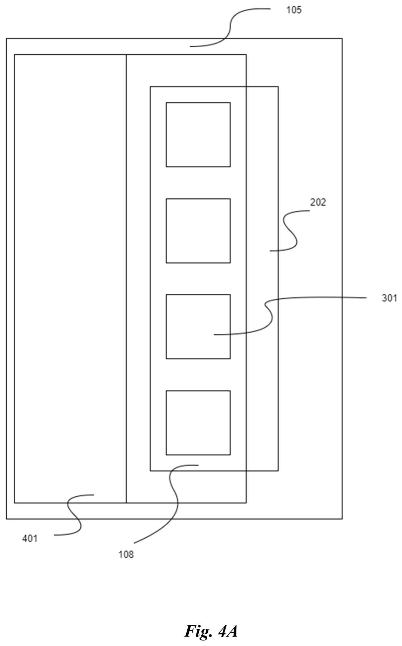

Fig. 4A shows a schematic diagram of one or more applicators 105. One or more applicators 105 comprise one or more treatment arrays 108 and one or more treatment elements 301. One or more applicators 105 may also include one or more spacing elements 202 and one or more layers 401 (e.g PCB, substrate, or others). The treatment array 108 may also be integrated into the applicator 105.

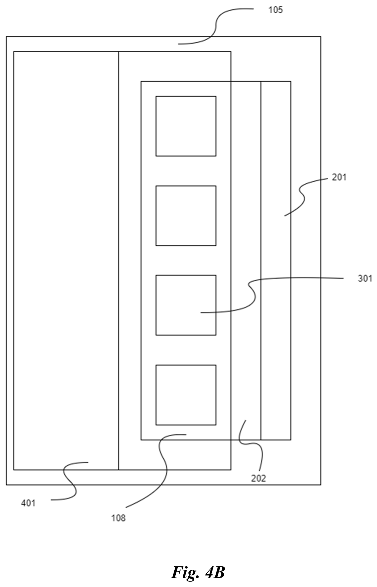

Fig. 4B shows a schematic diagram of one or more applicators 105. One or more applicators 105 comprise one or more treatment arrays 108 and one or more treatment elements 301. One or more applicators may also include one or more attaching elements 201, one or more spacing elements 202 and one or more layers 401.

A cooling unit 107 may comprise a cooling element 402.

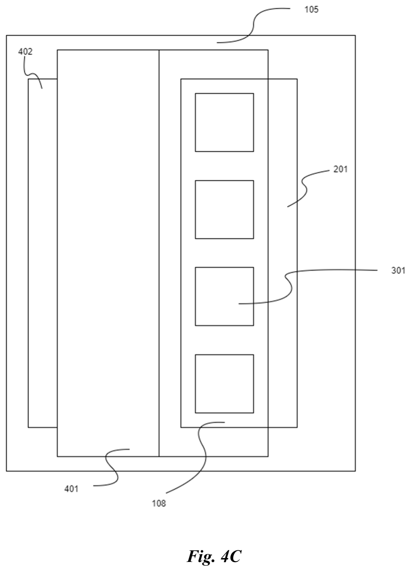

Fig. 4C shows a schematic diagram of one or more applicators 105. One or more applicators 105 comprise one or more treatment arrays 108 and one or more treatment elements 301. One or more applicators 105 may also include one or more attaching elements 201, one or more cooling elements 402 and one or more layers 401. The cooling element 402 may provide cooling to the skin surface and/or the adjacent tissue. The cooling element 402 may be in communication with the cooling unit 107 and/or may be a part of the cooling unit 107.

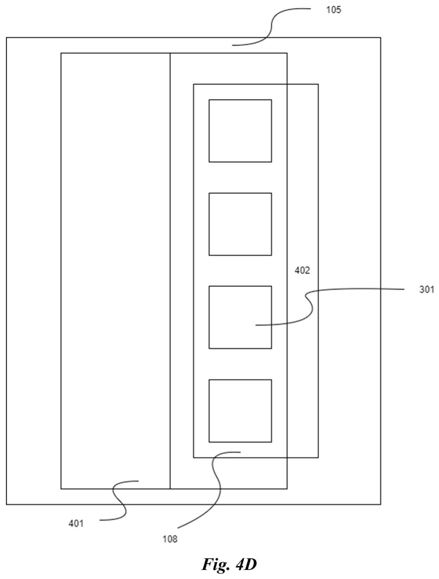

Fig. 4D shows a schematic diagram of one of more applicators 105. One or more applicators 105 comprise one or more treatment arrays 108 and one or more treatment elements 301. One or more applicators 105 may also include one or more cooling elements 402 and one or more layers 401. The cooling element 402 may provide cooling to the skin surface and/or the adjacent tissue. The cooling element 402 may be positioned between the treatment array 108 and the adjacent tissue. The cooling element 402 may introduce a reverse thermal gradient in the adjacent tissue.

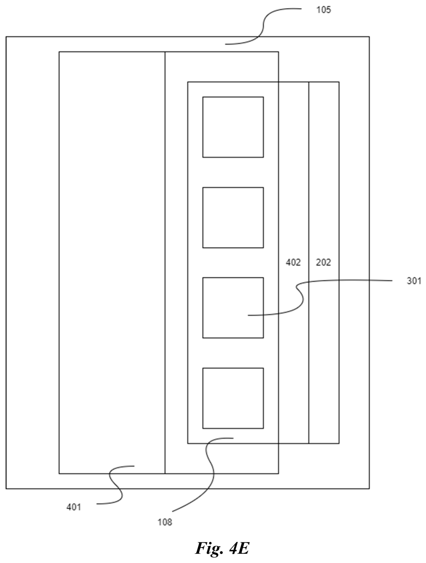

Fig. 4E shows a schematic diagram of one of more applicators 105. One or more applicators 105 may also include one or more spacing elements 202, one or more cooling elements 402. The spacing element 202 may be positioned between the cooling element 402 and the adjacent tissue. The spacing element 202 may also be positioned between the treatment array 108 and the cooling element 402. One or more applicators 105 may also include the attaching element, positioned between the adjacent tissue and the cooling element 402 or between the adjacent tissue and the spacing element 202.

The attaching element 201 may provide an attachment of the applicator 105 to the skin surface and/or a body region. The attaching element 201 may be, for example, a sticker, a belt, or a vacuum cup that may be in communication with a vacuum source.

The spacing element 202 may be provided between the applicator 105 and/or one or more treatment arrays 108, and it may improve the energy transfer of the electromagnetic energy to the soft tissue. In one aspect, the spacing element 202 may also have a cooling function. The spacing element 202 may be in communication with the cooling unit 107 and/or be a part of the cooling unit 107. The spacing element 202 may include a cooling means, such as one or more Peltier elements, a cooling fluid, a heat conductor, a heating element, or other means.

The cooling element 402 may prevent any harmful influence of the energy delivery and may prevent overheating the tissue adjacent to the skin surface. The cooling element 402 may provide cooling to the skin surface, adjacent tissue, applicator 105, treatment array 108 and/or treatment elements 301. The cooling element 402 may be in communication with the cooling unit 107 and/or be a part of the cooling unit 107. The cooling element 402 may include a cooling means, such as one or more Peltier elements, a cooling fluid, a nozzle spraying a cooling fluid, a heat conductor, a heating element, or other means. The cooling fluid may be a liquid or a gas. The cooling fluid may be air, oxygen, water, ethanol, oil, or any combination of these fluids. The temperature of the cooling element 402 during the treatment may be in the range of -80 °C to 36 °C or -70 °C to 35 °C or -60 °C to 34 °C or -20 °C to 30 °C or 0 °C to 27 °C or 5 °C to 25 °C, or 5 °C to 20 °C, or 5 °C to 15 °C.

The system may comprise a refrigerant to cool the cooling fluid. The refrigerant may be any kind of refrigerant available, such as R-32, R-124a, R-410a, R-290, R-600a, R-717, R-1234yf, R-744, R-404a, R-134a, R-143a, R-407c, R-134a, R-410a, R-452a, R-404, R-507 and others.

The cooling fluid may have a flow rate in a range from 0.001 L/minute to 10 000 L/minute, or from 0.01 L/minute, to 1000 L/minute, or from 0.01 L/minute to 100 L/minute, or from 1 L/minute to 10 000 L/minute, or from 1 L/minute to 5000 L/minute, or from 50 L/minute to 1000 L/minute or from 100 L/minute to 1500 L/minute, or from 500 L/minute to 1500 L/minute, or from 800 L/minute to 1200 L/minute, or from 1000 L/minute to 1500 L/minute or from 1100 L/minute to 1300 L/minute, or from 1 L/minute to 10 L/minute, or from 1 L/minute to 50 L/minute, or from 10 L/minute to 200 L/minute, or from 1 L/minute to 500 L/minute.

The flow rate may vary during treatment; it may range from 0 to 100 % of the maximal flow rate. The flow rate of the pump 903 may be controlled by the control unit 103.

The cooling element 402 may be integrated into the treatment array 108 and/or the cooling element 402 may directly cool the treatment element 301. The cooling element 402 may directly cool, for example, the antenna, the waveguide, the microstrip antenna or the SIW antenna 501. The cooling fluid may directly circulate in, for example, the antenna, the waveguide, the microstrip antenna or the SIW antenna 501.

The cooling fluid may be sprayed by a nozzle and may be configured to cool the treatment element 301 and/or to cool the target tissue.

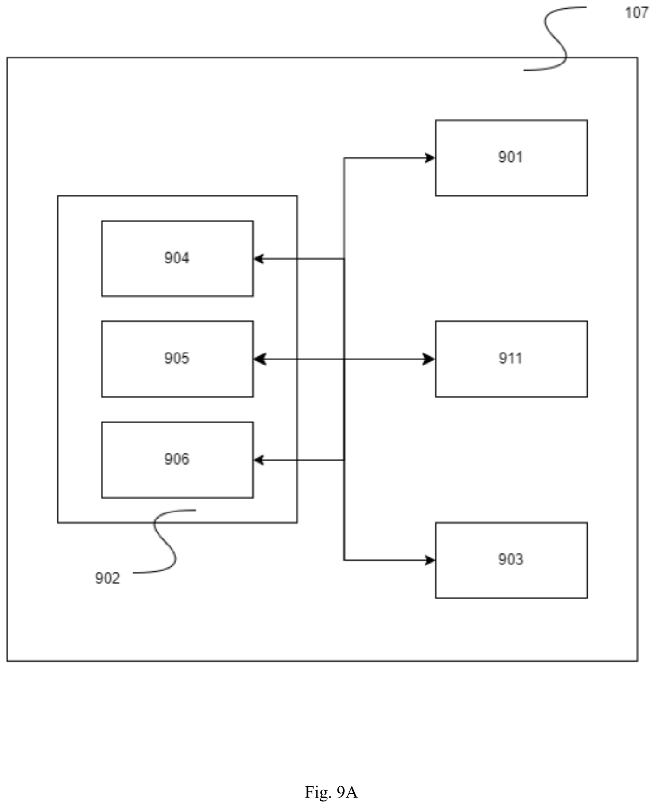

Figures 9A-B show a schematic diagram of the cooling unit 107. A cooling unit 107 may comprise a cooling element 402. The cooling element 402 may include a cooling means, such as one or more Peltier elements, a cooling fluid, a nozzle spraying a cooling fluid, a heat conductor, a heating element, or other means. The cooling fluid may be a liquid or a gas. The cooling fluid may be air, oxygen, water, ethanol, oil, or any combination of these fluids.

Figure 9A shows a schematic diagram of the cooling unit 107. The cooling element 402 may be a cooling fluid 911. The cooling unit 107 may comprise at least one fluid container 901, a tubing system 902 and/or a pump 903. The cooling unit 107 may comprise the cooling fluid 911. The tubing system 902 may comprise one or more tubes. The tubing system 902 may comprise an inlet tube 904, an outlet tube 905 and/or pool 906. The inlet tube 904 and the outlet tube 905 may be configured to circulate the cooling fluid 911. The tubes may be positioned within the main unit 101, within the applicator 105. The device may comprise at least one connecting tube, configured to connect the applicator 105 to the main unit 101. The device may comprise at least one treatment element 301 that is cooled by the cooling fluid 911.

In one aspect of the invention, the main unit 101 comprises the cooling unit 107.

In another aspect of the invention, the main unit 101 comprises a first part of the cooling unit 107 and the applicator 105 comprises a second part of the cooling unit 107.

In one aspect of the invention, the main unit 101 comprises the fluid container 901 and/or the pump 903. The device may comprise at least one connecting tube. The at least one connecting tube may be configured to connect the main unit 101 to the applicator 105. The applicator 105 may comprise the tubing system 902 (which may comprise the inlet tube 904 and the outlet tube 905), the pool 906, the cooling plate 908, and/or at least one channel 910. The cooling unit 107 may be configured to circulate the cooling fluid 911.

In one aspect of the invention, the main unit 101 comprises the fluid container 901 and/or the pump 903. The device may comprise at least one connecting tube. The at least one connecting tube may be configured to connect the main unit 101 to the applicator 105. The applicator 105 may comprise the tubing system 902 (which may comprise the inlet tube 904 and the outlet tube 905), the pool 906, the cooling plate 908, the Peltier element 907 and/or at least one channel 910. The cooling unit 107 may be configured to circulate the cooling fluid 911.

In another aspect of the invention, the applicator 105 comprises the cooling unit 107.

In another aspect of the invention, the applicator 105 comprises the Peltier element 907.

In some aspects of the invention, the main unit 101 comprises the Peltier element 907.

In some aspects of the invention, the applicator may comprise a temperature sensor configured to measure a temperature of the cooling fluid 911 in the applicator.

The tubing system 902 may comprise tubes with a diameter in a range from 0.001 mm to 100 mm, or from 0.01 mm to 50 mm, or from 0.01 to 25 mm, or from 0.01 mm to 10 mm, or from 0.01 mm to 1 mm, or from 0.1 to 50 mm, or from 0.1 to 25 mm, or from 0.1 to 10 mm, or from 0.1 mm to 1 mm, or from 1 mm to 50 mm, or from 1 mm to 25 mm, or from 1 mm to 10 mm.

The tubing system 902 may be made out of a material such as ceramics, glass, polymers, elastomers, ceramics, and others. The tubing system 902 may be made out of a combination of those materials.

The pump 903 may be uni-directional or bi-directional. The pump 903 may be a peristaltic pump.

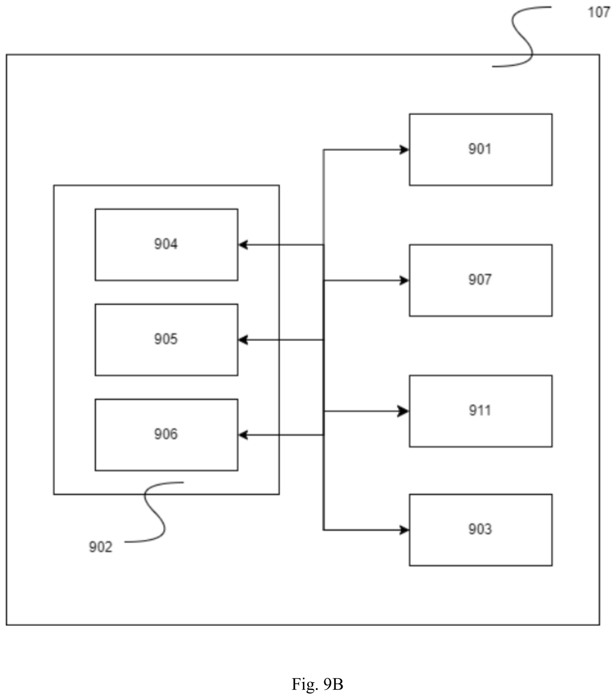

Figure 9B shows a schematic diagram of the cooling unit 107. The cooling unit 107 may comprise a cooling element 402. A cooling element 402 may be a Peltier element 907 and/or a cooling fluid 911. The Peltier element 907 may be in communication with the cooling unit 107, the cooling fluid 911, and/or the tubing system 902.

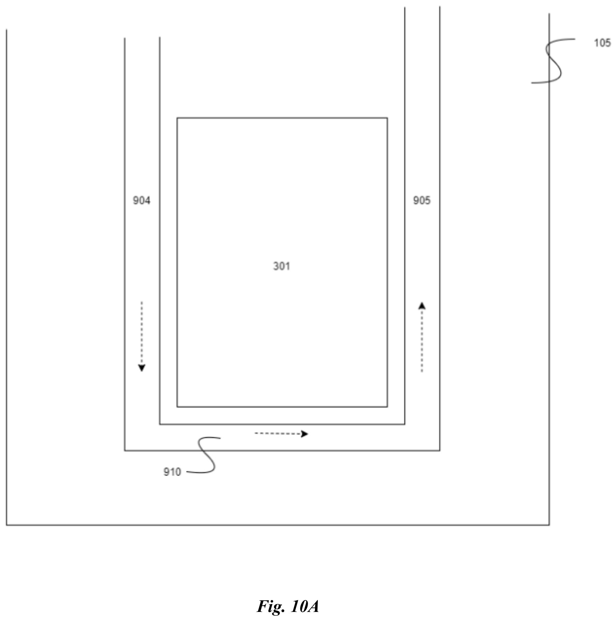

Figs. 10A-D show schematic diagrams of the applicator 105.

Figure 10A shows one aspect of the device. The cooling unit 107 may be configured to circulate the cooling fluid 911. Applicator 105 may comprise the treatment element 301, the inlet tube 904 and the outlet tube 905. The inlet tube 904 and the outlet tube 905 may be configured to circulate the cooling fluid 911. The inlet tube 904 may be configured to supply the cooled cooling fluid to the channel 910. The outlet tube 905 may be configured to discharge the cooling fluid from the channel 910 after receiving heat from the skin surface, adjacent tissue, applicator 105, treatment array 108 and/or treatment elements 301.

The inlet tube 904 and the outlet tube 905 may be configured to create a reverse thermal gradient in the target tissue and/or to cool the treatment element 301.

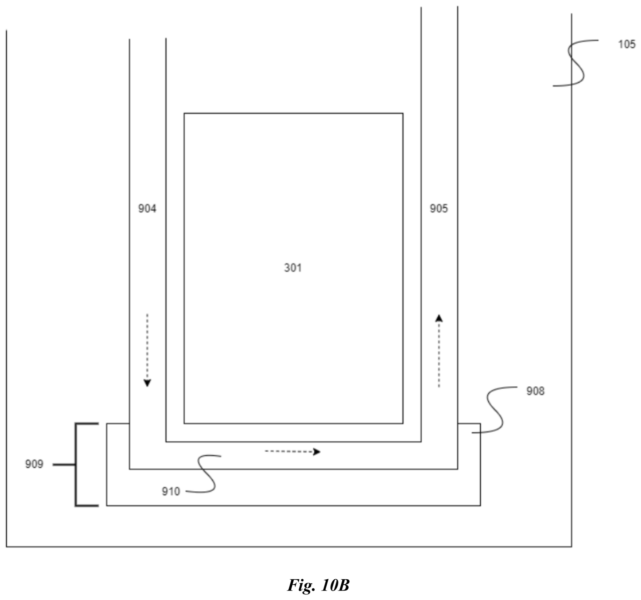

Figure 10B shows another aspect of the device. The cooling unit 107 may comprise a cooling plate 908 having a thickness 909. The cooling plate 908 may comprise at least one channel 910.

The cooling plate 908 and the spacing element 202 may be the same element.

In some aspects, the treatment element may be coupled to a spacing element or shaping element and the cooling plate may be spaced from the spacing element or shaping element by at least one channel.

In some aspects, at least one channel may have smaller, bigger, or the same area as an area of the treatment element and/or the spacing element and/or the shaping element in a cross section view taken at the aperture of the applicator. Thus the cooling fluid is in the space between the cooling plate and the treatment element and/or the spacing element and/or the shaping element.

The cooling plate 908 may be configured to be coupled to the patient (e.g. in contact with the skin) during the treatment.

The cooling plate 908 may be in communication with the at least one channel 910, with the inlet tube 904, with the outlet tube 905, with the fluid container, a tubing system 902 and/or a pump.

The at least one channel 910 may have a diameter in a range from 0.000001 mm to 100 mm, or from 0.0001 mm to 100 mm, or from 0.001 mm to 100 mm, or from 0.001 mm to 50 mm, or from 0.001 mm to 10 mm, or from 0.001 mm to 1 mm, or from 0.001 mm to 0.1 mm, or from 0.001 mm to 0.01 mm, or from 0.01 mm to 10 mm, or from 0.01 mm to 1 mm, or from 0.1 mm to 1 mm, or from 1 mm to 50 mm, or from 1 mm to 25 mm, or from 1 mm to 10 mm, or from 1 mm to 7 mm, or from 1 mm to 5 mm.

The device may comprise at least one channel 910. The device may comprise a number of channels 910 in a range of 1 to 1000 channels, or from 1 to 500 channels, or from 1 to 250 channels, or from 1 to 100 channels, or from 1 to 50 channels, or from 1 to 25 channels, or from 1 to 10 channels, or from 10 channels to 100 channels.

The device may comprise at least one channel 910. The device may comprise a number of channels 910 in a range of 2 to 1000 channels, or from 2 to 500 channels, or from 2 to 250 channels, or from 2 to 100 channels, or from 2 to 50 channels, or from 2 to 25 channels, or from 2 to 10 channels, or from 10 channels to 100 channels.

The at least one channel 910 may be made out of a material such as ceramics, glass, polymers, elastomers, and others. The at least one channel may be made out of a combination of those materials.

The thickness 909 of the cooling plate 908 may be in range from 0.001 mm to 100 mm, or from 0.001 mm to 50 mm, or from 0.001 mm to 20 mm, or from 0.001 mm to 10 mm, or from 0.001 mm to 1 mm, or from 0.001 mm to 0.1 mm, or from 0.01 mm to 1 mm, or from 0.1 mm to 1 mm, or from 1 mm to 100 mm, or from 1 mm to 50 mm, or from 1 mm to 25 mm, or from 1 mm to 10 mm, or from 5 mm to 50 mm, or from 5 mm to 40 mm, or from 5 mm to 30 mm, or from 10 mm to 50 mm, or from 10 mm to 40 mm, or from 10 mm to 30 mm, or from 0.1 mm to 5 mm, or from 0.1 mm to 2 mm, or from 0.1 mm to 0.5 mm.

The cooling plate 908 may comprise a length and a width.

The length of the cooling plate 908 may be in a range from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1·10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻⁵mm , or from 1 mm to 1000 mm, or from 100 mm to 1000 mm, or from 500 mm to 1000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻³mm to 200 mm, or from 1·10⁻³mm to 100 mm, or from 1 mm to 100 mm, or from 1 mm to 75 mm, or from 1 mm to 50 mm, or from 1 mm to 25 mm, or from 5 mm to 25 mm, or from 10 mm to 20 mm, or from 5 mm to 30 mm, or from 0.5 mm to 5 mm, or from 10 mm to 100 mm, or from 20 mm to 70 mm, or from 40 mm to 60 mm.

The width of the cooling plate 908 may be in a range from 1·10⁻⁶mm to 1000 mm, or from 1·10⁻⁶mm to 500 mm, or from 1·10⁻⁶mm to 100 mm, or from 1·10⁻⁶mm to 50 mm, or from 1·10⁻⁶mm to 10 mm, or from 1·10⁻⁶mm to 5 mm, or from 1.10⁻⁶mm to 1 mm, or from 1·10⁻⁶mm to 1·10⁻²mm , or from 1·10⁻⁶mm to 1·10⁻³mm , or from 1·10⁻⁶mm to 1·10⁻⁵mm , or from 1 mm to 1000 mm, or from 100 mm to 1000 mm, or from 500 mm to 1000 mm, or from 1·10⁻³mm to 500 mm, or from 1·10⁻³mm to 200 mm, or from 1·10⁻³mm to 100 mm, or from 1·10⁻³mm to 10 mm, or from 1 mm to 200 mm, or from 10 mm to 200 mm, or from 25 mm to 200 mm, or from 50 mm to 200 mm, or from 100 mm, to 200 mm, or from 1·10⁻³mm to 200 mm, or from 1·10⁻³mm to 100 mm, or from 1 mm to 100 mm, or from 1 mm to 75 mm, or from 1 mm to 50 mm, or from 1 mm to 25 mm, or from 5 mm to 25 mm, or from 10 mm to 20 mm, or from 5 mm to 30 mm, or from 0.5 mm to 5 mm, or from 5 mm to 100 mm, or from 5 mm to 50 mm, or from 5 mm to 30 mm, or from 10 mm to 30 mm.

The cooling plate 908 may be made out of a material such as ceramics, glass, polymers, elastomers, polycarbonate, polyethylene, polypropylene, metals, silicone-based compounds, dielectric insulating materials, aluminum nitride, and others. The cooling plate 908 may be made of a combination of the listed materials.

The channels 910 may be configured to cool the treatment element 301 and/or to cool the adjacent tissue.

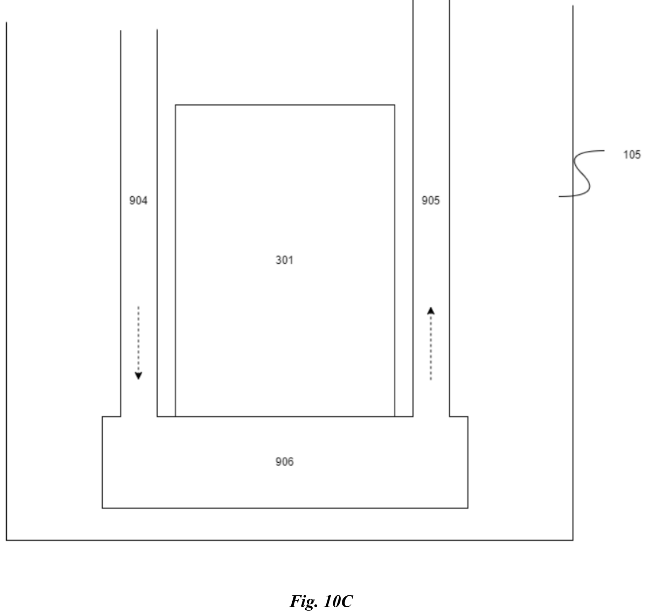

Figure 10C shows yet another aspect of the device. The applicator may also include a fluid pool 906. The fluid pool 906 may be in communication with the inlet tube 904 and the outlet tube 905 and may be configured to be coupled to the patient (e.g. in contact with the patient).

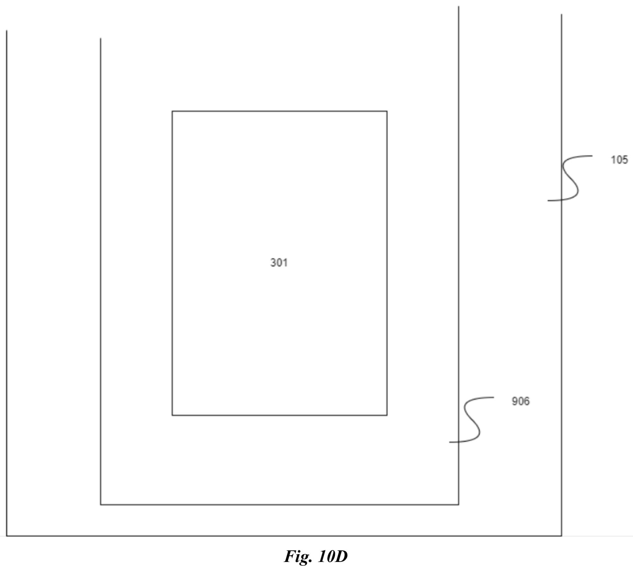

Figure 10D shows yet another aspect of the device. The applicator may include a fluid pool 906 that surrounds the treatment element 301 and may be configured to be coupled to the patient (e.g in contact with the skin).

The volume of the cooling fluid 911 circulating through the cooling unit 107 may be in a range from 0.001 L to 100 L, or from 0.1 L to 50 L, or from 0.1 L to 25 L, or from 0.5 L to 20 L, or from 1 L to 15 L, or from 1 L to 10 L, or from 1.5 L to 5 L, or from 0.1 L to 1 L.

The temperature of the cooling fluid 911 during the treatment may be in the range of -80 °C to 36 °C, or -70 °C to 35 °C, or -60 °C to 34 °C, or -20 °C to 30 °C, or 0 °C to 27 °C, or 5 °C to 25 °C, or 5 °C to 20 °C, or 5 °C to 15 °C.

The cooling unit 107 may be configured to remove heat from the target tissue. The cooling unit 107 may be configured to create a reverse thermal gradient in the adjacent tissue. The coldest temperatures may be achieved near the cooling surfaces or in the tissue near the applicators. Cooling may be configured to reduce the temperature of the tissue to a temperature that is not destructive to the tissue.

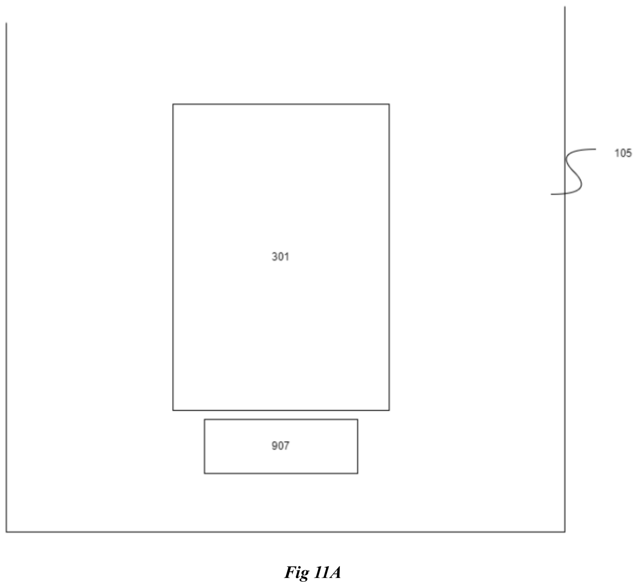

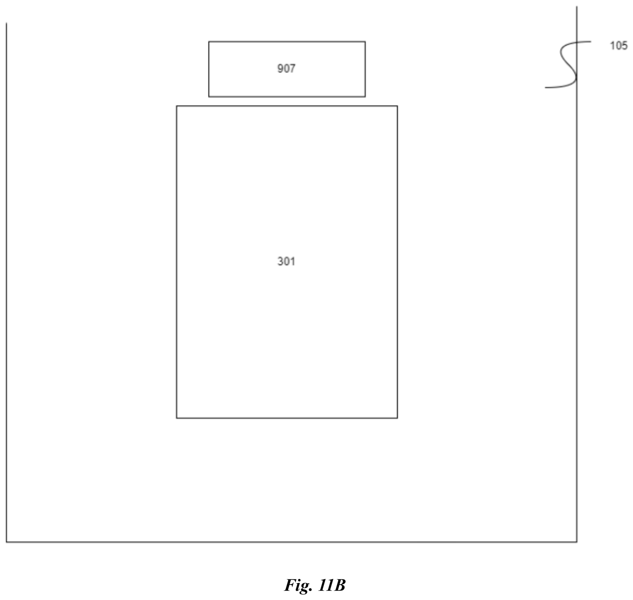

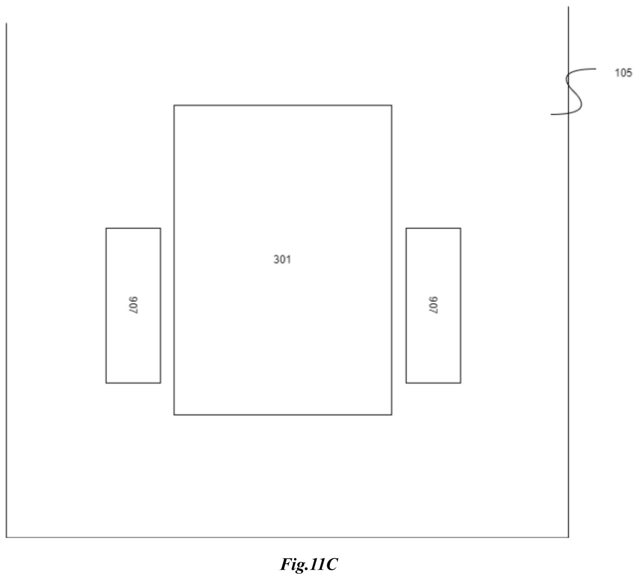

Figs. 11A-C show schematic diagrams of the applicator 105. The applicator 105 may comprise at least one Peltier element 907. The Peltier element 907 may be configured to cool the treatment element 301 and/or to create a reverse thermal gradient in the target tissue. The Peltier element 907 may be located between the treatment element 301 and the adjacent tissue and/or the treatment element 301 may be located between the Peltier element 907 and the adjacent tissue. The at least one Peltier element 907 may be located next to the treatment element 301.

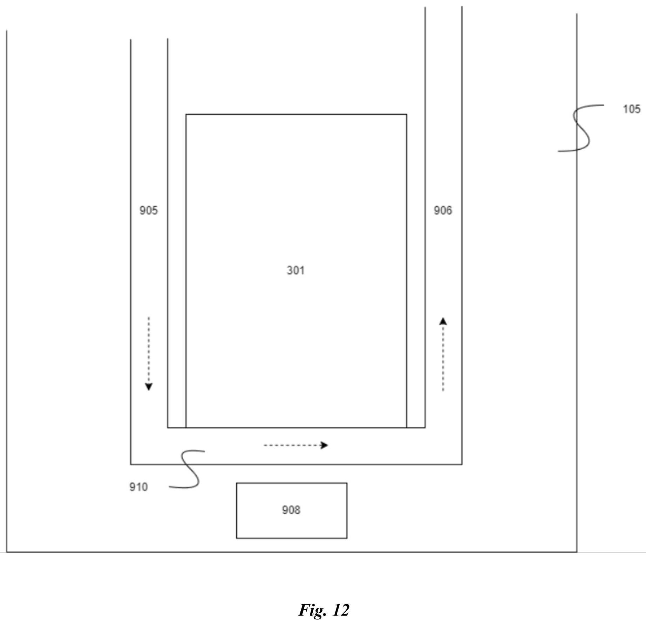

Fig 12 shows a schematic diagram of the applicator 105. The applicator 105 may comprise the inlet tube 904 and the outlet tube 905. The inlet tube 904 and the outlet tube 905 may be configured to circulate the cooling fluid. The applicator 105 may also comprise a Peltier element 907, which may be in communication with the tubing system 902 and the cooling fluid 911.

The Peltier element 907 may be positioned inside the applicator 105, inside the treatment element 301, inside the tubing system, inside the cooling plate 908, and/or inside the pool 906.

The cooling unit 107 may be configured to remove between 0.01 Watts and 100 Watts or 0.01 Watts and 50 Watts of energy from the treated area of tissue.

The cooling unit 107 may be configured to remove between 0.01 Watts and 100 Watts or 0.01 Watts and 50 Watts of power from the treated area of tissue.

The at least two treatment elements 301 (e.g. SIW antennas 501, microwave waveguide, or antenna) in the treatment array 108 may be positioned in applicator 105 in various configurations, depending on the intended use of the device.

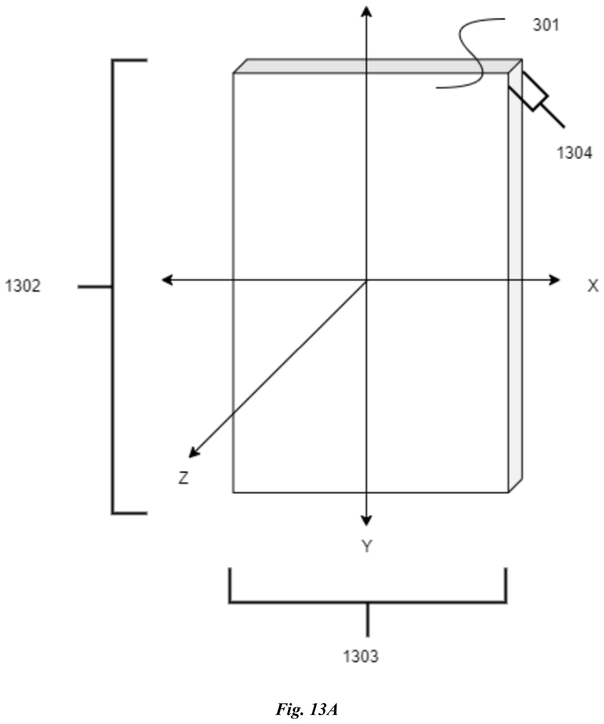

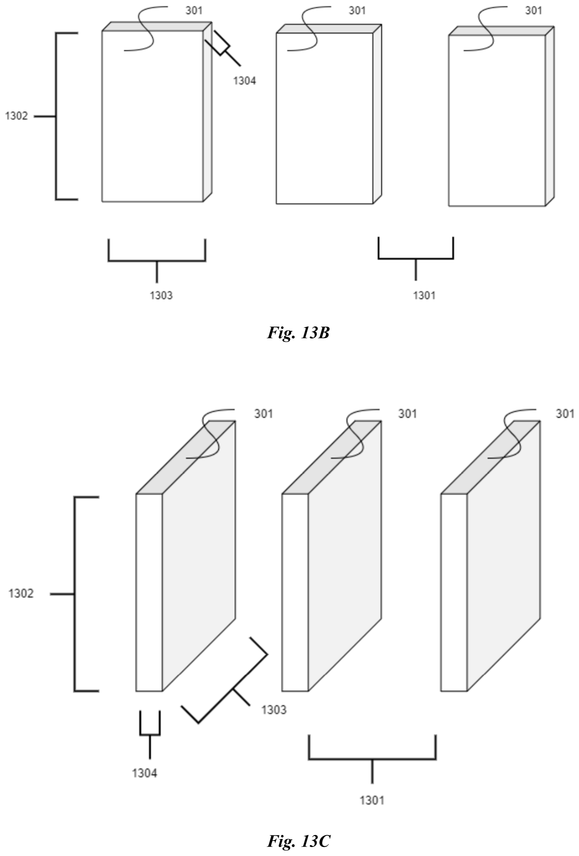

Figure 13A shows a schematic diagram of a treatment element 301. The treatment element 301 may comprise a length 1302, width 1303 and a thickness 1304, wherein the length 1302 multiplied by the width 1303 equals to the surface area of the SIW antenna.

The length 1302 of the treatment element 301 may be in range from 1 mm to 1000 mm, or from 1 mm to 100 mm , or from 1 mm to 100 mm, or from 1 mm to 50 mm, or from 2 mm to 15 mm, or from 5 mm to 50 mm, or from 5 mm to 30 mm, or from 0.5 mm to 5 mm, or from 1 mm to 5 mm, or from 1 mm to 200 mm, or from 100 mm to 200 mm, or from 10 mm to 150 mm, or from 10 mm to 100 mm, or from 10 mm to 80 mm, or from 40 mm to 80 mm, or from 60 mm to 70 mm, or from 50 mm to 100 mm, or from 10 mm to 50 mm, or from 20 mm to 50 mm, or from 30 mm to 40 mm, or from 40 mm to 50 mm, or from 40 mm to 80 mm, or from 50 mm to 80 mm, or from 60 mm to 70 mm.

The width 1303 of the treatment element 301 may be in range from 1 mm to 1000 mm, or from 1 mm to 100 mm , or from 1 mm to 100 mm, or from 1 mm to 50 mm, or from 2 mm to 15 mm, or from 5 mm to 50 mm, or from 5 mm to 30 mm, or from 0.5 mm to 5 mm, or from 1 mm to 5 mm or from 1 mm to 200 mm, or from 100 mm to 200 mm, or from 10 mm to 150 mm, or from 10 mm to 100 mm, or from 10 mm to 80 mm, or from 40 mm to 80 mm, or from 60 mm to 70 mm, or from 50 mm to 100 mm, or from 10 mm to 50 mm, or from 20 mm to 50 mm, or from 30 mm to 40 mm, or from 40 mm to 50 mm, or from 10 mm to 40 mm, or from 20 mm to 40 mm, or from 30 to 40 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm. .

The thickness 1304 of treatment element 301 the may be in a range from 0.001 mm to 100 mm, or from 0.01 mm to 10 mm, or from 0.05 mm to 1 mm, or from 0.1 mm to 1 mm, or from 0.2 mm to 1 mm, or from 0.2 mm to 0.5 mm, or from 0.5 mm to 1 mm, or from 1 mm to 10 mm, or from 1 mm to 5 mm, or from 1.5 mm to 4 mm, or from 2 mm to 4 mm, or from 2 mm to 15 mm, or from 10 mm to 15 mm, or from 1 mm to 15 mm, or from 1 mm to 7 mm, or from 1 mm to 5 mm, or from 3 mm to 4 mm, or from 1 mm to 25 mm, or from 5 mm to 25 mm, or from 8 mm to 25 mm, or from 8 mm to 12 mm, or from 10 mm to 20 mm, or from 15 to 25 mm, or from 8 mm to 12 mm, or from 14 mm to 16 mm, or from 18 mm to 22 mm..

The treatment element 301 may comprise a surface area in a range from 0.01 mm² to 1 ·10⁶ mm², or from 0.01 mm² to 1 ·10 000 mm², or from 1 mm² to 1 ·10 000 mm², or from 1 mm² to 2500 mm², or from 4 mm² to 500 mm², or from 4 mm² to 225 mm², or from 25 mm² to 2500 mm², or from 25 mm² to 1000 mm², or from 25 mm² to 900 mm², 0.25 mm² to 25 mm², or from 1 mm² to 25 mm² , or from 1000 mm² to 5000 mm², or from 1000 mm² to 4000 mm², or from 1500 mm² to 3000 mm², or from 2000 mm² to 2500 mm², or from 2200 mm² to 2400 mm², or from 1000 mm² to 2000 mm².

The treatment element 301 may comprise a volume in a range from 1 · 10⁻⁵ mm³ to 1 · 10⁸ mm³, or from 1 · 10⁻⁴ mm³ to 1 · 10⁵ mm³, or from 0.05 mm³ to 1 · 10 000 mm³, or from 0.1 mm³ to 10 000 mm³, or from 1 mm³ to 7500 mm³, or from 1 mm³ to 5000 mm³, or from 1 mm³ to 2500 mm³, or from 1000 to 5000, or from 100 mm³ to 10 000 mm³, or from 1000 mm³ to 10 000 mm³, or from 5000 mm³ to 10 000 mm³, or from 2500 mm³ to 7500 mm³, or 5 000 mm³ to 50 000 mm³, or from 10 000 mm³ to 40 000 mm³, or from 25 000 mm³ to 35 000 mm³, or from 3000 mm³ to 10 000 mm³, or from 4000 mm³ to 9000 mm³, or from 5000 mm³ to 9000 mm³, or from 7000 mm³ to 8000 mm³, or from 7500 mm³ to 8000 mm³, or from 7000 mm³ to 7500 mm³, or from 10 000 mm³ to 50 000 mm³, or from 10 000 mm³ to 25 000 mm³.

Figures 13B-D show a schematic diagram of various configurations that the at least two treatment elements 301 (for example a microwave antenna, waveguide, SIW antenna and others) may be positioned in the applicator 105.

The treatment elements 301 may comprise axis X, axis Y and axis Z. The X axis of the at least two treatment elements 301 may be rotated in one plane in relation to each other and may have a common angle in a range from 0 ° to 360 °, 0 ° to 180 °, 0 ° to 120 °, 0 ° to 90 °, 0 ° to 45 °,0 ° to 22.5 °, or 1 ° to 18 °.

The Y axis of the at least two treatment elements 301may be rotated in one plane in relation to each other and may have a common angle in a range from 0 ° to 360 °, 0 ° to 180 °, 0 ° to 120 °, 0 ° to 90 °, 0 ° to 45 °,0 ° to 22.5 °, or 1 ° to 18 °.

The Z axis of the at least two treatment elements 301 may be rotated in one plane in relation to each other and may have a common angle in a range from 0 ° to 360 °, 0 ° to 180 °, 0 ° to 120 °, 0 ° to 90 °, 0 ° to 45 °,0 ° to 22.5 °, or 1 ° to 18 °.

The treatment elements 301 may be aligned behind each other. Figures 13B-D show various aligning configurations.

Each treatment element 301 may comprise 1 to 6, or 1 to 3 degrees of freedom. The positioning of the treatment element 301 may result in adjusting the radiation field of the applicator 105.

In one aspect, the treatment elements 301 may be aligned behind each other in the same position as shown in Figures 13B-C. The treatment elements 301 may be adjacent to each other, or may have a spacing distance between each other. In another aspect, the treatment elements 301 may be aligned behind each other and may be tilted towards each other as shown in Figure 13D.

The X axis of two treatment elements 301 may have an angle in a range from 0 ° to 360°, or 0 ° to 180 °, or from 170 ° to 190 °, or from 0 ° to 10 °.

In another aspect three treatment elements 301 may be aligned behind each other as shown in Figures 13B-D. The three treatment elements 301 may have an angle in a range from 0 ° to 360°, or 0 ° to 180 °, or from 170 ° to 190 °, or from 0 ° to 10 °, or from 110 ° to 130 °.

The at least two treatment elements 301 may be separated by a spacing distance 1301. The spacing distance may be in range from 0.001 mm to 1000 mm, or from 0.01 mm to 100 mm, or from 0.1 mm to 75 mm, or from 0.1 mm to 50 mm, or from 0.1 mm to 25 mm, or from 0.1 mm to 10 mm, or from 0.1 mm to 1 mm, or from 1 mm to 100 mm, or from 1 mm to 75 mm, or from 1 mm to 50 mm, or from 1 mm to 25 mm, or from 10 mm to 50 mm, or from 15 mm to 35 mm.

Multiple applicators 105 may be used at the same time, allowing a wider variability of the treatment. For example, the treatment depth, the irradiated tissue volume, focusing, and induced thermal gradient in the soft tissue may vary. Communication between multiple applicators 105 and between each of the applicators 105 and the power supply 102, the control unit 103, the user interface 104, one or more sensors 106, cooling unit 107, and one or more treatment arrays 108 may be employed, ensuring more efficient and safe treatment.

In a specific example, at least two applicators 105 may be provided.

Multiple therapies may run separately or may be combined at the same time. This may improve the efficiency of the treatment and may reduce the time needed for the treatment.

The method of treatment of the subject may comprise additional steps. For example, radio frequency, electrostimulation, plasma beam, laser, and others may be applied.

The size of one or more applicators 105 may vary based on the treated tissue and the treated body region. The one or more applicators 105 may have a circular, elliptical, square, rectangular, triangular, or other various shapes. One or more applicators 105 may have a symmetrical or asymmetrical shape. One or more applicators 105 may have a surface in a range from 1 cm² to 1·10⁴ cm², or from 1 cm² to 1000 cm², or from 1 cm² to 500 cm², or from 1 cm² to 100 cm², or from 10 cm² to 100 cm². The applicator 105 may have adjustable size and length and may be flexible. The applicator 105 may bend or may be constructed from a rigid material.

The device may include a user interface 104. The user interface 104 may be included in the main unit 101 and/or in one or more applicators 105. The user interface 104 may include at least one button, a stop button, LCD display, keyboard, switch, and/or a circular control element. The user interface 104 may show various parameters that are relevant to the ongoing treatment. The parameters may be at least one of the temperature of the treated soft tissue, the temperature of the applicator 105, frequency of the stimulation signal, power of the stimulation signal, temperature of the cooling unit 107, the flow rate of the cooling fluid, the temperature of the cooling fluid, the impedance of the treated tissue, the position of one or more applicators 105, treatment time, quality of the contact between the applicator 105 and/or one or more treatment arrays 108 and the skin surface, information about the subject and/or error messages. The user interface 104 may be in communication with the main unit 101, the power supply 102, the control unit 103, the one or more applicators 105, the one or more sensors 106, the cooling unit 107, or one or more treatment arrays 108.

The operator may set various treatment parameters and various treatment procedures through the user interface 104. The treatment parameters may include at least the temperature of the applicator 105, the temperature of the treated soft tissue, the temperature of the microwave antenna, the impedance of the microwave antenna, treatment time, rate of cooling, the temperature of the cooling unit 107, the temperature of the cooling fluid, the output energy and/or output power and pressure. Treatment parameters may be changed before the treatment or during the treatment. The software may include one or more predefined protocols, from which the operator may choose the most suitable one. The operator may also create his own protocol.

The device may include a database of subjects, providing information about the subjects and providing information about previous/ongoing treatments. The device may include a counter, which is able to count the number of performed therapies, the time of providing specific therapies, used treatment parameters, and others. Data from the counter may be stored in the device's memory or in external storage. The device may be able to download new treatment protocols and/or new software.

The applicator 105 may be an internal applicator. The internal applicator may be configured to be positioned into and to treat body canals and cavities, such as the vagina, vulva, cervix, urethra, rectum, anus, bladder, veins, nose and mouth. The internal applicator may be of a shape suitable to be inserted into cavities, such as tubular, circular, phallic and others.

The internal applicator may comprise one or more detachable parts. The one or more detachable parts may be removably coupled to the applicator handle. The one or more detachable parts may include one or more treatment elements 301. The one or more treatment elements 301 may also be positioned in the applicator handle. The one or more detachable parts may not include any treatment elements 301.

Detachable parts may be combined to create an applicator of any size, shape, length, width, type of energy or elasticity. The one or more detachable parts may be reusable or disposable. A detachable part containing one treatment element 301 may be replaced with a detachable part that contains two or more treatment elements 301 if needed.

In the internal applicator, the treatment elements 301 (e.g. SIW antennas 501) may be aligned behind each other as shown in Figures 13B-D. In one aspect, the internal applicator may comprise a number of treatment elements 301 (e.g. SIW antennas) such that the internal applicator is configured to radiate in a circular manner. The neighboring treatment elements 301 (e.g. SIW antennas 501) may have X axes tilted towards each other such that the X axes have a common angle in a range from 0 ° to 180 °, or from 170 ° to 190 °, or from 0 ° to 10 °, or from 110 ° to 130 °, or from 80 ° to 110 °, or from 70 ° to 80 °, or from 50 ° to 70 °, or from 45 ° to 55 °, or from 40 ° to 50 °, or from 30 ° to 40 °, or from 20 ° to 40 °, or from 10 ° to 30 °, or from 5 ° to 15 °.

The applicator 105 may include at least one sensor 106. The sensor 106 may be, for example, a temperature sensor, an impedance sensor and/or a force sensor. In one aspect, the sensor 106 may be utilized to verify proper attachment of the applicator 105 to the body part and/or skin surface.

The applicator 105 may include a force sensor. The force sensor may be configured to indicate that the applicator 105 is correctly positioned on the body part and that the applicator 105 is in contact with the body part. The force sensor may be configured to detect and measure the amount of force applied by the operator and/or automated mechanism. When the force measured by the force sensor reaches a predetermined threshold, it indicates that the applicator 105 is in a correct position and the treatment may start. The force sensor is in communication with the control unit 103. The force detection may be essential for activating specific treatment steps and ensuring optimal skin contact and treatment consistency.

In one aspect of the invention, a method of microwave treatment may comprise the following steps: placing the applicator in contact with the target area of the tissue, detecting the contact using one or more sensors, performing a pre-cooling to prepare the target area of the tissue, applying electromagnetic energy in the form of one or more pulses through the treatment element, and measuring reflected waves during the energy application using sensors configured for this purpose. Following the energy delivery, the method may further include a post-cooling to reduce residual heat in the target area of the tissue. Each step of the method may be monitored and adjusted based on feedback from integrated sensors, ensuring safe and effective treatment.

The device may comprise at least one sensor, wherein the at least one sensor may be in the form of an electrode. In one aspect, the sensor (e.g. electrode) may be part of the applicator. The electrode sensor may be configured to measure phase shift and/or impedance. The electrode sensor may provide a different type of energy than the treatment array or treatment element. The sensor may provide information about the contact between the applicator and the skin. In one aspect, the electrode sensor may be used to determine the thickness of the treated tissue layers. The sensor may also provide feedback on tissue conductivity during treatment, enabling adjustments to energy delivery parameters. The feedback provided by the sensor may allow automatic optimization of treatment efficacy and safety by adjusting power and/or cooling in real-time.

The device may comprise at least one sensor, e.g., a reflection sensor, wherein the sensor may be used for measuring the reflection coefficient or impedance of reflected waves, wherein the sensor may be positioned in the vicinity of the treatment element.

The device may comprise at least one sensor, e.g., a reflection sensor, wherein the sensor may be used for measuring the reflection coefficient or impedance of reflected waves, wherein the sensor may be positioned in the main unit of the device.

Without being bound by a theory, when the EM radiation is applied to the tissue comprising different structures (e.g., skin, fat, muscle, bone), some of the EM radiation is reflected back to the treatment element depending precisely on the tissue structure. The reflection coefficient may comprise information about the amplitude of the reflected wave or the phase shift of the reflected waves of the EM radiation. When the reflection sensor measures the reflection coefficient, it may be possible to determine the structure of the tissue, e.g., the thickness of each layer (skin, fat, muscle, bone), and it may even be possible to determine the treatment area of the body part. As an example, by measuring and determining the reflection coefficient, it may be possible to determine whether we treat the forehead or cheek of the patient or even if we treat a part of the cheek comprising a thick fat layer or a part comprising a thin, fat layer.

The device may comprise at least one sensor configured to measure the reflection coefficient, wherein the sensor may be positioned adjacent to the treatment element to facilitate accurate and real-time monitoring of reflections during operation. By the optimized position of the reflection sensor close to the treatment element, it is possible to eliminate signal loss and interferences compared to the case when the reflection sensor is not positioned in the applicator but instead is in the main unit connected with the applicator by the connecting tube. The feedback provided by the sensor to the control unit may allow automatic optimization of treatment efficacy and safety by adjusting power and/or pulse duration and/or cooling in real-time.

Simultaneously, additional sensors, such as impedance and/or temperature sensors may be engaged to assess the skin's condition and ensure optimal treatment parameters. These sensors provide real-time feedback on tissue impedance and temperature, allowing the system to adjust treatment settings accordingly.

The integration of these sensors not only enhances treatment precision but also contributes to patient safety. The system can detect and respond to deviations in skin impedance or temperature, automatically adjusting treatment parameters to ensure optimal outcomes and reduce the risk of adverse effects.

The applicator 105 may comprise at least one button. The at least one button may be in communication with the control unit 103, the power supply 102, the user interface 104, one or more sensors 106, cooling unit 107, and/or one or more treatment arrays 108. The at least one button may be configured to start and/or end the treatment or may be configured to indicate the intention of the operator to start and/or end the treatment.

The applicator 105 may comprise a sliding mechanism. When the operator and/or automated mechanism places the applicator 105 in contact with the body part and/or skin and applies force to the applicator 105, the sliding mechanism may be engaged and may slide into the applicator 105. The sliding action may trigger the force sensor, which detects the applied force and confirms the readiness of the applicator 105 for the treatment.

During the treatment, various indication mechanisms may be used to signal the course and various periods of the treatment to the operator and/or automated mechanism. Such mechanisms may be, for example, sound mechanisms, visual mechanisms, vibrational mechanisms, mechanical mechanisms or others. Such a mechanism may ensure a continuous and safe course of the treatment and may simplify the execution of the treatment for the operator.

The applicator 105 may comprise a treatment array 108. The treatment array 108 may comprise at least one treatment element 301. The treatment array 108 may be configured to deliver at least one energy pulse, for example, a microwave energy pulse.

The energy delivery pulse may have a duration in a range from 0.001 s to 3600 s, or from 0.001 s to 1000 s, or from 0.001 s to 500 s, or from 0.001 s to 100 s, or from 0.001 s to 50 s, or from 0.001 s to 25 s, or from 0.001 s to 10 s, or from 0.001 s to 5 s, or from 0.001 s to 1 s, or from 0.01 s to 1000 s , or from 0.1 s to 1000 s, or from 1 s to 1000 s, or from 5 s to 1000 s, or from 10 s to 1000 s, or from 50 s to 1000 s, or from 100 s to 1000 s, or from 500 s to 1000 s, or from 0.1 s to 500 s , or from 0.1 s to 100 s, or from 0.1 s to 75 s, or from 0.1 s to 50 s, or from 0.1 s to 25 s, or from 0.5 s to 100 s, or from 0.5 s to 75 s , or from 0.5 s to 50 s, or from 0.5 s to 25 s, or from 0.5 s to 15 s, or from 0.5 s to 10 s, or from 0.5 s to 5 s, or from 0.5 to 2 s, or from 0.5 to 1 s. In one aspect of the device, the device may comprise treatment protocols. The treatment protocols may be selectable by a user via the user interface. In one aspect, the user may set some parameters of the treatment protocol, e.g., intensity, output power, or pulse duration.

In some aspects, the energy delivery pulse may have a duration in a range of from 1 ms to 50 ms, or from 5 ms to 20 ms, or from 8 ms to 15 ms, or from 9 ms to 12 ms, or from 9.5 ms to 10.5 ms, or from 1 ms to 2 s, or from 5 ms to 1.5 s, or from 10 ms to 1 s, or from 0.01 s to 2 s, or from 0.01 s to 1.2 s.

In one aspect of the invention, the device may comprise a protocol for the application of electromagnetic energy to the SMAS. The protocol may involve placing the applicator in contact with the skin and initiating the delivery of energy only after proper contact is established. The treatment element may emit electromagnetic energy at a specific frequency, e.g. frequency of 2.4 GHz. The duration of the electromagnetic pulses applied to the skin may be in a range of 0.1 to 5 seconds, or in a range of 0.3 to 4.5 seconds, or in a range of 1 to 3.9 seconds, or in a range of 1.5 to 2.75 seconds. In one aspect, cooling may be implemented before the application of electromagnetic pulses, during the exposure to electromagnetic pulses, or after the exposure. The duration of the cooling may range from 0.5 to 15 seconds, or in a range of 2 to 13 seconds, or in a range of 4 to 10 seconds, or in a range of 6 to 9 seconds.

In one aspect of the invention, the device may comprise a protocol for the application of electromagnetic energy to the epidermis and/or dermis of the skin. The protocol may involve placing the applicator in contact with the skin and initiating the delivery of energy only after proper contact is established. The treatment element may emit electromagnetic energy at a specific frequency, e.g. at a frequency of 2.4 GHz, 5.8 GHz, or 24 GHz. The duration of the electromagnetic pulses applied to the skin may be in a range of 0.1 to 5 seconds, or in a range of 0.3 to 4.5 seconds, or in a range of 1 to 3.9 seconds, or in a range of 1.5 to 2.75 seconds or it may be in a range of 2.5 to 15 seconds, or in a range of 4 to 13 seconds, or in a range of 6 to 11 seconds, or in a range of 7 to 9 seconds. In one aspect, cooling may be implemented before the application of electromagnetic pulses, during the exposure to electromagnetic pulses, or after the exposure. The duration of the cooling may range from 0.5 to 15 seconds, or in a range of 2 to 13 seconds, or in a range of 4 to 10 seconds, or in a range of 6 to 9 seconds.

In one aspect of the invention, the device may comprise a protocol for the application of electromagnetic energy to the adipose tissue. The protocol may involve placing the applicator in contact with the skin and initiating the delivery of energy only after proper contact is established. The treatment element may emit electromagnetic energy at a specific frequency, e.g. a frequency of 2.4 GHz. The duration of the electromagnetic pulses applied to the skin may be in a range of 0.5 to 1 seconds, or in a range of 0.3 to 4.5 seconds, or in a range of 1 to 3.9 seconds, or in a range of 1.5 to 2.75 seconds or it may be in a range of 2.5 to 15 seconds, or in a range of 4 to 13 seconds, or in a range of 6 to 11 seconds, or in a range of 7 to 9 seconds or it may be in a range of 11 to 20 seconds, or in a range of 12.5 to 18.4 seconds, or in a range of 14 to 16 seconds. In one aspect, cooling may be implemented before the application of electromagnetic pulses, during the exposure to electromagnetic pulses, or after the exposure. The duration of the cooling may range from 0 to 15 seconds, or in a range of 2 to 13 seconds, or in a range of 4 to 10 seconds, or in a range of 6 to 9 seconds or it may be continuously cooled during the whole treatment process.

In one aspect of the invention, the one pulse may have power in a range from 1 Watt to 1000 Watts, or in a range from 250 Watts to 750 Watts, or in a range from 450 Watts to 550 Watts.

In another aspect, the one pulse may have power in a range of 1 W to 550 W, or in a range of 5 W to 450 W, or in a range of 12 W to 400 W, or in a range of 15 W to 300 W, or in a range of 17 W to 275 W, or in a range of 19 W to 225 W, or in a range of 26 W to 187 W.

The applicator 105 may be configured to apply at least one energy delivery pulse after the applicator 105 is attached to the body part. In another aspect, the applicator 105 may be configured to apply at least two consecutive energy delivery pulses after the applicator 105 is attached to the body part.

The applicator 105 may be in communication with the cooling unit 107. The cooling unit 107 may provide cooling to the parts of the applicator 105 and/or to the body part and/or the skin of the body part. The cooling may be provided during the treatment.

In one aspect, cooling by the cooling unit 107 may be divided into periods, such as a pre-cooling period, cooling in combination with energy delivery (such as for example microwave energy delivery) period and/or post-cooling period.

The pre-cooling period may start once the applicator 105 is attached to the body part. The tissue may be pre-cooled to a predefined temperature or for a predefined time. In one aspect, the temperature of the tissue may be measured by a temperature sensor, other change of the tissue temperature may be indicated by, for example, an impedance sensor. Then, at least one energy delivery pulse may be delivered to the tissue. During the energy delivery, the cooling in combination with energy delivery period may take place. After at least one energy delivery pulse is delivered to the tissue, the post-cooling period may start. The tissue may be post-cooled to a predefined temperature or for a predefined time. When the post-cooling period is over, the operator and/or automated mechanism may elevate the applicator 105 from the body part and/or skin.

The cooling may have a duration in a range from 0.001 s to 3600 s, or from 0.001 s to 1000 s, or from 0.001 s to 500 s, or from 0.001 s to 100 s, or from 0.001 s to 50 s, or from 0.001 s to 25 s, or from 0.001 s to 10 s, or from 0.001 s to 5 s, or from 0.001 s to 1 s, or from 0.01 s to 1000 s , or from 0.1 s to 1000 s, or from 1 s to 1000 s, or from 5 s to 1000 s, or from 10 s to 1000 s, or from 50 s to 1000 s, or from 100 s to 1000 s, or from 500 s to 1000 s, or from 0.1 s to 500 s , or from 0.1 s to 100 s, or from 0.1 s to 75 s, or from 0.1 s to 50 s, or from 0.1 s to 25 s, or from 0.5 s to 100 s, or from 0.5 s to 75 s , or from 0.5 s to 50 s, or from 0.5 s to 25 s, or from 0.5 s to 15 s, or from 0.5 s to 10 s, or from 0.5 s to 5 s.

The pre-cooling period may have a duration in a range from 0.001 s to 3600 s, or from 0.001 s to 1000 s, or from 0.001 s to 500 s, or from 0.001 s to 100 s, or from 0.001 s to 50 s, or from 0.001 s to 25 s, or from 0.001 s to 10 s, or from 0.001 s to 5 s, or from 0.001 s to 1 s, or from 0.01 s to 1000 s , or from 0.1 s to 1000 s, or from 1 s to 1000 s, or from 5 s to 1000 s, or from 10 s to 1000 s, or from 50 s to 1000 s, or from 100 s to 1000 s, or from 500 s to 1000 s, or from 0.1 s to 500 s , or from 0.1 s to 100 s, or from 0.1 s to 75 s, or from 0.1 s to 50 s, or from 0.1 s to 25 s, or from 0.5 s to 100 s, or from 0.5 s to 75 s , or from 0.5 s to 50 s, or from 0.5 s to 25 s, or from 0.5 s to 15 s, or from 0.5 s to 10 s, or from 0.5 s to 5 s, or from 0.1 s to 5 s, or from 0.1 s to 2 s, or from 0.1 s to 1 s, or from 0.1 s to 0.8 s.

The cooling in combination with energy delivery period may have a duration in a range from 0.001 s to 3600 s, or from 0.001 s to 1000 s, or from 0.001 s to 500 s, or from 0.001 s to 100 s, or from 0.001 s to 50 s, or from 0.001 s to 25 s, or from 0.001 s to 10 s, or from 0.001 s to 5 s, or from 0.001 s to 1 s, or from 0.01 s to 1000 s , or from 0.1 s to 1000 s, or from 1 s to 1000 s, or from 5 s to 1000 s, or from 10 s to 1000 s, or from 50 s to 1000 s, or from 100 s to 1000 s, or from 500 s to 1000 s, or from 0.1 s to 500 s , or from 0.1 s to 100 s, or from 0.1 s to 75 s, or from 0.1 s to 50 s, or from 0.1 s to 25 s, or from 0.5 s to 100 s, or from 0.5 s to 75 s , or from 0.5 s to 50 s, or from 0.5 s to 25 s, or from 0.5 s to 15 s, or from 0.5 s to 10 s, or from 0.5 s to 5 s, or from 0.5 to 2 s, or from 0.5 to 1 s, or from 0.1 s to 0.8 s, or from 0.2 s to 0.5 s.

The post-cooling period may have a duration in a range from 0.001 s to 3600 s, or from 0.001 s to 1000 s, or from 0.001 s to 500 s, or from 0.001 s to 100 s, or from 0.001 s to 50 s, or from 0.001 s to 25 s, or from 0.001 s to 10 s, or from 0.001 s to 5 s, or from 0.001 s to 1 s, or from 0.01 s to 1000 s , or from 0.1 s to 1000 s, or from 1 s to 1000 s, or from 5 s to 1000 s, or from 10 s to 1000 s, or from 50 s to 1000 s, or from 100 s to 1000 s, or from 500 s to 1000 s, or from 0.1 s to 500 s , or from 0.1 s to 100 s, or from 0.1 s to 75 s, or from 0.1 s to 50 s, or from 0.1 s to 25 s, or from 0.5 s to 100 s, or from 0.5 s to 75 s , or from 0.5 s to 50 s, or from 0.5 s to 25 s, or from 0.5 s to 15 s, or from 0.5 s to 10 s, or from 0.5 s to 5 s, or from 0.5 to 2 s, or from 0.5 to 1 s, or from 0.1 s to 0.8 s, or from 0.2 s to 0.5 s.

The treatment and the individual steps of the treatment may be controlled and performed by an operator (such as, for example, a surgeon) or by an automated mechanism controlled by the controller unit 103 that consecutively executes individual steps of the treatment (such as, for example, attaching the applicator 105 to the body part, pre-cooling, firing the energy pulse, cooling during the energy delivery, stopping the energy pulse, post-cooling, and lifting the applicator 105 from the body part). Some steps of the treatment may be performed automatically, and some may be performed by the operator.

In one aspect of the device, to initiate the treatment, the operator may begin by pressing the at least one button while the applicator 105 is positioned in the air, signaling the intent to start the treatment procedure. Then, the operator and/or automated mechanism places the applicator 105 in the desired position, contacting the body part. During this step, the integrated force sensor detects the force exerted by the operator and/or automated mechanism. The applicator 105 may comprise a sliding mechanism. As the operator and/or automated mechanism positions the applicator 105 near the skin surface, the sliding mechanism slides in and the force sensor detects the applied force when the operator and/or automated mechanism presses the applicator 105 against the skin. This action triggers the control unit 103, confirming proper skin contact and readiness for the treatment.

In one aspect, the additional sensor (e.g. impedance or temperature sensor) may further measure relevant quantities, which may be determined by the control unit and confirm the proper contact of the applicator with the body part.

Upon establishing firm contact with the body part, the system initiates a pre-cooling period. In one aspect, the system initiate a pre-cooling period for a predetermined time. In another aspect, during this period, the temperature sensor monitors the skin's surface temperature, ensuring that the tissue is adequately cooled to a predetermined level before proceeding to the next treatment phase. The tissue is cooled by the cooling unit until it reaches a predetermined temperature threshold.

The parameters of cooling may be the same during the whole treatment process or may change based on a pre-defined cooling pattern or based on the state of the body part.

Once the tissue reaches the desired temperature, the system may automatically trigger the energy pulse (for example a microwave energy pulse). The parameters of the energy pulse may be tailored to the skin conditions, level of pre-cooling and predefined treatment parameters. During the treatment, the tissue may be cooled by the cooling unit 107. Cooling during the treatment, pre-cooling and post-cooling may have the same parameters or may have different parameters.

The parameters of the cooling may be, for example, the duration of the cooling, the duration of pre-cooling, the duration of cooling in combination with energy delivery, the duration of post-cooling, the temperature of the cooling fluid, the flow rate of the cooling fluid, the power of the cooling unit 107 and others.

The state of the body part may be, for example, the temperature of the body part, the impedance of the body part, the force applied to the body part and others.

The cooling unit 107 may be in communication with the control unit 103 and at least one sensor 106.

During the treatment session, the force sensor may continue to monitor the force applied by the operator and/or automated mechanism, ensuring consistent and effective treatment delivery. The force sensor may provide feedback to indicate whether the applied force is within optimal operational thresholds.

Upon completion of the energy pulse delivery the cooling unit 107 may start a post-cooling period. The temperature sensor may regulate the post-treatment cooling period, ensuring patient comfort, and minimizing post-treatment side effects.

An indication mechanism may be used to signal the pressing of the first button, prompting the operator and/or automated mechanism to attach the applicator 105 on the body part. Further, an indication mechanism may be used to signal the correct attachment of the applicator 105 to the body part, the incorrect attachment of the applicator 105 to the body part, start of the cooling, end of the cooling, start of the energy pulse, and end of the cooling pulse, prompting the operator and/or automated mechanism to lift the probe from the body part, and/or marking the end of the treatment session.

This automated and feedback-driven method may ensure precise treatment timing and consistency, minimizing operator and/or automated mechanism error and optimizing patient outcomes.

In addition to force detection, the applicator 105 may incorporate various safety features, including error detection mechanisms triggered by premature applicator 105 detachment or other operational anomalies. These safety measures may enhance treatment reliability and patient safety during the treatment.

In another aspect of the applicator, incorporated safety features may comprise shut-down of the system.

In one aspect of the invention, some of the individual steps of the treatment may be automated and some may be performed by the operator. The operator may start and end the treatment. Other individual steps of the treatment are executed by the automated mechanism and controlled by the controller unit 103.

In another aspect of the invention, some steps of the treatment may be automated, and some steps of the treatment may be performed by the operator. For example, the operator may indicate the intention to start the treatment and may attach the applicator 105 to the body part. The other individual steps of the treatment, such as, for example, pre-cooling, firing the energy pulse, cooling during the energy delivery, stopping the energy pulse and post-cooling may be executed by an automated mechanism and controlled by the control unit 103.

In another aspect of the invention, all of the individual steps may be performed by the operator.

In other aspects of the invention, any combination of automated steps and steps performed by the operator may be utilized.

In one aspect of the invention, the device may be configured to provide microwave treatment to treat SMAS. The device may comprise an applicator configured to be in contact with tissue of the subject's body, wherein the applicator may comprise a treatment array configured to radiate a microwaves to the subject's body comprising SMAS causing a heating of the subject's body. The device may comprise a cooling unit comprising a circulating cooling fluid configured to create a reverse thermal gradient in the tissue of the subject's body.

The device may comprise a spacing element configured to improve microwave radiation transfer to the tissue and having a relative permittivity in a range from 1 to 150. The device may comprise a spacing element configured to improve microwave radiation transfer to the tissue and having a thickness in a range from 0.001 mm to 100 mm. The SMAS may be heated to a temperature in a range from 37.5 °C to 90 °C. The device may comprise at least one sensor, wherein the at least one sensor may be a temperature sensor configured to measure the skin temperature. The device may comprise at least one sensor, wherein the at least one sensor may be an impedance sensor configured to provide information about the coupling of the applicator.

In another aspect of the invention, the device may be configured to provide microwave treatment to treat subjects' body. The device may comprise an applicator configured to be in contact with a skin of the subject's body, The applicator may comprise a treatment element comprising a top metallic plate, a bottom metallic plate and at least one post. The at least one post may form an interconnection between the top metallic plate and the bottom metallic plate. The at least one post may be configured to generate microwaves. The treatment element may be configured to radiate microwaves to the subject's body. The applicator may comprise a cooling plate comprising at least one channel comprising a circulating cooling fluid configured to create a reverse thermal gradient in the subject's body.

The at least one post may have a diameter in a range from 0.01 mm to 10 mm. The treatment element may have a volume in a range from 10 mm³ to 50,000 mm³. The treatment element may comprise a substrate having a relative permittivity in a range from 1 to 150. The treatment element may comprise a substrate having a dielectric strength in a range from 5 MV/m to 50 MV/m. The device may comprise a cooling plate comprising at least one channel, wherein the at least one channel may have a diameter in a range from 0.01 mm to 10 mm. The temperature of the cooling fluid may be in a range from 0 °C to 27 °C.

The at least one channel in the cooling plate may be configured as a microchannel, groove, or tubular conduit for the circulation of a cooling fluid. The channel may have a circular, elliptical, or rectangular cross-section, and may have a hydraulic diameter in a range from 0.01 mm to 10 mm, or from 0.05 mm to 5 mm, or from 0.1 mm to 2 mm depending on the desired cooling capacity and fluid dynamics.

The length of the channel may range from 1 mm to 500 mm, or from 5 mm to 200 mm, and may be configured in a straight, serpentine, or spiral path to enhance heat exchange. The surface roughness of the inner wall of the channel may be optimized for laminar or turbulent flow, with an Ra value in a range from 0.01 µm to 5 µm.

The material of the channel or the cooling plate may be thermally conductive and biocompatible, such as aluminum, copper, stainless steel, ceramics, or high-performance polymer composites. The wall thickness of the channel may range from 0.05 mm to 2 mm, depending on mechanical strength and thermal resistance requirements.

In yet another aspect of the invention, the device for microwave treatment to treat subject's body may comprise at least one applicator configured to be in contact with tissue of the subject's body, wherein the applicator may comprise a treatment element configured to radiate microwaves to the subject's body causing a heating of the tissue between adipose tissue and muscles. The device may comprise a cooling unit comprising a circulating cooling fluid configured to create a reverse thermal gradient in the tissue of the subject's body.

The treatment element may be a waveguide comprising a loading material, wherein the dielectric strength of the loading material may be in range from 0.1 MV/m to 100 MV/m. The treatment element may be a waveguide comprising a material having an electrical conductivity in a range 4 ·10⁶ S/m to 7 ·10⁷ S/m. The treatment element may be a waveguide having a length in a range from 0.1 mm to 200 mm. The cooling fluid may have a flow rate in a range from 0.01 L/minute to 100 L/minute. The device may comprise at least one sensor, wherein the at least one sensor may be a sensor measuring the reflection of the electromagnetic waves. The radiated microwaves may have a frequency in a range from 1 GHz to 10 GHz.

The applicator may comprise at least one shielding element, wherein this element may be used to suppress the reflection of the electromagnetic waves and may be located within the applicator. The shielding element may comprise metal mash around and/or at the bottom of the treatment element, carbon-loaded foam around and/or at the bottom of the treatment element, ferrite tiles around and/or at the bottom of the treatment element or any other appropriate element used for the suppression of the reflection of the electromagnetic waves.

In some aspects, the shielding element may comprise metal, or carbon-loaded foam, or ferrite tiles around and/or at the bottom of the treatment element, or any other appropriate material used for the suppression of the reflection of the electromagnetic waves.

The device may comprise at least one sensor, wherein the at least one sensor may be a sensor measuring the reflection of the electromagnetic waves. The sensor for measuring the reflection of electromagnetic waves may be used to provide information about the coupling and/or the contact with the skin. The sensor for measuring the reflection of electromagnetic waves may be used to provide information about proximity to the skin surface. The sensor for measuring the reflection of electromagnetic waves may also be used to provide feedback on how the skin is responding to the treatment. The sensor for measuring the reflection of electromagnetic waves may be also used to detect excessive absorption of the radiation by the skin, which could lead to overheating. The feedback provided by the sensor may be able to prevent overheating.

In another aspect of the invention the device may provide microwave treatment of the patient tissue. The device may comprise a generator and/or an amplifier configured to generate and/or amplify a power for microwave radiation, a user interface configured to provide a treatment protocol which may be selected by a user of the device and a control unit configured to control the generator or the amplifier by the selected treatment protocol. The device may further comprise an applicator configured to be in contact with tissue of the subject's body, wherein the applicator may comprise a treatment element coupled with the generator and/or the amplifier and configured to radiate microwaves to the subject's body causing a heating of the subject's body. The device may further comprise a connecting tube configured to connect the applicator with the generator and/or the amplifier. The device may comprise a cooling unit comprising a circulating cooling fluid configured to create a reverse thermal gradient in the tissue of the subject's body.

In one aspect, the method of treatment may comprise an initial step of identifying a sensitivity-responsive area on the patient's face. This may involve manual palpation, patient feedback, thermal threshold testing, or other sensory response evaluation techniques. Based on the identified sensitive location, the system may be configured to automatically or manually adjust one or more treatment parameters, such as energy output, pulse duration, duty cycle, or treatment depth. This adaptive approach may allow for personalized treatment settings tailored to the patient's individual sensitivity profile, thereby improving both treatment efficacy and overall comfort.

In one aspect of the invention, the delivered microwave energy may be used as a controlled deep-heating source within musculoskeletal structures and may be used to complement physiotherapeutic methods such as manual therapy, stretching, or mobilization techniques. The microwave energy may increase tissue temperature, may enhance tissue extensibility, may reduce muscle stiffness, and may improve the mechanical effectiveness of subsequent physiotherapeutic interventions.

In one aspect of the invention, the microwave energy may increase tissue temperature to enhance tissue elasticity or flexibility, reduce muscle stiffness, and improve mechanical response, wherein the temperature shift may be detected by at least one sensor within the applicator and may be used to regulate heating in a closed-loop manner. In another aspect of the invention, the temperature of the skin or underlying tissue may be increased by an amount in the range of 1 to 20 °C, or in a range of 3 to 15 °C, or in a range of 10 to 12 °C, depending on the anatomical region, baseline perfusion, and intended physiotherapeutic response.

In another aspect of the invention, the microwave energy may be used in sports medicine and rehabilitation settings to deliver controlled deep heat to musculoskeletal structures, which may support post-injury recovery, may reduce muscular tension, and may promote tissue regeneration.

The microwave heating profile may be used to facilitate stretching, mobilization, or proprioceptive neuromuscular facilitation techniques. The device may be used to treat areas such as tendons, ligaments, muscle compartments, and joint capsules.

In one aspect of the invention, the microwave energy generated by the treatment element may be used to target physiologically relevant structures such as fascia, tendons, ligaments, muscle fibers, or vascular networks. The applicator may be configured to form field maxima at predefined depths, which may be used to selectively heat or stimulate physiotherapeutic target zones. Beam-shaping parameters, slot geometry, frequency tuning, dielectric loading, or phase control may be used to adjust penetration depth and intensity distribution according to physiotherapy goals.

In one aspect of the invention, the microwave frequency to achieve a penetration depth may be in a range of 1 to 30 mm, or in a range of 2 to 15 mm, or in a range 3 to 10 mm, depending on tissue type. In another aspect of the invention, the frequency of microwave energy may be in a range of 1 to 10 GHz, or in a range 2 to 8 GHz, or in a range 3 to 6 GHz, wherein this frequency may be used for deeper structures such as muscle or fascia.

## Claims

1. A device for delivering microwave energy to a tissue, comprising:
at least one treatment element configured to emit microwave energy, wherein the microwave energy is used for the treatment of the tissue of a patient.

2. The device according to claim 1, wherein the treatment element has
(a) a length in a range from 1 mm to 100 mm,
(b) a width in a range from 1 mm to 100 mm, and/or
(c) a thickness in a range from 0.001 mm to 100 mm.

3. The device according to any of the preceding claims, wherein the treatment element comprises a microwave waveguide configured to emit microwave energy toward the tissue, the waveguide comprising:
(a) metalized sides defining a microwave waveguide structure, with the aperture side being non-metalized and oriented toward the tissue; and/or
(b) a loading material disposed within at least a portion of the waveguide, the loading material having a relative permittivity in a range from 1 to 150.

4. The device according to claim 3, wherein the waveguide has a cross-sectional shape of a quadrangle, a rectangle, an oval, a circle, a cone, a square, or a square with rounded edges.

5. The device according to claim 3 or 4, wherein the waveguide comprises at least one post,
wherein an impedance of the microwave waveguide is transformable by introduction of at least one tuning element into at least one post, and
wherein the at least one tuning element is a tuning pin, a tuning probe, a tuning screw, an adjustable stub, an iris plate, a dielectric post, a sliding short, a capacitive coupler, an inductive coupler, a waveguide insert, a flap, or a ferrite element.

6. The device according to any one of claims 3 to 5, wherein the metalized sides of the microwave waveguide comprise a copper, aluminum, brass, silver, or gold, and/or
wherein the loading material comprises ceramics having the relative permittivity in a range from 1 to 50.

7. The device according to any one of claims 3 to 6, wherein the device further comprises:
a shaping element coupled to the aperture of the microwave waveguide and configured to modify the microwave energy distribution and/or to improve thermal uniformity in the treated tissue.

8. The device according to claim 7, wherein the shaping element and the microwave waveguide each comprise a ceramic material, and wherein the ceramic material of the shaping element is the same as that of the microwave waveguide.

9. The device according to claim 7 or 8, wherein the shaping element fully covers a surface of an aperture and comprises at least two lateral extent configured to extend laterally beyond the opposite side of the waveguide aperture, wherein each lateral extent is configured to extend beyond the respective side of the aperture in a range from 0.5 mm to 8 mm.

10. The device according to any one of claims 7 to 9, wherein a ratio between a lateral width of the shaping element and a lateral width of the microwave waveguide is in a range from 1.1 to 1.5, and wherein a ratio between a lateral extension of the shaping element beyond the microwave waveguide, in particular per side, and the width of the microwave waveguide is in a range from 0.10 to 0.25.

11. The device according to claim 1 or 2, wherein the treatment element comprises at least one SIW antenna configured to emit microwave energy toward the tissue, the at least one SIW antenna comprising:
(a) a top metallic layer disposed on the first side of the substrate;
(b) a bottom metallic layer disposed on the second side of the substrate;
(c) a feeding point; and/or
(d) at least one post.

12. The device according to claim 11, wherein the at least one post has a square, rectangular, quadrangular, polygonal, oblong, or elliptical shape and wherein each post has a diameter in a range from 0.001 mm to 100 mm.

13. The device according to claim 12, wherein the posts are arranged in rows and columns, the number of rows being in a range from 1 to 1000, and the number of columns being in a range from 1 to 1000.

14. The device according to one of claims 11 to 13, wherein the at least one SIW antenna has a length and/or width in a range from 1 mm to 1000 mm.

15. The device according to any preceding claims, wherein the microwave energy used for heating the tissue is a microwave field having a frequency in a range from 300 MHz to 300 GHz.
